# EUROPEAN PATENT SPECIFICATION

(11) **EP 1 791 973 B1**
(45) Date of publication and mention of the grant of the patent: **21.10.2009**
(21) Application number: 04782716.7
(22) Date of filing: 31.08.2004
(51) Int. Cl.: C12Q 1/68, C12N 9/10

(54) **DOT1 HISTONE METHYLTRANSFERASES AS A TARGET FOR IDENTIFYING THERAPEUTIC AGENTS FOR LEUKEMIA**
DOT1-HISTON-METHYLTRANSFERASEN ALS ZIEL ZUR IDENTIFIZIERUNG VON THERAPEUTIKA GEGEN LEUKÄMIE
DOT1 HISTONE METHYLTRANSFERASES UTILISEES EN TANT QUE CIBLE POUR IDENTIFIER DES AGENTS THERAPEUTIQUES DESTINES AU TRAITEMENT DE LA LEUCEMIE

(43) Date of publication of application: 06.06.2007
(73) Proprietor: UNIVERSITY OF NORTH CAROLINA AT CHAPEL HILL, Chapel Hill, NC 27599-4105 (US)
(72) Inventor: ZHANG, Yi, Chapel Hill, NC 27514 (US); FENG, Qin, Houston, TX 77030 (US); OKADA, Yuki, Carrboro, NC 27510 (US); XU, Guoliang, Shanghai 200031 (CN)
(74) Representative: Harrison Goddard Foote
(86) International application number: PCT/US2004/028289
(87) International publication number: WO 2006/025832

(56) References cited:
- WO-A2-03/016497
- FENG QIN ET AL: "Methylation of H3-lysine 79 is mediated by a new family of HMTases without a SET domain" CURRENT BIOLOGY, vol. 12, no. 12, 25 June 2002 (2002-06-25), pages 1052-1058, XP002462438 ISSN: 0960-9822
- MIN JINRONG ET AL: "Structure of the catalytic domain of human DOT1L, a non-SET domain nucleosomal histone methyltransferase." CELL, vol. 112, no. 5, 7 March 2003 (2003-03-07), pages 711-723, XP002462439 ISSN: 0092-8674
- DATABASE UniProt 15 November 2002 (2002-11-15), "DOT1L_HUman" XP002462440 retrieved from EBI Database accession no. Q8TEK3

### SEQUENCE LISTING

<110> University of North Carolina-Chapel Hill
   Zhang, Yi
   Feng, Qin
   Okada, Yuki
   Xu, Guoliang
<120> DOT1 HISTONE METHYLTRANSFERASES AS A TARGET FOR IDENTIFYING
   THERAPEUTIC AGENTS FOR LEUKEMIA
<130> 5470.394WO
<160> 22
<170> PatentIn version 3.2
<210> 1
   <211> 4889
   <212> DNA
   <213> Homo sapiens
<400> 1
   cccgcctagc atggtgcggc ggccgcgcgc gcggacatgg gggagaagct ggagctgaga      60
   ctgaagtcgc ccgtgggggc tgagcccgcc gtctacccgt ggccgctgcc ggtctacgat     120
   aaacatcacg atgctgctca tgaaatcatc gagaccatcc gatgggtctg tgaagaaatc     180
   ccggatctca agctcgctat ggagaattac gttttaattg actatgacac caaaagcttc     240
   gagagcatgc agaggctctg cgacaagtac aaccgtgcca tcgacagcat ccaccagctg     300
   tggaagggca ccacgcagcc catgaagctg aacacgcggc cgtccactgg actcctgcgc     360
   catatcctgc agcaggtcta caaccactcg gtgaccgacc ccgagaagct caacaactac     420
   gagcccttct cccccgaggt gtacggggag acctccttcg acctggtggc ccagatgatt     480
   gatgagatca agatgaccga cgacgacctg tttgtggact tggggagcgg tgtgggccag     540
   gtcgtgctcc aggttgctgc tgccaccaac tgcaaacatc actatggcgt cgagaaagca     600
   gacatcccgg ccaagtatgc ggagaccatg gaccgcgagt tcaggaagtg gatgaaatgg     660
   tatggaaaaa agcatgcaga atacacattg gagagaggcg atttcctctc agaagagtgg     720
   agggagcgaa tcgccaacac gagtgttata tttgtgaata attttgcctt tggtcctgag     780
   gtggatcacc agctgaagga gcggtttgca aacatgaagg aaggtggcag aatcgtgtcc     840
   tcgaaaccct ttgcacctct gaacttcaga ataaacagta gaaacttgag tgacatcggc     900
   accatcatgc gcgtggtgga gctctcgccc ctgaagggct cggtgtcgtg gacggggaag     960
   ccagtctcct actacctgca cactatcgac cgcaccatac ttgaaaacta tttttctagt    1020
   ctgaaaaacc caaaactcag ggaggaacag gaggcagccc ggcgccgcca gcagcgcgag    1080
   agcaagagca acgcggccac gcccactaag ggcccagagg gcaaggtggc cggccccgcc    1140
   gacgccccca tggactctgg tgctgaggaa gagaaggcgg gagcagccac cgtgaagaag   1200
   ccgtctccct ccaaagcccg caagaagaag ctaaacaaga aggggaggaa gatggctggc 1260
   cgcaagcgcg ggcgccccaa gaagatgaac actgcgaacc ccgagcggaa gcccaagaag 1320
   aaccaaactg cactggatgc cctgcacgct cagaccgtgt ctcagacggc ggcctcctca 1380
   ccccaggatg cctacagatc ccctcacagc ccgttctacc agctacctcc gagcgtgcag 1440
   cggcactccc ccaacccgct gctggtggcg cccaccccgc ccgcgctgca gaagcttcta 1500
   gagtccttca agatccagta cctgcagttc ctggcataca caaagacccc ccagtacaag 1560
   gccagcctgc aggagctgct gggccaggag aaggagaaga acgcccagct cctgggtgcg 1620
   gctcagcagc tcctcagcca ctgccaggcc cagaaggagg agatcaggag gctgtttcag 1680
   caaaaattgg atgagctggg tgtgaaggcg ctgacctaca acgacctgat tcaagcgcag 1740
   aaggagatct ccgcccataa ccagcagctg cgggagcagt cggagcagct ggagcaggac 1800
   aaccgcgcgc tccgcggcca gagcttgcag ctgctcaagg ctcgctgcga ggagctgcag 1860
   ctggactggg ccacgctgtc gctggagaag ctgttgaagg agaagcaggc cctgaagagc 1920
   cagatctcgg agaagcagag gcactgcctg gagctgcaga tcagcattgt ggagctagag 1980
   aagagccagc ggcagcagga gctcctgcag ctcaagtcct gtgtgccgcc tgacgacgcc 2040
   ctgtccctgc acctgcgtgg gaagggcgcc ctgggccgcg agctggagcc tgacgccagc 2100
   cggctgcacc tggagctgga ctgcaccaag ttctcgctgc ctcacttgag cagcatgagc 2160
   ccggagctct ccatgaacgg ccaggctgct ggctatgagc tctgcggtgt gctgagccgg 2220
   ccttcgtcga agcagaacac gccccagtac ctggcctcac ccctggacca ggaggtggtg 2280
   ccctgtaccc ctagccacgt cggccggccg cgcctggaga agctgtctgg cctagccgca 2340
   cccgactaca ctaggctgtc cccggccaag attgtgctga ggcggcacct gagccaggac 2400
   cacacggtgc ccggcaggcc ggctgccagt gagctgcatt cgagagctga gcacaccaag 2460
   gagaacggcc ttccctacca gagccccagc gtgcctggca gcatgaagct gagccctcag 2520
   gacccgcggc ccctgtcccc tggggccttg cagcttgctg gagagaagag cagtgagaag 2580
   ggcctgagag agcgcgccta cggcagcagc ggggagctca tcaccagcct gcccatcagc 2640
   atcccgctca gcaccgtgca gcccaacaag ctcccggtca gcattcccct ggccagcgtg 2700
   gtgctgccca gccgcgccga gagggcgagg agcaccccca gtcccgtgct gcagccccgt 2760
   gacccctcgt ccacacttga aaagcagatt ggtgctaatg cccacggtgc tgggagcaga 2820
   agccttgccc tggcccccgc aggcttctcc tacgctggct cggtggccat cagcggggcc 2880
   ttggcgggca gcccggcctc tctcacacct ggagccgagc cggccacctt ggatgagtcc 2940
   tccagctctg ggagcctttt tgccaccgtg ggtcccgcag gctccacgcc acagcacccc 3000
   ctgctgctgg cacagccccg gaactcgctt cctgcctctc ccgcccacca gctctcctcc 3060
   agtccccggc ttggtggggc cgcccagggc ccgttgcccg aggccagcaa gggagacctg 3120
   ccctccgatt ccggcttctc agatcctgag agtgaagcca agaggaggat tgtgttcacc 3180
   atcaccactg gtgcgggcag tgccaagcag tcgccctcca gcaagcacag ccccctgacc 3240
   gccagcgccc gtggggactg tgtgccgagc cacgggcagg acagtcgcag gcgcggccgg 3300
   cggaagcgag catctgcggg gacgcccagc ttgagcgcag gcgtgtcccc caagcgccga 3360
   gccctgccgt ccgtcgctgg ccttttcaca cagccttcgg ggtctcccct caacctcaac 3420
   tccatggtca gtaacatcaa ccagcccctg gagattacag ccatctcgtc cccggagacc 3480
   tccctgaaga gctcccctgt gccctaccag gaccacgacc agccccccgt gctcaagaag 3540
   gagcggcctc tgagccagac caatggggca cactactccc cactcacctc agacgaggag 3600
   ccaggctctg aggacgagcc cagcagtgct cgaattgaga gaaaaattgc aacaatctcc 3660
   ttagaaagca aatctccccc gaaaaccttg gaaaatggtg gtggcttggc gggaaggaag 3720
   cccgcgcccg ccggcgagcc agtcaatagc agcaagtgga agtccacctt ctcgcccatc 3780
   tccgacatcg gcctggccaa gtcggcggac agcccgctgc aggccagctc cgccctcagc 3840
   cagaactccc tgttcacgtt ccggcccgcc ctggaggagc cctctgccga tgccaagctg 3900
   gccgctcacc ccaggaaagg ctttcccggc tccctgtcgg gggctgacgg actcagcccg 3960
   ggcaccaacc ctgccaacgg ctgcaccttc ggcgggggcc tggccgcgga cctgagttta 4020
   cacagcttca gtgatggtgc ttctcttccc cacaagggcc ccgaggcggc cggcctgagc 4080
   tccccgctga gcttcccctc gcagcgcggc aaggagggct cggacgccaa ccctttcctg 4140
   agcaagaggc agctggacgg cctggctggg ctgaagggcg agggcagccg cggcaaggag 4200
   gcaggggagg gcggcctacc gctgtgcggg cccacggaca agaccccact gctgagcggc 4260
   aaggccgcca aggcccggga ccgcgaggtc gacctcaaga atggccacaa cctcttcatc 4320
   tctgcggcgg ccgtgcctcc cggaagcctc ctcagcggcc ccggcctggc cccggcggcg 4380
   tcctccgcag gcggcgcggc gtcctccgcc cagacgcacc ggtccttcct gggccccttc 4440
   ccgccgggac cgcagttcgc gctcggcccc atgtccctgc aggccaacct cggctccgtg 4500
   gccggctcct ccgtgctgca gtcgctgttc agctctgtgc cggccgccgc aggcctggtg 4560
   cacgtgtcgt ccgctgccac cagactgacc aactcgcacg ccatgggcag cttttccggg 4620
   gtggcaggcg gcacagttgg aggtaactag gatttctacc tcaaccgcga gacctatgca 4680
   aggacggtgt ggaccaactc gcgcccgcgg catggtgccc gccggcctgc cgggctccca 4740
   cccctggacg gcagaggcaa ggacggacgg gagctccact gtgaatcggc ggcacgcgcc 4800
   gcaggaggct gggactggtc cagtttgtac tgtcgatagt tttagataaa gtatttatca 4860
   ttttttaaaa agtaaaaaaa aaaaaaaaa 4889
<210> 2
   <211> 1537
   <212> PRT
   <213> Homo sapiens
<400> 2
   Met Gly Glu Lys Leu Glu Leu Arg Leu Lys Ser Pro Val Gly Ala Glu
   1               5                   10                  15
   Pro Ala Val Tyr Pro Trp Pro Leu Pro Val Tyr Asp Lys His His Asp
               20                  25                  30
   Ala Ala His Glu Ile Ile Glu Thr Ile Arg Trp Val Cys Glu Glu Ile
           35                  40                  45
   Pro Asp Leu Lys Leu Ala Met Glu Asn Tyr Val Leu Ile Asp Tyr Asp
       50                  55                  60
   Thr Lys Ser Phe Glu Ser Met Gln Arg Leu Cys Asp Lys Tyr Asn Arg
   65                  70                  75                  80
   Ala Ile Asp Ser Ile His Gln Leu Trp Lys Gly Thr Thr Gln Pro Met
                   85                  90                  95
   Lys Leu Asn Thr Arg Pro Ser Thr Gly Leu Leu Arg His Ile Leu Gln
               100                 105                 110
   Gln Val Tyr Asn His Ser Val Thr Asp Pro Glu Lys Leu Asn Asn Tyr
           115                 120                 125
   Glu Pro Phe Ser Pro Glu Val Tyr Gly Glu Thr Ser Phe Asp Leu Val
       130                 135                 140
   Ala Gln Met Ile Asp Glu Ile Lys Met Thr Asp Asp Asp Leu Phe Val
   145                 150                 155                 160
   Asp Leu Gly Ser Gly Val Gly Gln Val Val Leu Gln Val Ala Ala Ala
                   165                 170                 175
   Thr Asn Cys Lys His His Tyr Gly Val Glu Lys Ala Asp Ile Pro Ala
               180                 185                 190
   Lys Tyr Ala Glu Thr Met Asp Arg Glu Phe Arg Lys Trp Met Lys Trp
   195 200 205
   Tyr Gly Lys Lys His Ala Glu Tyr Thr Leu Glu Arg Gly Asp Phe Leu
   210 215 220
   Ser Glu Glu Trp Arg Glu Arg Ile Ala Asn Thr Ser Val Ile Phe Val
   225 230 235 240
   Asn Asn Phe Ala Phe Gly Pro Glu Val Asp His Gln Leu Lys Glu Arg
   245 250 255
   Phe Ala Asn Met Lys Glu Gly Gly Arg Ile Val Ser Ser Lys Pro Phe
   260 265 270
   Ala Pro Leu Asn Phe Arg Ile Asn Ser Arg Asn Leu Ser Asp Ile Gly
   275 280 285
   Thr Ile Met Arg Val Val Glu Leu Ser Pro Leu Lys Gly Ser Val Ser
   290 295 300
   Trp Thr Gly Lys Pro Val Ser Tyr Tyr Leu His Thr Ile Asp Arg Thr
   305 310 315 320
   Ile Leu Glu Asn Tyr Phe Ser Ser Leu Lys Asn Pro Lys Leu Arg Glu
   325 330 335
   Glu Gln Glu Ala Ala Arg Arg Arg Gln Gln Arg Glu Ser Lys Ser Asn
   340 345 350
   Ala Ala Thr Pro Thr Lys Gly Pro Glu Gly Lys Val Ala Gly Pro Ala
   355 360 365
   Asp Ala Pro Met Asp Ser Gly Ala Glu Glu Glu Lys Ala Gly Ala Ala
   370 375 380
   Thr Val Lys Lys Pro Ser Pro Ser Lys Ala Arg Lys Lys Lys Leu Asn
   385 390 395 400
   Lys Lys Gly Arg Lys Met Ala Gly Arg Lys Arg Gly Arg Pro Lys Lys
   405 410 415
   Met Asn Thr Ala Asn Pro Glu Arg Lys Pro Lys Lys Asn Gln Thr Ala
   420 425 430
   Leu Asp Ala Leu His Ala Gln Thr Val Ser Gln Thr Ala Ala Ser Ser
   435 440 445
   Pro Gln Asp Ala Tyr Arg Ser Pro His Ser Pro Phe Tyr Gln Leu Pro
       450                 455                 460
   Pro Ser Val Gln Arg His Ser Pro Asn Pro Leu Leu Val Ala Pro Thr
   465                 470                 475                 480
   Pro Pro Ala Leu Gln Lys Leu Leu Glu Ser Phe Lys Ile Gln Tyr Leu
                   485                 490                 495
   Gln Phe Leu Ala Tyr Thr Lys Thr Pro Gln Tyr Lys Ala Ser Leu Gln
               500                 505                 510
   Glu Leu Leu Gly Gln Glu Lys Glu Lys Asn Ala Gln Leu Leu Gly Ala
           515                 520                 525
   Ala Gln Gln Leu Leu Ser His Cys Gln Ala Gln Lys Glu Glu Ile Arg
       530                 535                 540
   Arg Leu Phe Gln Gln Lys Leu Asp Glu Leu Gly Val Lys Ala Leu Thr
   545                 550                 555                 560
   Tyr Asn Asp Leu Ile Gln Ala Gln Lys Glu Ile Ser Ala His Asn Gln
                   565                 570                 575
   Gln Leu Arg Glu Gln Ser Glu Gln Leu Glu Gln Asp Asn Arg Ala Leu
               580                 585                 590
   Arg Gly Gln Ser Leu Gln Leu Leu Lys Ala Arg Cys Glu Glu Leu Gln
           595                 600                 605
   Leu Asp Trp Ala Thr Leu Ser Leu Glu Lys Leu Leu Lys Glu Lys Gln
       610                 615                 620
   Ala Leu Lys Ser Gln Ile Ser Glu Lys Gln Arg His Cys Leu Glu Leu
   625                 630                 635                 640
   Gln Ile Ser Ile Val Glu Leu Glu Lys Ser Gln Arg Gln Gln Glu Leu
                   645                 650                 655
   Leu Gln Leu Lys Ser Cys Val Pro Pro Asp Asp Ala Leu Ser Leu His
               660                 665                 670
   Leu Arg Gly Lys Gly Ala Leu Gly Arg Glu Leu Glu Pro Asp Ala Ser
           675                 680                 685
   Arg Leu His Leu Glu Leu Asp Cys Thr Lys Phe Ser Leu Pro His Leu
   690 695 700
   Ser Ser Met Ser Pro Glu Leu Ser Met Asn Gly Gln Ala Ala Gly Tyr
   705 710 715 720
   Glu Leu Cys Gly Val Leu Ser Arg Pro Ser Ser Lys Gln Asn Thr Pro
   725 730 735
   Gln Tyr Leu Ala Ser Pro Leu Asp Gln Glu Val Val Pro Cys Thr Pro
   740 745 750
   Ser His Val Gly Arg Pro Arg Leu Glu Lys Leu Ser Gly Leu Ala Ala
   755 760 765
   Pro Asp Tyr Thr Arg Leu Ser Pro Ala Lys Ile Val Leu Arg Arg His
   770 775 780
   Leu Ser Gln Asp His Thr Val Pro Gly Arg Pro Ala Ala Ser Glu Leu
   785 790 795 800
   His Ser Arg Ala Glu His Thr Lys Glu Asn Gly Leu Pro Tyr Gln Ser
   805 810 815
   Pro Ser Val Pro Gly Ser Met Lys Leu Ser Pro Gln Asp Pro Arg Pro
   820 825 830
   Leu Ser Pro Gly Ala Leu Gln Leu Ala Gly Glu Lys Ser Ser Glu Lys
   835 840 845
   Gly Leu Arg Glu Arg Ala Tyr Gly Ser Ser Gly Glu Leu Ile Thr Ser
   850 855 860
   Leu Pro Ile Ser Ile Pro Leu Ser Thr Val Gln Pro Asn Lys Leu Pro
   865 870 875 880
   Val Ser Ile Pro Leu Ala Ser Val Val Leu Pro Ser Arg Ala Glu Arg
   885 890 895
   Ala Arg Ser Thr Pro Ser Pro Val Leu Gln Pro Arg Asp Pro Ser Ser
   900 905 910
   Thr Leu Glu Lys Gln Ile Gly Ala Asn Ala His Gly Ala Gly Ser Arg
   915 920 925
   Ser Leu Ala Leu Ala Pro Ala Gly Phe Ser Tyr Ala Gly Ser Val Ala
   930 935 940
   Ile Ser Gly Ala Leu Ala Gly Ser Pro Ala Ser Leu Thr Pro Gly Ala
   945 950 955 960
   Glu Pro Ala Thr Leu Asp Glu Ser Ser Ser Ser Gly Ser Leu Phe Ala
   965 970 975
   Thr Val Gly Ser Arg Ser Ser Thr Pro Gln His Pro Leu Leu Leu Ala
   980 985 990
   Gln Pro Arg Asn Ser Leu Pro Ala Ser Pro Ala His Gln Leu Ser Ser
   995 1000 1005
   Ser Pro Arg Leu Gly Gly Ala Ala Gln Gly Pro Leu Pro Glu Ala
   1010 1015 1020
   Ser Lys Gly Asp Leu Pro Ser Asp Ser Gly Phe Ser Asp Pro Glu
   1025 1030 1035
   Ser Glu Ala Lys Arg Arg Ile Val Phe Thr Ile Thr Thr Gly Ala
   1040 1045 1050
   Gly Ser Ala Lys Gln Ser Pro Ser Ser Lys His Ser Pro Leu Thr
   1055 1060 1065
   Ala Ser Ala Arg Gly Asp Cys Val Pro Ser His Gly Gln Asp Ser
   1070 1075 1080
   Arg Arg Arg Gly Arg Arg Lys Arg Ala Ser Ala Gly Thr Pro Ser
   1085 1090 1095
   Leu Ser Ala Gly Val Ser Pro Lys Arg Arg Ala Leu Pro Ser Val
   1100 1105 1110
   Ala Gly Leu Phe Thr Gln Pro Ser Gly Ser Pro Leu Asn Leu Asn
   1115 1120 1125
   Ser Met Val Ser Asn Ile Asn Gln Pro Leu Glu Ile Thr Ala Ile
   1130 1135 1140
   Ser Ser Pro Glu Thr Ser Leu Lys Ser Ser Pro Val Pro Tyr Gln
   1145 1150 1155
   Asp His Asp Gln Pro Pro Val Leu Lys Lys Glu Arg Pro Leu Ser
   1160 1165 1170
   Gln Thr Asn Gly Ala His Tyr Ser Pro Leu Thr Ser Asp Glu Glu
   1175 1180 1185
   Pro Gly Ser Glu Asp Glu Pro Ser Ser Ala Arg Ile Glu Arg Lys
   1190 1195 1200
   Ile Ala Thr Ile Ser Leu Glu Ser Lys Ser Pro Pro Lys Thr Leu
   1205 1210 1215
   Glu Asn Gly Gly Gly Leu Ala Gly Arg Lys Pro Ala Pro Ala Gly
   1220 1225 1230
   Glu Pro Val Asn Ser Ser Lys Trp Lys Ser Thr Phe Ser Pro Ile
   1235 1240 1245
   Ser Asp Ile Gly Leu Ala Lys Ser Ala Asp Ser Pro Leu Gln Ala
   1250 1255 1260
   Ser Ser Ala Leu Ser Gln Asn Ser Leu Phe Thr Phe Arg Pro Ala
   1265 1270 1275
   Leu Glu Glu Pro Ser Ala Asp Ala Lys Leu Ala Ala His Pro Arg
   1280 1285 1290
   Lys Gly Phe Pro Gly Ser Leu Ser Gly Ala Asp Gly Leu Ser Pro
   1295 1300 1305
   Gly Thr Asn Pro Ala Asn Gly Cys Thr Phe Gly Gly Gly Leu Ala
   1310 1315 1320
   Ala Asp Leu Ser Leu His Ser Phe Ser Asp Gly Ala Ser Leu Pro
   1325 1330 1335
   His Lys Gly Pro Glu Ala Ala Gly Leu Ser Ser Pro Leu Ser Phe
   1340 1345 1350
   Pro Ser Gln Arg Gly Lys Glu Gly Ser Asp Ala Asn Pro Phe Leu
   1355 1360 1365
   Ser Lys Arg Gln Leu Asp Gly Leu Ala Gly Leu Lys Gly Glu Gly
   1370 1375 1380
   Ser Arg Gly Lys Glu Ala Gly Glu Gly Gly Leu Pro Leu Cys Gly
   1385 1390 1395
   Pro Thr Asp Lys Thr Pro Leu Leu Ser Gly Lys Ala Ala Lys Ala
       1400                1405                1410
   Arg Asp Arg Glu Val Asp Leu Lys Asn Gly His Asn Leu Phe Ile
       1415                1420                1425
   Ser Ala Ala Ala Val Pro Pro Gly Ser Leu Leu Ser Gly Pro Gly
       1430                1435                1440
   Leu Ala Pro Ala Ala Ser Ser Ala Gly Gly Ala Ala Ser Ser Ala
       1445                1450                1455
   Gln Thr His Arg Ser Phe Leu Gly Pro Phe Pro Pro Gly Pro Gln
       1460                1465                1470
   Phe Ala Leu Gly Pro Met Ser Leu Gln Ala Asn Leu Gly Ser Val
       1475                1480                1485
   Ala Gly Ser Ser Val Leu Gln Ser Leu Phe Ser Ser Val Pro Ala
       1490                1495                1500
   Ala Ala Gly Leu Val His Val Ser Ser Ala Ala Thr Arg Leu Thr
       1505                1510                1515
   Asn Ser His Ala Met Gly Ser Phe Ser Gly Val Ala Gly Gly Thr
       1520                1525                1530
   Val Gly Gly Asn
       1535
<210> 3
   <211> 53
   <212> PRT
   <213> Homo sapiens
<400> 3
   Arg Lys Leu Pro Phe Gln Arg Leu Val Arg Glu Ile Ala Gln Asp Phe
   1 5 10 15
   Lys Thr Asp Leu Arg Phe Gln Ser Ala Ala Ile Gly Ala Leu Gln Glu
   20 25 30
   Ala Ser Glu Ala Tyr Leu Val Gly Leu Phe Glu Asp Thr Asn Leu Cys
   35 40 45
   Ala Ile His Ala Lys
   50
<210> 4
   <211> 11
   <212> PRT
   <213> Homo sapiens
<400> 4
   Ile Ala Gln Asp Phe Lys Thr Asp Leu Arg Phe
   1 5 10
<210> 5
   <211> 293
   <212> PRT
   <213> Saccharomyces cerevisiae
<400> 5
   Glu Thr Ile Leu Pro Asp Leu Asn Ala Ser Phe Asp Asn Ser Asp Thr
   1               5                   10                  15
   Lys Gly Phe Val Asn Ala Ile Asn Leu Tyr Asn Lys Met Ile Arg Glu
               20                  25                  30
   Ile Pro Arg Gln Arg Ile Ile Asp His Leu Glu Thr Ile Asp Lys Ile
           35                  40                  45
   Pro Arg Ser Phe Ile His Asp Phe Leu His Ile Val Tyr Thr Arg Ser
       50                  55                  60
   Ile His Pro Gln Ala Asn Lys Leu Lys His Tyr Lys Ala Phe Ser Asn
   65                  70                  75                  80
   Tyr Val Tyr Gly Glu Leu Leu Pro Asn Phe Leu Ser Asp Val Tyr Gln
                   85                  90                  95
   Gln Cys Gln Leu Lys Lys Gly Asp Thr Phe Met Asp Leu Gly Ser Gly
               100                 105                 110
   Val Gly Asn Cys Val Val Gln Ala Ala Leu Glu Cys Gly Cys Ala Leu
           115                 120                 125
   Ser Phe Gly Cys Glu Ile Met Asp Asp Ala Ser Asp Leu Thr Ile Leu
       130                 135                 140
   Gln Tyr Glu Glu Leu Lys Lys Arg Cys Lys Leu Tyr Gly Met Arg Leu
   145                 150                 155                 160
   Asn Asn Val Glu Phe Ser Leu Lys Lys Ser Phe Val Asp Asn Asn Arg
   165 170 175
   Val Ala Glu Leu Ile Pro Gln Cys Asp Val Ile Leu Val Asn Asn Phe
   180 185 190
   Leu Phe Asp Glu Asp Leu Asn Lys Lys Val Glu Lys Ile Leu Gln Thr
   195 200 205
   Ala Lys Val Gly Cys Lys Ile Ile Ser Leu Lys Ser Leu Arg Ser Leu
   210 215 220
   Thr Tyr Gln Ile Asn Phe Tyr Asn Val Glu Asn Ile Phe Asn Arg Leu
   225 230 235 240
   Lys Val Gln Arg Tyr Asp Leu Lys Glu Asp Ser Val Ser Trp Thr His
   245 250 255
   Ser Gly Gly Glu Tyr Tyr Ile Ser Thr Val Met Glu Asp Val Asp Glu
   260 265 270
   Ser Leu Phe Ser Pro Ala Ala Arg Gly Arg Arg Asn Arg Gly Thr Pro
   275 280 285
   Val Lys Tyr Thr Arg
   290
<210> 6
   <211> 292
   <212> PRT
   <213> Homo sapiens
<400> 6
   Leu Ala Met Glu Asn Tyr Val Leu Ile Asp Tyr Asp Thr Lys Ser Phe
   1 5 10 15
   Glu Ser Met Gln Arg Leu Cys Asp Lys Tyr Asn Arg Ala Ile Asp Ser
   20 25 30
   Ile His Gln Leu Trp Lys Gly Thr Thr Gln Pro Met Lys Leu Asn Thr
   35 40 45
   Arg Pro Ser Thr Gly Leu Leu Arg His Ile Leu Gln Gln Val Tyr Asn
   50 55 60
   His Ser Val Thr Asp Pro Glu Lys Leu Asn Asn Tyr Glu Pro Phe Ser
   65 70 75 80
   Pro Glu Val Tyr Gly Glu Thr Ser Phe Asp Leu Val Ala Gln Met Ile
   85 90 95
   Asp Glu Ile Lys Met Thr Asp Asp Asp Leu Phe Val Asp Leu Gly Ser
   100 105 110
   Gly Val Gly Gln Val Val Leu Gln Val Ala Ala Ala Thr Asn Cys Lys
   115 120 125
   His His Tyr Gly Val Glu Lys Ala Asp Ile Pro Ala Lys Tyr Ala Glu
   130 135 140
   Thr Met Asp Arg Glu Phe Arg Lys Trp Met Lys Trp Tyr Gly Lys Lys
   145 150 155 160
   His Ala Glu Tyr Thr Leu Glu Arg Gly Asp Phe Leu Ser Glu Glu Trp
   165 170 175
   Arg Glu Arg Ile Ala Asn Thr Ser Val Ile Phe Val Asn Asn Phe Ala
   180 185 190
   Phe Gly Pro Glu Val Asp His Gln Leu Lys Glu Arg Phe Ala Asn Met
   195 200 205
   Lys Glu Gly Gly Arg Ile Val Ser Ser Lys Pro Phe Ala Pro Leu Asn
   210 215 220
   Phe Arg Ile Asn Ser Arg Asn Leu Ser Asp Ile Gly Thr Ile Met Arg
   225 230 235 240
   Val Val Glu Leu Ser Pro Leu Lys Gly Ser Val Ser Trp Thr Gly Lys
   245 250 255
   Pro Val Ser Tyr Tyr Leu His Thr Ile Asp Arg Thr Ile Leu Glu Asn
   260 265 270
   Tyr Phe Ser Ser Leu Lys Asn Pro Lys Leu Arg Glu Glu Gln Glu Ala
   275 280 285
   Ala Arg Arg Arg
   290
<210> 7
   <211> 293
   <212> PRT
   <213> Drosophila melanogaster
<400> 7
   Ser Ala Phe Glu Glu Thr Asn Leu His Gln Ile Asp Thr Ala Cys Tyr
   1               5                   10                  15
   Lys Thr Met Thr Gly Leu Val Asp Arg Phe Asn Lys Ala Val Asp Ser
               20                  25                  30
   Ile Val Ala Leu Glu Lys Gly Thr Ser Leu Pro Ala Glu Arg Leu Asn
           35                  40                  45
   Lys Phe Ala His Pro Ser Leu Leu Arg His Ile Leu Gln Leu Val Tyr
       50                  55                  60
   Asn Ala Ala Val Leu Asp Pro Asp Lys Leu Asn Gln Tyr Glu Pro Phe
   65                  70                  75                  80
   Ser Pro Glu Val Tyr Gly Glu Thr Ser Tyr Glu Leu Val Gln Gln Met
                   85                  90                  95
   Leu Lys His Val Thr Val Ser Lys Glu Asp Thr Phe Ile Asp Leu Gly
               100                 105                 110
   Ser Gly Val Gly Gln Val Val Leu Gln Met Ala Gly Ser Phe Pro Leu
           115                 120                 125
   Lys Thr Cys Ile Gly Ile Glu Lys Ala Asp Thr Pro Ala Arg Tyr Ala
       130                 135                 140
   Glu Arg Met Asp Val Ile Phe Arg Gln Tyr Met Gly Trp Phe Gly Lys
   145                 150                 155                 160
   Arg Phe Cys Glu Tyr Lys Leu Ile Lys Gly Asp Phe Leu Val Asp Glu
                   165                 170                 175
   His Arg Glu Asn Ile Thr Ser Ser Thr Leu Val Phe Val Asn Asn Phe
               180                 185                 190
   Ala Phe Gly Pro Thr Val Asp His Gln Leu Lys Glu Arg Phe Ala Asp
           195                 200                 205
   Leu Arg Asp Gly Ala Arg Val Val Ser Ser Lys Ser Phe Cys Pro Leu
       210                 215                 220
   Asn Phe Arg Ile Thr Asp Arg Asn Leu Ser Asp Ile Gly Thr Ile Met
   225 230 235 240
   His Val Ser Glu Ile Pro Pro Leu Lys Gly Ser Val Ser Trp Thr Cys
   245 250 255
   Lys Pro Val Ser Tyr Tyr Leu His Val Ile Asp Arg Thr Ile Leu Glu
   260 265 270
   Arg Tyr Phe Gln Arg Leu Lys Thr Lys Gly Gly Asn Asp His Glu Ser
   275 280 285
   Val Gly Thr Val Arg
   290
<210> 8
   <211> 278
   <212> PRT
   <213> Caenorhabditis elegans
<400> 8
   Val Pro Leu Pro Ser Asp Ala Val Val Gly Asn Pro Asp Lys Phe Ser
   1               5                   10                  15
   Gln Tyr Ile Asp Ala Leu Asn Leu Lys Leu Ala His Pro Asp Val Leu
               20                  25                  30
   Ser Arg Val Pro Ala Lys His His Glu Ile Thr Ser Thr Arg Leu Cys
           35                  40                  45
   Ser Leu Thr Val Ala Ile Lys Leu Ala Glu Leu Ala Tyr Arg Phe Ser
       50                  55                  60
   Val Pro Asp Ala Asn Val Leu Arg His Tyr Ala Val Gly Thr Ser Thr
   65                  70                  75                  80
   Val Tyr Gly Glu Leu His Cys Ser Gln Met Ala Ser Phe Val Asp Gln
                   85                  90                  95
   Leu Asn Met Gly Pro Ser Asp Tyr Phe Met Asp Leu Gly Ser Gly Ile
               100                 105                 110
   Gly His Leu Val Asn Phe Val Ala Ala Tyr Ala Arg Thr Gln Met Ser
           115                 120                 125
   Val Gly Val Glu Leu Met Asp Asn Leu Ala Glu Ile Ala Glu Lys Asn
       130                 135                 140
   Lys Glu Phe Asn Glu Arg Leu Leu Asn His Phe Gly Lys Lys Val Tyr
   145                 150                 155                 160
   Ala Thr Arg Phe Ile His Gly Ser Phe Thr Ser Pro Ala Val Ile Arg
                   165                 170                 175
   Glu Ile Gln Thr Lys Ala Thr Val Ile Leu Ala Asn Asn Val Arg Phe
               180                 185                 190
   Asp Pro Glu Leu Lys Leu Gln Leu Lys Glu Ile Leu Met Gly Cys Lys
           195                 200                 205
   Asp Arg Thr Arg Ile Ile Ser Ser Glu Pro Leu Val Pro Ser Arg Ala
       210                 215                 220
   Arg Gln Thr Asn Ser Arg Arg Ala Asp Asp Phe Val Lys Ile Ser Asp
   225                 230                 235                 240
   Glu Ser Leu Leu Asn Leu Val Asp Asn Asn Val Ser Trp Thr Ser Arg
                   245                 250                 255
   Lys Val Pro Phe Tyr Leu Thr Thr Ile Asn Arg Asn Lys Val Ser Phe
               260                 265                 270
   Phe Tyr Cys Val Arg Phe
           275
<210> 9
   <211> 56
   <212> PRT
   <213> Homo sapiens
<400> 9
   Glu Gly Lys Val Ala Gly Pro Ala Asp Ala Pro Met Asp Ser Gly Ala
   1 5 10 15
   Glu Glu Glu Lys Ala Gly Ala Ala Thr Val Lys Lys Pro Ser Pro Ser
   20 25 30
   Lys Ala Arg Lys Lys Lys Leu Asn Lys Lys Gly Arg Lys Met Ala Gly
   35 40 45
   Arg Lys Arg Gly Arg Pro Lys Lys
   50 55
<210> 10
   <211> 58
   <212> PRT
   <213> Saccharomyces cerevisiae
<400> 10
   Lys Gly Lys Asp Asn Gly Leu Val Pro Leu Val Glu Lys Val Ile Pro
   1 5 10 15
   Pro Ile His Lys Lys Thr Asn Asn Arg Asn Thr Arg Lys Lys Ser Ser
   20 25 30
   Thr Thr Thr Lys Lys Asp Val Lys Lys Pro Lys Ala Ala Lys Val Lys
   35 40 45
   Gly Lys Asn Gly Arg Thr Asn His Lys His
   50 55
<210> 11
   <211> 15
   <212> PRT
   <213> Homo sapiens
<400> 11
   Asn Glu Leu Ala Leu Lys Leu Ala Gly Leu Asp Ile Asn Lys Thr
   1 5 10 15
<210> 12
   <211> 15
   <212> PRT
   <213> Homo sapiens
<400> 12
   Pro Ala Leu Gln Lys Leu Leu Glu Ser Phe Lys Ile Gln Tyr Leu
   1               5                   10                  15
<210> 13
   <211> 15
   <212> PRT
   <213> Homo sapiens
<400> 13
   His Cys Leu Glu Leu Gln Ile Ser Ile Val Glu Leu Glu Lys Ser
   1               5                   10                  15
<210> 14
   <211> 15
   <212> PRT
   <213> Homo sapiens
<400> 14
   Pro Ala Leu Gln Lys Leu Leu Glu Ser Ala Lys Ala Gln Tyr Leu
   1               5                   10                  15
<210> 15
   <211> 66
   <212> PRT
   <213> Homo sapiens
<400> 15
   Ala Ser Ile Glu Gln Leu Leu Glu Arg Gln Trp Ser Glu Gly Gln Gln
   1               5                   10                  15
   Phe Leu Leu Glu Gln Gly Thr Pro Ser Asp Ile Leu Gly Met Leu Lys
               20                  25                  30
   Ser Leu His Gln Leu Gln Val Glu Asn Arg Arg Leu Glu Glu Gln Ile
           35                  40                  45
   Lys Asn Leu Thr Ala Lys Lys Glu Arg Leu Gln Leu Leu Asn Ala Gln
       50                  55                  60
   Leu Ser
   65
<210> 16
   <211> 66
   <212> PRT
   <213> Mus musculus
<400> 16
   Ala Ser Ile Glu Gln Leu Leu Glu Arg Gln Trp Ser Glu Gly Gln Gln
   1 5 10 15
   Phe Leu Leu Glu Gln Gly Thr Pro Gly Asp Ile Leu Gly Met Leu Lys
   20 25 30
   Ser Leu His Gln Leu Gln Val Glu Asn Arg Arg Leu Glu Glu Gln Ile
   35 40 45
   Lys Asn Leu Thr Ala Lys Lys Glu Arg Leu Gln Leu Leu Asn Ala Gln
   50 55 60
   Leu Ser
   65
<210> 17
   <211> 66
   <212> PRT
   <213> Drosophila melanogaster
<400> 17
   Gln Ser Leu Glu Gln Leu Leu Glu Arg Gln Trp Glu Gln Gly Ser Gln
   1 5 10 15
   Phe Leu Met Glu Gln Ala Gln His Phe Asp Ile Ala Ser Leu Leu Asn
   20 25 30
   Cys Leu His Gln Leu Gln Ser Glu Asn Leu Arg Leu Glu Glu His Val
   35 40 45
   Thr Ser Leu Ile Ala Arg Arg Asp His Leu Leu Ala Val Asn Ala Arg
   50 55 60
   Leu Gln
   65
<210> 18
   <211> 65
   <212> PRT
   <213> Caenorhabditis elegans
<400> 18
   Ser Phe Met Glu Gln Leu Leu Glu Arg Gln Trp Asp Gln Gly Ser Ser
   1 5 10 15
   Leu Leu Met Ala Asn Ala His Phe Asp Val Ala Gln Leu Leu Ser Cys
   20 25 30
   Leu Phe Gln Leu Lys Ser Glu Asn Phe Arg Leu Glu Glu Asn Leu Ser
   35 40 45
   Gly Leu Arg Lys Arg Arg Asp His Leu Phe Ala Leu Asn Ser Arg Leu
   50 55 60
   Ala
   65
<210> 19
   <211> 8
   <212> PRT
   <213> Artificial Sequence
<220>
   <223> Conserved octapeptide motif
<400> 19
   Glu Gln Leu Leu Glu Arg Gln Trp
   1 5
<210> 20
   <211> 21
   <212> PRT
   <213> Artificial Sequence
<220>
   <223> Synthetic positively charged C-RS10 peptide
<400> 20
   Cys Arg Ser Arg Ser Arg Ser Arg Ser Arg Ser Arg Ser Arg Ser Arg
   1 5 10 15
   Ser Arg Ser Arg Ser
   20
<210> 21
   <211> 8
   <212> PRT
   <213> Artificial Sequence
<220>
   <223> SV40 T-antigen nuclear localization signal
<400> 21
   Asp Pro Lys Lys Lys Arg Lys Val
   1 5
<210> 22
   <211> 12
   <212> PRT
   <213> Artificial Sequence
<220>
   <223> Concensus nuclear export, signal
<220>
   <221> MISC_FEATURE
   <222> (2)..(2)
   <223> "Xaa" denotes 2 or 3 of any amino acid
<220>
   <221> MISC_FEATURE
   <222> (8)..(8)
   <223> "Xaa" denotes 2 or 3 of any amino acid
<220>
   <221> MISC_FEATURE
   <222> (10)..(10)
   <223> "Xaa" denotes any amino acid
<400> 22
   Leu Xaa Leu Ile Val Phe Met Xaa Leu Xaa Leu Ile
   1 5 10

## Description

### STATEMENT OF FEDERAL SUPPORT

This invention was made with federal support under Grant Nos. GM63076 and GM68804 awarded by the National Institutes of Health/National Cancer Institute. The United States government has certain rights in the invention.

### FIELD OF THE INVENTION

The present invention relates to novel histone methyltransferases as well as nucleic acids encoding the same; also disclosed are methods for identifying compounds that modulate the activity of the histone methyltransferase, methods of identifying compounds that inhibit binding of a polypeptide to the histone methyltransferase, methods of identifying candidate compounds for the treatment of leukemia, and diagnostic methods based on histone methylation.

### BACKGROUND OF THE INVENTION

Higher-order chromatin structures are of profound importance in gene regulation and epigenetic inheritance (Wu and Grunstein (2000) Trends Biochem. Sci. 25:619-623). Post-translational modifications of core histones critically influence the establishment and maintenance of higher-order chromatin structures. The unstructured tails of certain core histones are extensively modified by acetylation, methylation, phosphorylation, ribosylation and ubiquitination. A "histone code" hypothesis, linking histone modifications to chromatin structures, has been the focus of intensive recent studies (Strahl and Allis (2000) Mol. Cell. Biol. 22:1298-1306; Turner (2000) Bioessays 22:836-845). Histone methylation has emerged as a major form of histone modification. (Strahl and Allis (2000) Mol. Cell. Biol. 22:1298-1306; Zhang and Reinberg (2001) Genes Dev. 15:2343-2360). In particular, a large family of SET domain-containing histone methyltransferases (HMTases) has been identified (Lachner and Jenuwein (2002) Curr. Opin. Cell Biol. 14:286-298). SET domain proteins have been shown to methylate various N-terminal lysine residues of histone H3 and H4. Histone lysine methylation has been associated with diverse biological processes ranging from transcriptional regulation to the faithful transmission of chromosomes during the cell division (Grewal and Elgin (2002) Curr. Opin. Genet. Dev. 12:178-187).

Further, lysine methylation catalyzed by SET domain containing proteins has been linked to cancer (Schneider, et al. (2002) Trends Biochem. Sci. 27:396-402). For example, the H3-K4 methyltransferase MLL is frequently translocated in leukemia (Ayton and Cleary (2001) Oncogene 20:5695-5707; Milne, et al. (2002) Mol. Cell 10:1107-1117; Nakamura, et al. (2002) Mol. Cell 10:1119-1128) and the H3-K27 methyltransferase EZH2 is overexpressed in a number of tumors and its expression level correlates with the invasiveness of these tumors (Bracken, et al. (2003) EMBO J. 22:5323-5335; Kleer, et al. (2003) Proc. Natl. Acad. Sci. USA 100:11606-1161-1; Varambally, et al. (2002) Nature 419:624-9).

Chromosomal translocation is one of the major causes of human cancer, particularly in acute leukemias. The most common chromosome rearrangement found in leukemia patients involves the mixed lineage leukemia gene MLL (also called ALL or HRX) located at 11q23 (Ayton and Cleary (2001) Oncogene 20:5695-5707). MLL is the human homologue of *Drosophila* Trithorax (Trx), a protein involved in maintaining the "on state" of the homeotic box (Hox) gene expression during embryonic development. MLL contains a number of functional motifs including the N-terminal AT hook DNA binding motif and the C-terminal SET domain required for its H3-lysine **4** methyltransferase activity (Milne, et al. (2002) Mol. Cell 10:1107-1117; Nakamura, et al. (2002) Mol. Cell 10:1119-1128). As a result of chromosome translocation, MLL N-termini become fused in-frame with one of more than 30 partner proteins (Ayton and Cleary (2001) Oncogene 20:5695-5707). Regardless of whether the fusion partner is normally localized to the nucleus or cytoplasm, the chimeras are always nuclear (Dimartino and Cleary (1999) Br. J. Haematol. 106:614-626). Given that the DNA binding domain of MLL is still retained in the fusion proteins, the MLL target genes will be differentially regulated as a result of loss of the MLL SET domain and gain of fusion partner function in the chimeras. HOXA9 has emerged as one of the most relevant MLL target genes in human acute myeloid leukemia (AML) as it is always up-regulated in AML (Golub, et al. (1999) Science 286:531-537), Indeed, the leukemogenic potential of Hoxa9 was directly demonstrated by the development of AML in mice receiving transplantation of bone marrow cells overexpressing Hoxa9 (Kroon, et al. (1998) EMBO J. 17:3714-3725). Both Hoxa7 and Hoxa9 have been shown to be required for MLL fusion proteins to transform myeloid progenitor cells (Ayton and Cleary (2003) Genes Dev. 17:2298-2307). However, the mechanism by which different MLL fusion proteins up-regulate HOXA9 and how higher levels of HOXA9 leads to leukemia remains to be elucidated.

Dot1 is an evolutionarily conserved protein that was originally identified in S. *cerevisiae* as a disruptor of telomeric silencing (Singer, et al, (1998) Genetics 150:613-632). It also functions at the pachytene checkpoint during the meiotic cell cycle (San-Segundo and Roeder (2000) Mol. Biol. Cell. 11:3601-3615). Sequence analysis of yeast Dot1 revealed that it possesses certain characteristic SAM binding motifs, similar to the ones in protein arginine methyltransferases (Dlakic (2001) Trends Biochem. Sci. 26:405-407).

### SUMMARY OF THE INVENTION

The present invention is based, in part, on the first identification of a post-translational modification within the globular domain of the histone. In particular, the inventors have observed methylation of lysine 79 of histone H3 ("H3-K79") and have identified a novel class of histone methyltransferase (HMTase) designated "DOT1" that methylates H3-K79 *in vivo.* DOT1 L (*see*, *e.g*., SEQ ID NO:2) is a member of the DOT1 family. Similar to other HMTases, the DOT1 polypeptides contain a S-adenosylmethionine (SAM) binding site and use SAM as a methyl donor. However, unlike other reported HMTases, the DOT1 polypeptides do not contain a SET domain.

The present invention describes an isolated nucleic acid comprising a nucleotide sequence encoding a DOT1L polypeptide, the nucleotide sequence selected from the group consisting of:
(a) a nucleotide sequence comprising the nucleotide sequence of **SEQ ID NO:1;**
(b) a nucleotide sequence that hybridizes to the complete complement of a nucleotide sequence comprising the nucleotide sequence of **SEQ ID NO:1** under stringent conditions, wherein the nucleotide sequence encodes a polypeptide having histone H3 lysine 79 (H3-K79) methyltransferase activity;
(c) a nucleotide sequence having at least 95% nucleotide sequence similarity with the nucleotide sequence of **SEQ ID NO:1,** wherein the nucleotide sequence encodes a polypeptide having H3-K79 methyltransferase activity;
(d) a nucleotide sequence that differs from the nucleotide sequence of (a) above due to the degeneracy of the genetic code; (f) a nucleotide sequence comprising a functional fragment of **SEQ ID NO:1,** wherein the functional fragment comprises at least the coding region of the histone methyltransferase catalytic domain of **SEQ ID NO:1** or a nucleotide sequence that hybridizes to the complete complement of the coding region of the histone methyltransferase catalytic domain of **SEQ ID NO:1** under stringent conditions, and wherein the functional fragment encodes a polypeptide having H3-K79 methyltransferase activity; (g) a nucleotide sequence comprising a functional fragment of **SEQ ID NO:1,** wherein the functional fragment comprises at least the coding region of the histone methyltransferase catalytic domain of **SEQ ID NO:1** or a nucleotide sequence having at least 95% nucleotide sequence similarity thereto, and wherein the functional fragment encodes a polypeptide having H3-K79 methyltransferase activity; and (h) a nucleotide sequence comprising a functional fragment of **SEQ ID NO:1,** wherein the functional fragment comprises at least the coding region of the histone methyltransferase catalytic domain of **SEQ ID NO:1** or a nucleotide sequence that differs therefrom due to the degeneracy of the genetic code, and wherein the functional fragment encodes a polypeptide having H3-K79 methyltransferase activity

Also described are vectors comprising the nucleic acids as well as cells comprising the nucleic acids and vectors.

The invention describes an isolated DOT1 L polypeptide comprising an amino acid sequence selected from the group consisting of: (a) the amino acid sequence of **SEQ ID NO:2;** (b) an amino acid sequence having at least 95% amino acid sequence similarity with the amino acid sequence of **SEQ ID NO:2,** wherein the DOT1 L polypeptide has histone H3 lysine 79 (H3-K79) methyltransferase activity; and (c) a functional fragment of at least 400 amino acids of the amino acid sequence of (a) or (b) above, wherein the functional fragment comprises a DOT1L histone methyltransferase catalytic domain and has H3-K79 methyltransferase activity.

Also described are fusion proteins comprising the DOT1L polypeptide. Further described are cells comprising the fusion proteins of the invention.

The invention describes a method of identifying a compound that modulates DOT1L histone H3 lysine 79 (H3-K79) methyltransferase activity, the method comprising contacting a DOT1L polypeptide of the invention with a nucleosome substrate comprising H3 in the presence of a test compound; detecting the level of H3-K79 methylation of the nucleosome substrate under conditions sufficient to provide H3-K79 methylation, wherein an elevation or reduction in H3-K79 methylation in the presence of the test compound as compared with the level of histone H3-K79 methylation in the absence of the test compound indicates that the test compound modulates DOT1L H3-K79 methyltransferase activity.

According to a first aspect, the invention provides a method of identifying a candidate compound for the prevention and/or treatment of leukemia, the method comprising: contacting a DOT1L polypeptide of the invention with a nucleosome substrate comprising histone H3 in the presence of a test compound; detecting the level of histone H3 lysine 79 (H3-K79) methylation of the nucleosome substrate under conditions sufficient to provide H3-K79 methylation, wherein a reduction in H3-K79 methylation in the presence of the test compound as compared with the level of H3-K79 methylation in the absence of the test compound indicates that the test compound is a candidate compound for the prevention and/or treatment of leukemia.

The invention describes a method of identifying a compound that inhibits binding of DOT1L polypeptide to a second polypeptide, comprising: contacting a DOT1L polypeptide according to the invention with a second polypeptide in the presence of a test compound; detecting the level of binding between DOT1L potypeptide and the second polypeptide under conditions sufficient for binding of DOT1L polypeptide to the second polypeptide, wherein a reduction in binding between DOT1L polypeptide and the second polypeptide in the presence of the test compound as compared with the level of binding in the absence of the test compound indicates that the test compound inhibits binding of DOT1L polypeptide to the second polypeptide.

As another aspect, the invention provides a method of identifying a candidate compound for the prevention and/or treatment of leukemia, comprising: contacting a DOT1L polypeptide of the invention with AF10 or an MLL fusion protein in the presence of a test compound; detecting the level of binding between the DOT1L polypeptide and AF10 or MLL fusion protein under conditions sufficient for binding of DOT1L polypeptide to AF10 or MLL fusion protein, wherein a reduction in binding between DOT1L polypeptide and AF10 or the MLL fusion protein in the presence of the test compound as compared with the level of binding in the absence of the test compound indicates that the test compound is a candidate compound for the prevention and/or treatment of leukemia.

As a further aspect, the invention provides a method of diagnosing whether a subject has or is at risk for developing leukemia and/or determining the prognosis for the course of the disease, comprising: obtaining a biological sample comprising nucleosomes from a subject; detecting the level of histone H3 lysine 79 (H3-K79) methylation associated with the HoxA9 gene; wherein an elevation in HoxA9-associated H3-K79 methylation in the biological sample as compared with the level of HoxA9-associated H3-K79 methylation in a non-leukemic biological sample is diagnostic that the subject has or is at risk of developing leukemia and/or is prognostic of the course of the disease in the subject.

These and other aspects of the invention are set forth in more detail in the description of the invention below.

### BRIEF DESCRIPTION OF THE DRAWINGS

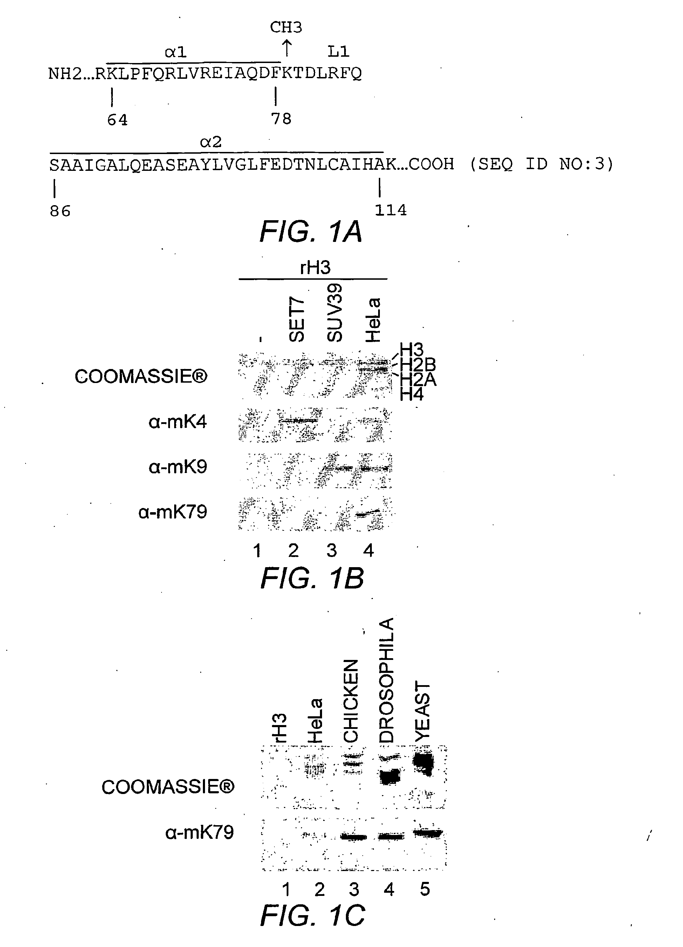

**Figures 1A-1C** show that H3-K79 methylation is conserved from yeast to human. (**A**) Location of K79 of histone H3 relative to the two α-helices (α1 and α2) and loop 1 (L1) (**SEQ ID NO:3**) based on the known nucleosome structure (Luger, et al. (1997) Nature 389:251-260). (**B**) Characterization of the H3-mK79 antibody. HeLa core histones, recombinant histone H3 that are not methylated, methylated by SET7 or SUV39 were analyzed by COOMASSIE^{®} staining and western blot analysis using the mK4-, mK9-, and mK79-specific antibodies. The mK79-specific antibody was raised against an H3 peptide containing di-methylated K79 (Ile-Ala-Gln-Asp-Phe-^{m}Lys-Thr-Asp-Leu-Arg-Phe; **SEQ ID NO:4**). (C) H3-K79 methylation occurs in a wide range of organisms. Equivalent amounts of histones, as evidenced by COOMASSIE^{®} staining, from the indicated organisms were analyzed by western blot analysis using the H3-mK79-specific antibody.

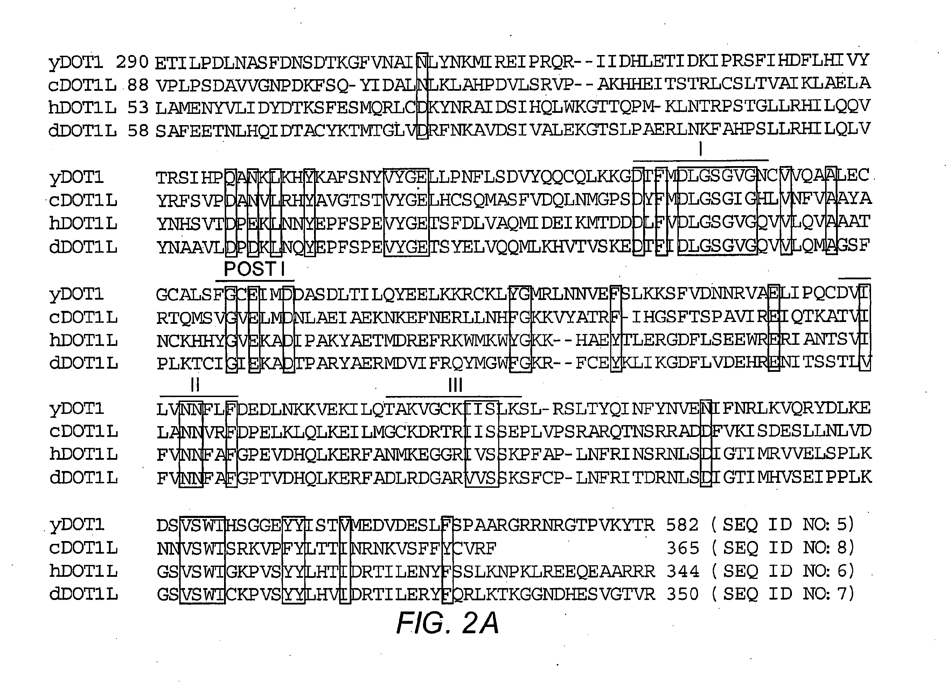

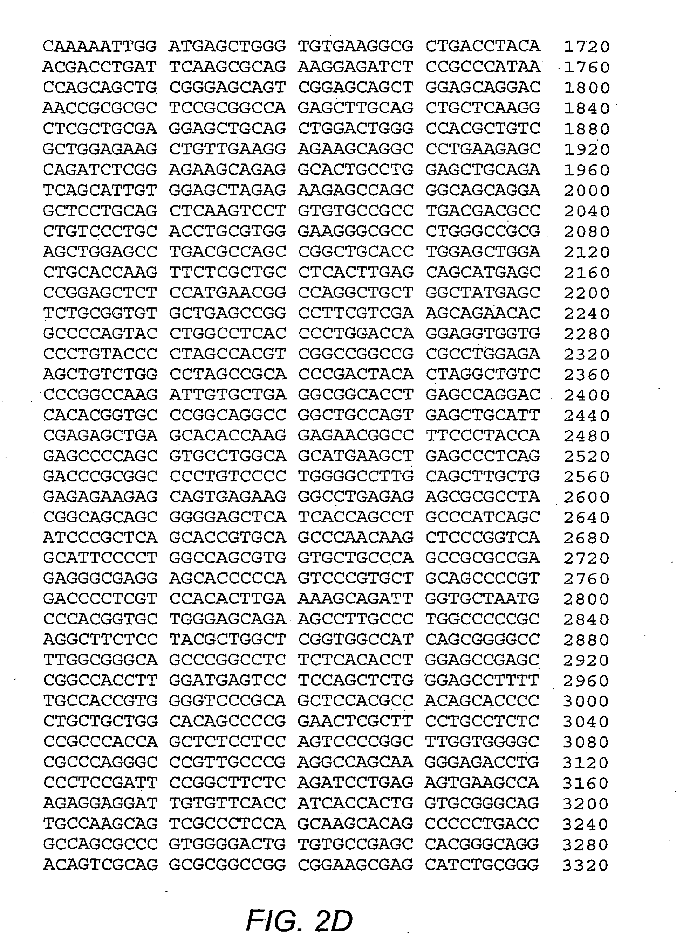

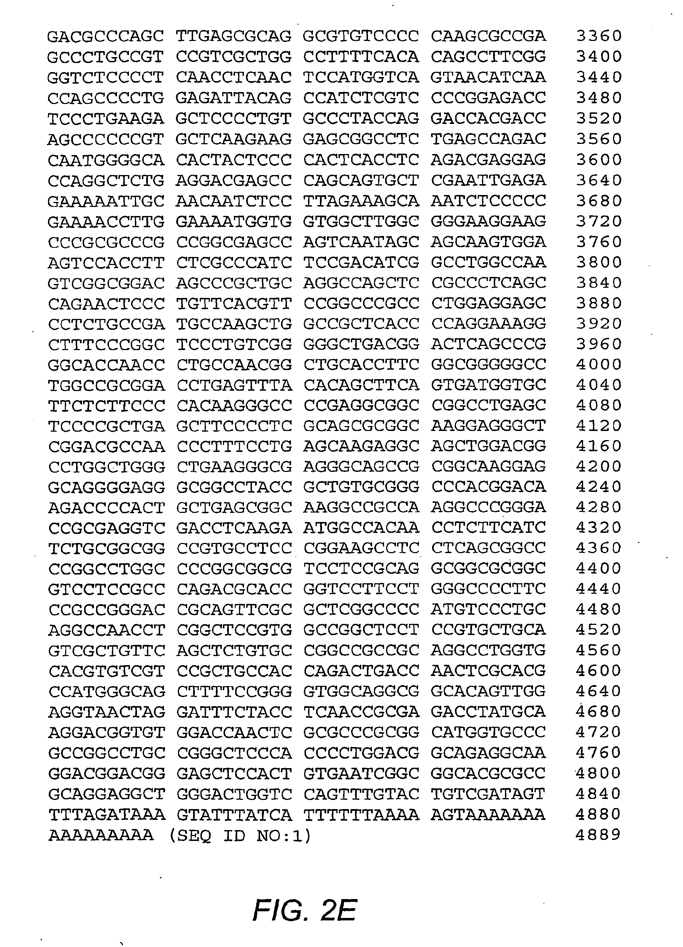

**Figures 2A-2E** show the identification of a human DOT1-like protein. (**A**) Alignment of the amino acid sequences of the DOT1 family proteins. Only the most conserved regions are shown. Sequences used in the alignment include yeast DOT1 (NP_010728; **SEQ ID NO:5**), and its homologs from human (AF509504; **SEQ ID NO:6**), *Drosophila* (AAF54122; **SEQ ID NO:7**), and C. *elegans* (NP_510056; **SEQ ID NO:8**). Four additional putative C. *elegans* proteins in GENBANK (NP_509981, NP_490970, NP_509997, NP_508351) also show homology to DOT1. Sequences predicted to be involved in SAM binding (Dlakic (2001) Trends Biochem. Sci. 26:405-407; incorporated by reference herein in its entirety for teachings of SAM binding domains in DOT1) are indicated. Numbers represent the amino acid number of respective proteins. Gaps are indicated by "-". Amino acids that are identical or have similar properties are boxed. (**B**) Amino acid sequence of human DOT1 L (**SEQ ID NO:2**). Predicted SAM binding motifs are underlined. Mutation of the boxed amino acids abolished the HMTase activity of hDOT1 L. (C-E) Nucleic acid sequence encoding human DOT1L (**SEQ ID NO:1**).

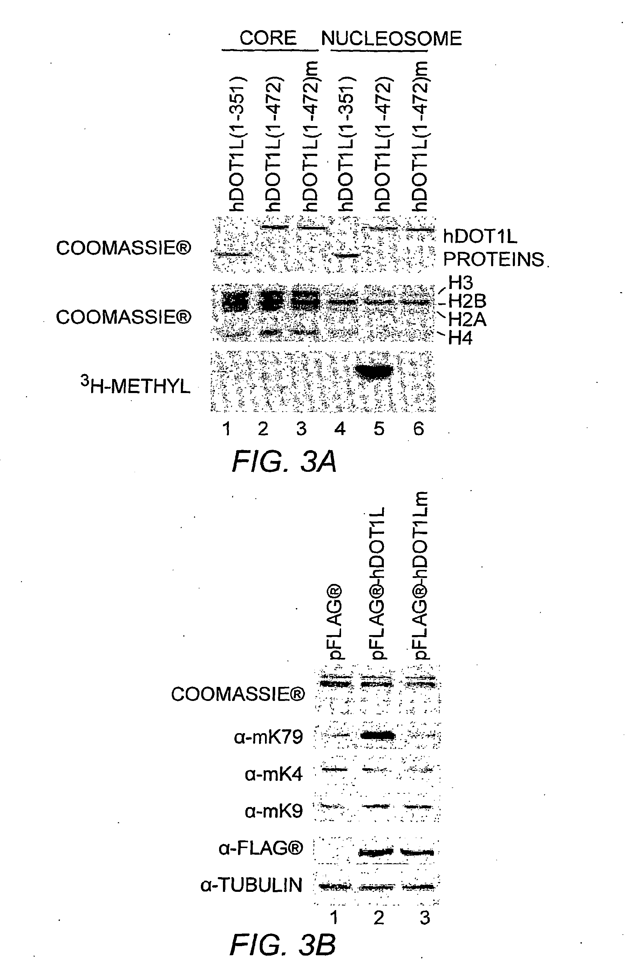

**Figures 3A and 3B** demonstrate that hDOT1 L is a nucleosomal H3-K79-specific HMTase. (**A**) Recombinant hDOT1L is a nucleosomal H3-specific HMTase *in vitro.* About 0.2 µg of different versions of recombinant hDOT1L protein were incubated with 10 µg of core histones or equivalent amounts of nucleosomes using standard methods (Wang, et al. (2001) Mol. Cell 8:1207-1217). The reaction mixtures were resolved in SDS-PAGE followed by COOMASSIE^{®} staining and fluorogram analysis. Recombinant hDOT1L proteins shown in the upper panel are 15-fold of what were used in the HMTase assay. (**B**) hDOT1L methylates H3-K79 *in vivo.* Empty vector, as well as vectors that encode FLAG^{®}-tagged wild-type or mutant (m) hDOT1L were transfected into 293T cells using the QIAGEN^{®} EFFECTENE™ Transfection Reagent. Two days after transfection, cells were collected for the preparation of total cell lysates and histones. Expression of wild-type and mutant hDOT1L was verified by western blot analysis using anti- FLAG^{®} antibody. Equal loading of lysates was confirmed by probing for tubulin. Equal loading of histones was verified by COOMASSlE^{®} staining.

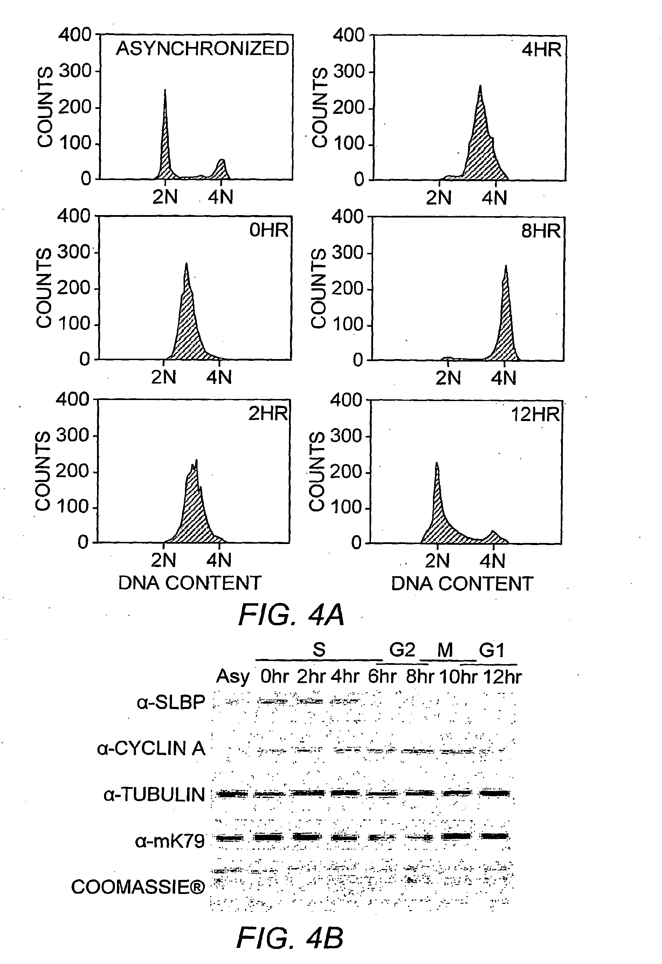

**Figures 4A-4E** show that the level of H3-K79 methylation is cell cycle regulated. (**A**) Cells released from double thymidine block were analyzed by flow cytometry. The numbers of cells (arbitrary units) were plotted against DNA content. (**B**) Cell extracts and histones derived from samples analyzed in (**A**) were analyzed by western blot analysis. SLBP and cyclin A were used as cell cycle markers, tubulin was used as loading control. Equal loading of histones were revealed by COOMASSIE^{®} staining. The level of H3-K79 methylation was analyzed by probing with the mK79-specific antibodies. The cell cycle stage of each sample is indicated on top of the panel. 'asy' represents synchronized cell extracts. (**C, D**) Identical to (**A, B**) except that the cells were arrested at M-phase before release. (**E**) mK79 level in relation to cell DNA content. Cells were labeled with FITC-conjugated goat anti-rabbit secondary antibody in the absence (left panel) or presence of primary antibody (middle and right panels) as indicated. DNA in each cell was labeled with propidium iodide (PI). Each spot represents an individual cell. The numbers represent an arbitrary FITC unit. Unlabeled cells were analyzed by flow cytometry (left panel insert) to serve as standard for DNA content. The intensity of FITC signal (measurement of Mi-2 or mK79) is plotted against PI signal (measurement of DNA content that correlates with cell cycle stages). Arrow heads and arrows represent early S-phase and late S phase, respectively.

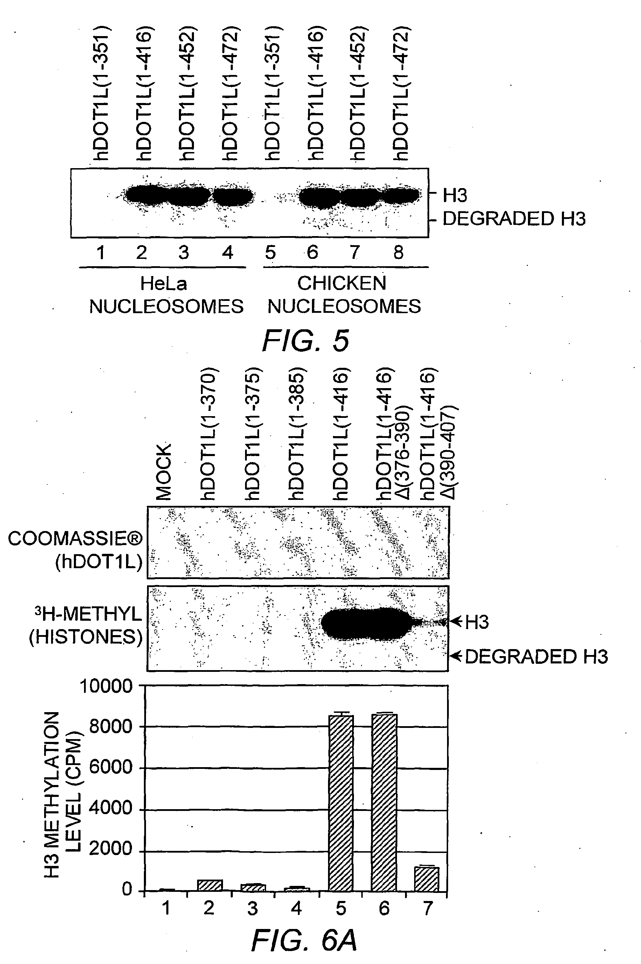

**Figures 5** shows that hDOT1 L(1-416) is an active histone methyltransferase. Purified HeLa and chicken nucleosomes were used as substrates in the assay and proteins were analyzed by autoradiography.
**Figures 6A-6D** show HMTase activity and mono-nucleosome binding of hDOT1L derivatives. (**A**) The positively charged region (amino acids 390-407) is important for the HMTase activity of hDOT1L. Equal amount of hDOT1L deletion mutants (upper panel), indicated on top of the panel, were analyzed for HMTase activity (middle panel) and the quantitation is presented in the bottom panel. (**B**) The positively charged region required for HMTase activity is also required for mono-nucleosome binding. Mono-nucleosome binding of the hDOT1L deletions mutants was analyzed by gel mobility shift assays at three different protein concentrations (µM). (C) Amino acid sequence (amino acids 361-416; **SEQ ID NO:9)** of the C-terminal of hDOT1 L(1-416) encompassing the charged region required for the HMTase activity. A stretch of similar amino acids located at the N-terminal of yeast Dot1 is also shown **(SEQ ID NO:10).** Identical amino acids between the yeast and human proteins are single underlined, and similar amino acids are double underlined. (**D**) The HMTase activities of hDOT1L(1-416) and Δ(376-390) in the absence and presence of increasing concentrations (µM) of a positively charged competitor peptide were analyzed by autoradiograph (top panel) and the quantitation is shown at the bottom panel.

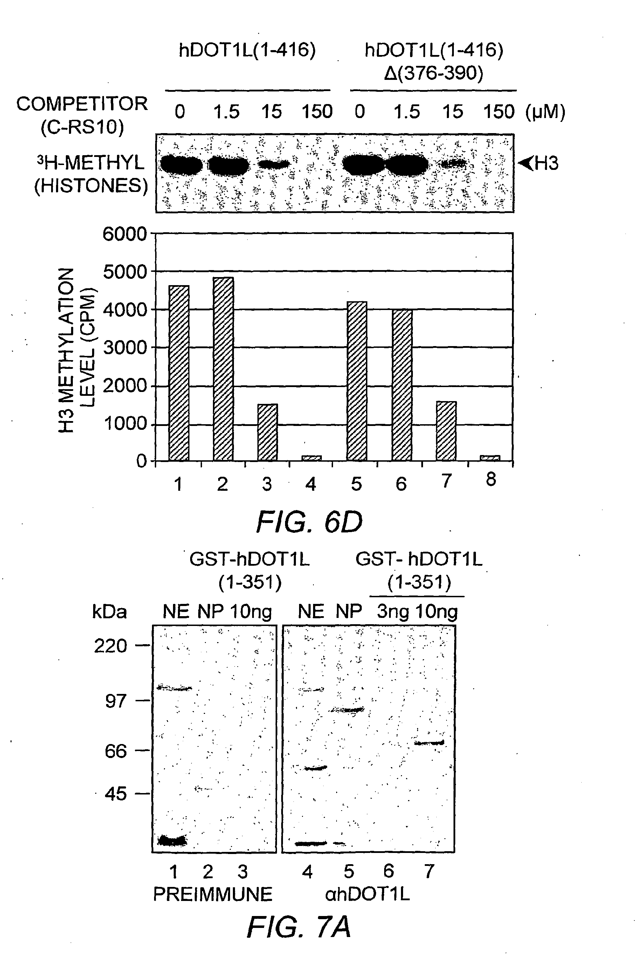

**Figures 7A-7C** show that hDOT1L is degraded extensively in cells. (**A**) Recombinant hDOT1L(1-351) digested by thrombin from GST-hDOT1L(1-352) was used to generate a rabbit polyclonal antibody. The antibody was affinity purified by incubating the serum with the antigen. Protein (100 µg) derived from HeLa nuclear extracts (NE) or nuclear pellet (NP), or recombinant GST-hDOT1L(1-351) were subjected to SDS-PAGE and blotted with either preimmune serum or affinity-purified hDOT1L antibody. (**B**) Full-length hDOT1L exists only in the nucleus. N-terminal FLAG^{®}-tagged or C-terminal HA-tagged hDOT1L was transfected into 293T cells. Proteins (100 µg) derived from nuclear or cytoplasm fractions of transfected cells were subjected to western blot analysis using antibodies against FLAG^{®} or HA. (C) hDOT1 L tagged with an N-terminal FLAG^{®} and a C-terminal HA was transfected into U2OS cells. Localization of hDOT1 L was viewed by immunofluorescent staining using a monoclonal antibody against FLAG^{®} and a polyclonal antibody against HA. Transfected cells with nuclear localization or cytoplasmic localization were counted and presented as percentage of total transfected cells.

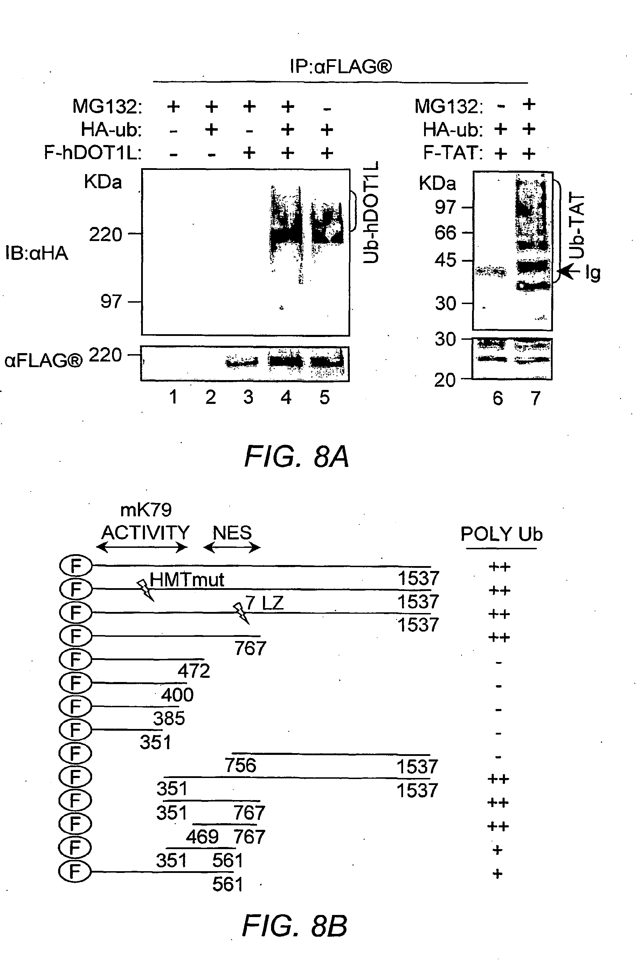

**Figures 8A-8C** show that hDOT1 L is subjected to ubiquitination in *vino.* (**A**) hDOT1 L is subjected to ubiquitination but its degradation is not through the 26S proteasome pathway. FLAG^{®}-tagged hDOT1 L and Tat were co-expressed with HA-ubiquitin in the presence or absence of proteasome inhibitor MG132. Protein extracts derived from the transfected cells were immunoprecipitated with anti-FLAG^{®} antibody and blotted with antibody against FLAG^{®}. (B) hDOT1L ubiquitination occurs in the region between amino acid 469-756. Diagram of the hDOT1 L constructs tested in the ubiquitination assays described in A. Results of the assays are summarized to the right of the panel. "++" represents full level of polyubiquitination; "+" represents lower level of polyubiquitination; "-" represents mono or no ubiquitination. (C) hDOT1 L can be ubiquitinated equally well in both nucleus and cytoplasm. Western blot of the ubiquitination assays of the two hDOT1L(1-767) constructs indicated. Lanes 5 and 6 indicate a similar level of ubiquitination regardless of whether the protein is present in cytoplasm (lane 5) or nucleus (lane 6).

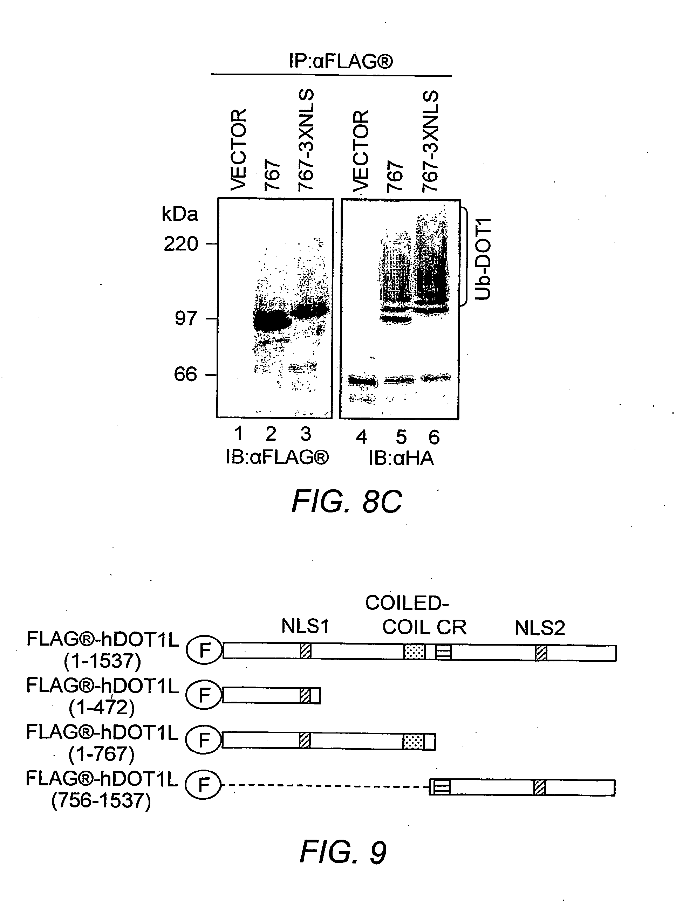

**Figures 9** illustrates that hDOT1 L contains signals for nuclear and cytoplasm localization and presents a diagram of full-length and deletion hDOT1 L constructs. NLS1 and NLS2 represent two nuclear localization signals (NLS) identified by the PredictNLS program. In addition, a coiled-coil and a region conserved between *Drosophila* and human DOT1 L (CR) is also indicated.

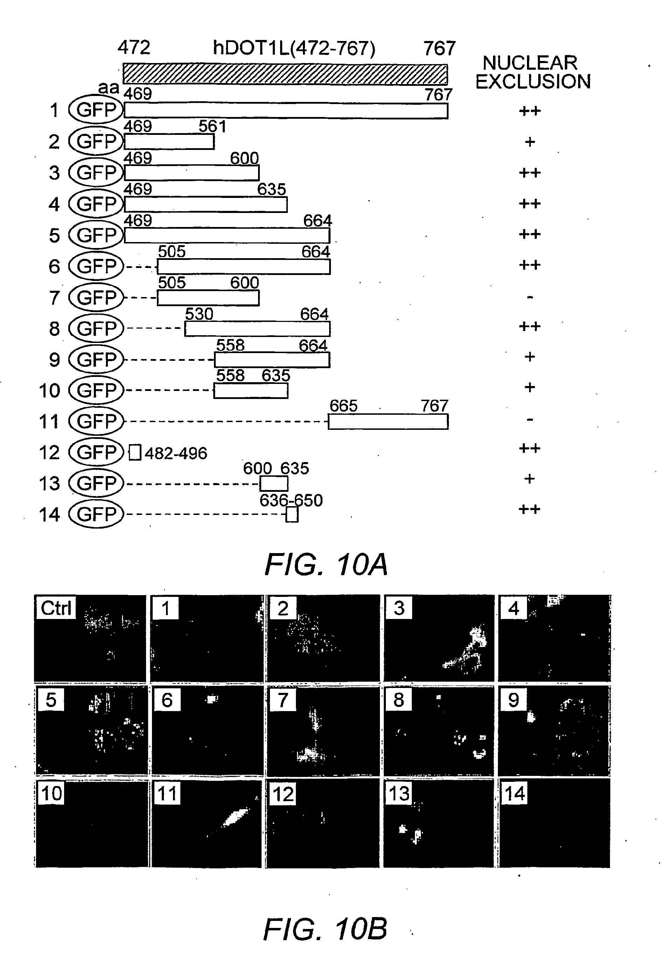

**Figures 10A-10E** show the identification of the three nuclear export signals (NESs) in hDOT1 L. (**A**) Diagram of the different EGFP-hDPT1L fusion constructs and summary of their nuclear exclusion patterns. "++", "+", and "-" represent strong, weak, and no nuclear exclusion, respectively. (**B**) Representative pictures of the constructs, shown in **A**, transfected into U2OS cells. (**C**) Top panel is a sequence alignment of NES1 **(SEQ ID NO:12)** and NES2 (**SEQ ID NO:13**) of hDOT1L with NES of protein kinase A inhibitor (PKI; **SEQ ID NO:11**). Conserved amino acids are underlined and amino acids mutated in NES1 M are boxed. Bottom panels show mutation on the two C-terminal hydrophobic amino acids of NES1 **(SEQ ID NO:14**) disrupts NES1 function. (D) Demonstration of the NES1 function in hDOT1L localization in the context of full-length protein. (**E**) The function of each of the three NESs of hDOT1 L can be blocked by LMB treatment.

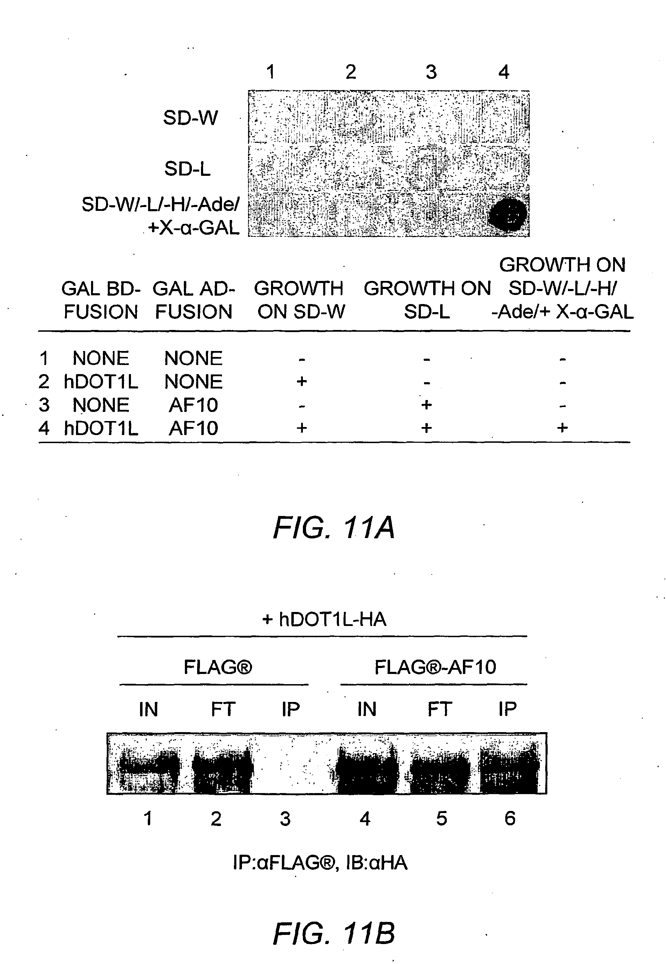

**Figures 11A and 11B** show the identification of AF10, a MLL fusion partner in leukemia, as an hDOT1L associated protein. (**A**) Yeast two-hybrid results demonstrating that only yeast cells containing both hDOT1L and AF10 plasmids could grow and form a blue colony on SD-Trp/-Leu/-His/-adenine/+X-α-Gal plates ("SD-W/-L/-H/-Ade/+X-α-Gal"). (**B**) AF10 and hDOT1L co-immunoprecipitate when co-expressed in 293T cells. FLAG^{®} antibody was used for immunoprecipitation, and HA antibody was used for western blot analysis.

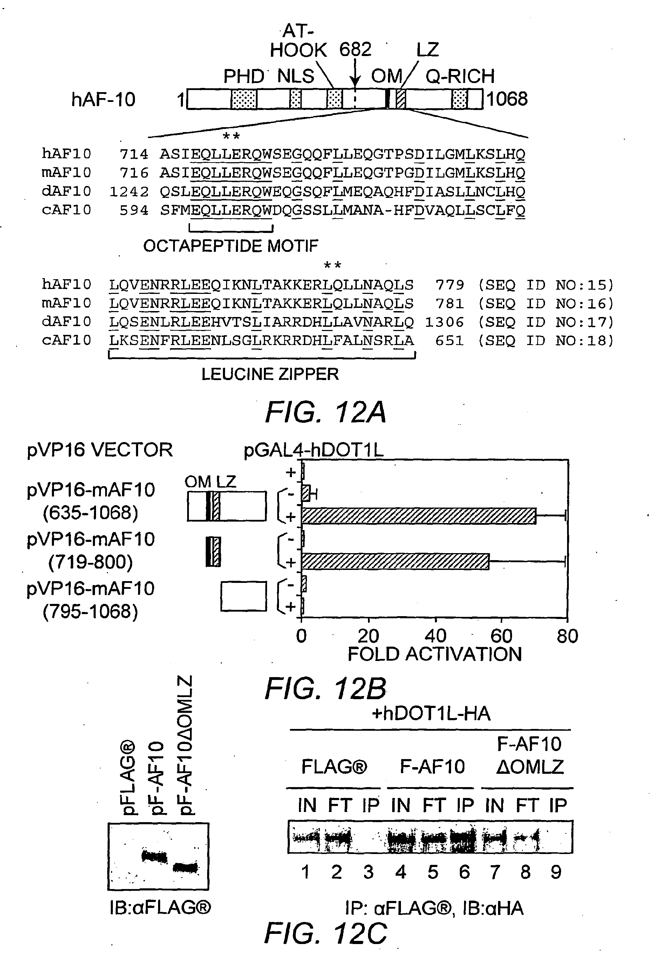

**Figures 12A-12D** show that the octapeptide motif and Leucine zipper (OM-LZ) region of AF10, required for leukemogenesis of MLL-AF10, is also required for hDOT1L and AF10 interaction. (**A**) Diagram of AF10 functional motifs and sequence alignment of the OM and LZ regions. Arrow indicates the break point for MLL-AF10 fusion. Human AF10 (AY598745; **SEQ ID NO:15**), and its homologs from mouse ("mAF10", 054826; **SEQ ID NO:16**), *Drosophila,* ("dAF10", Alhambra, AAF72595; **SEQ ID NO:17**), and C. *elegans* ("cAF10", Cezf, 2122400A; **SEQ ID NO:18**) were compared. Conserved amino acids are underlined. Mutations on the amino acids marked by * disrupt the interaction between hDOT1L and AF10. (**B**) Mammalian two-hybrid analysis identified the OM-LZ region of AF10 which is sufficient for mediating AF10 and hDOT1L interaction. **(C**) Co-immunoprecipitation experiments demonstrating that the OM-LZ region of AF1 0 is necessary for the AF10 and hDOT1L interaction. Western blot analysis shown on the left panel indicated that the wild-type and the deletion mutant proteins were expressed to a similar level. (**D**) Mammalian two-hybrid assays demonstrating that both the evolutionarily conserved octapeptide (Glu-Gln-Leu-Leu-Glu-Arg-Gln-Trp; **SEQ ID NO:19)** motif (OM) and the leucine zipper (LZ) of AF10 contribute to the interaction. The mutations are marked by * in panel **A**.

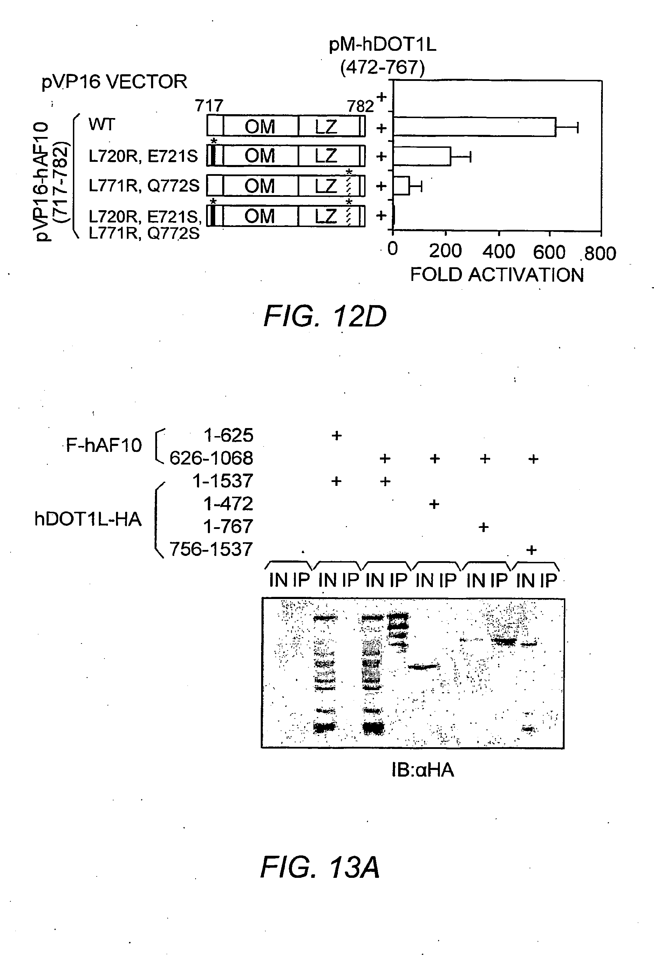

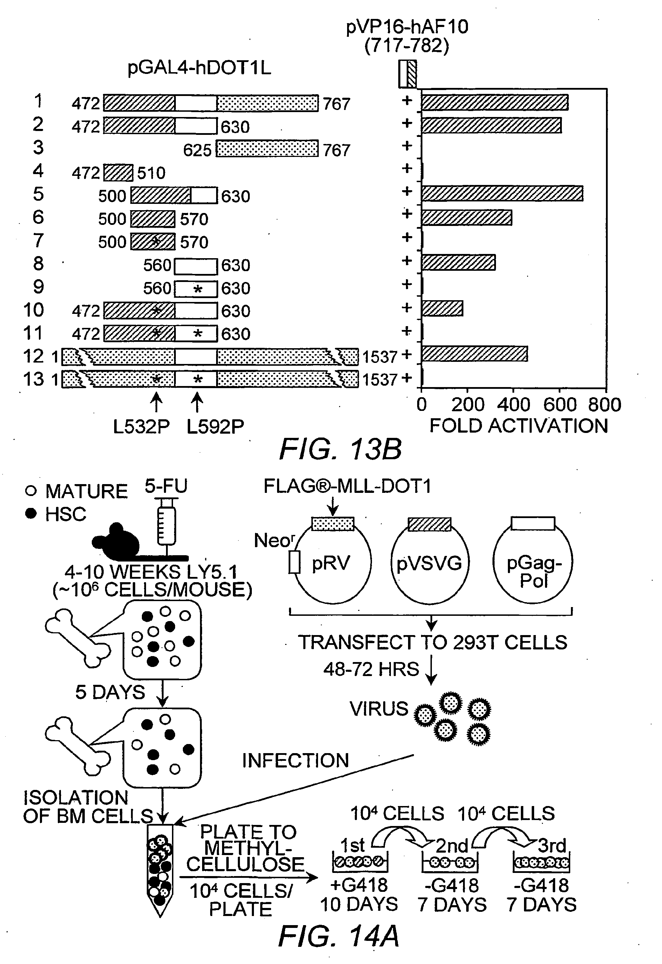

**Figures 13A** **and** **13B** show that a leucine rich region in hDOT1L mediates the AF10 and hDOT1L interaction (**A**) Co-immunoprecipitation assay identified a leucine rich region (amino acids 472-767) of hDOT1L to be important for AF1 0 and hDOT1L interaction. (**B**) Mammalian two-hybrid assays confirmed and further narrowed the interaction regions of hDOT1 L to amino acids 500-630. In addition, mutagenesis studies demonstrated that leucines 532 and 592 are both important for the interaction. Numbers indicate the amino acid number of hDOT1 L.
**Figures 14A-14C** shows that bone marrow transformation by MLL-hDOT1 L requires the HMTase activity and a coiled-coil region (amino acids 416-670) of hDOT1L. (**A**) Schematic representation of the retroviral transduction procedures. (**B**) Diagram of retroviral constructs expressing MLL, MLL-AF10, and MLL-hDOT1 L. The numbers refer to the amino acid number of corresponding proteins. The HMTase defective mutant (containing GCG to RCR change) is marked by *. (**C**) Relative colony numbers generated per 10⁴ transduced bone marrow cells in the first, second, and third round of plating. The various control and fusion proteins expressed are indicated. Data presented is the average of four independent experiments with error bars.

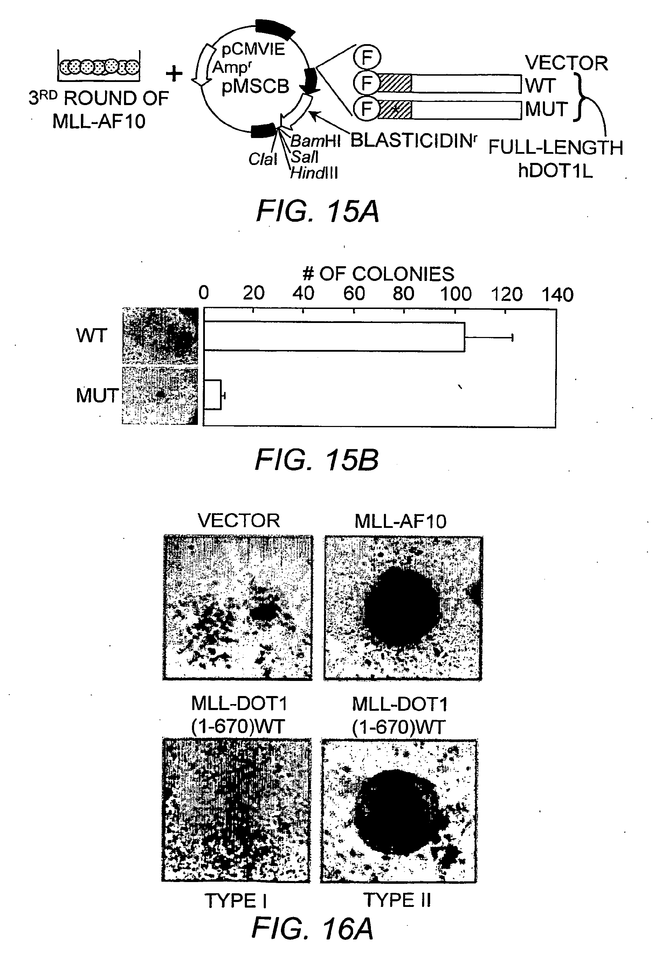

**Figures 15A and 15B** show that transduction of the HMTase-defective hDOT1L into MLL-AF10 transformed bone marrow cells attenuates the proliferation ability of the transformed cells. (**A**) Diagram depicting the assay. (**B**) Colony numbers generated from transduction of 10⁴ cells of the third round of MLL-AF10-transformed bone marrow cells with wild-type or HMTase-defective MLL-hDOT1L(1-670). Data presented is the average of two independent experiments with error bars.
**Figures 16A-16C** show MLL-hDOT1L(1-670) immortalizes murine myeloid progenitors and uncharacterized cells. (**A**) Morphology of colonies formed in methylcellulose by MLL-AF10- and MLL-hDOT1L(1-670)-transduced cells. (**B**) Total and individual numbers of the two types of colonies formed in each round transduced by MLL-hDOT1L(1-670). (C) FACS analysis of the early myeloid markers, Mac1 and c-Kit, of the cells derived from second round plating of MLL-AF10- or hDOT1L(1-670)-transduced cells. Percent of cells expressing the indicated surface antigens is indicated.

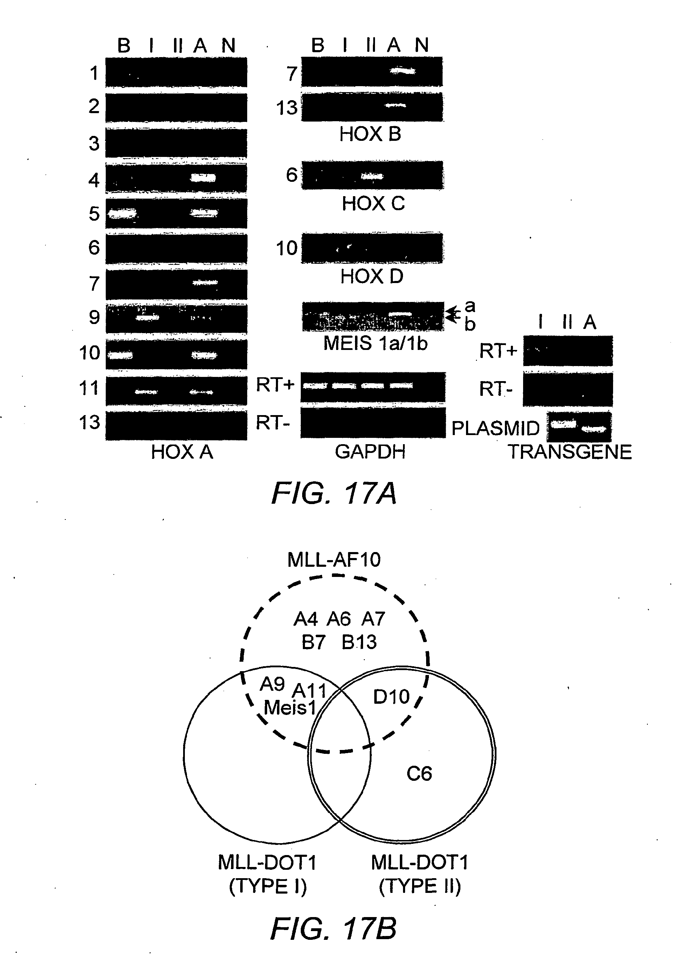

**Figures 17A and 17B** show a comparison of Hox gene expression patterns between primary bone marrow cells and MLL-AF10- or MLL-hDOT1 L(1-670)-transduced cells. (**A**) RT-PCR showing expression response to MLL-AF10 or MLL-hDOT1L(1-670) transduction for each of the HoxA genes and other Hox genes. The expression patterns of the two Meis1 isoforms (a, b correspond to the upper and lower bands of the doublet, respectively) were also analyzed due to the reported collaborative role of Hoxa and Meis genes in myeloid leukemogenesis (Nakamura, et al. (1996) Nat. Genet. 12:149-153). GAPDH serves as a control for equal input in RT-PCR for different samples. Presence of MLL-AF10 or MLL-hDOT1L transgenes in the transformed cells was verified by PCR. "B" refers to primary bone marrow; "I" and "II" refer to the two different types of colonies arising from MLL-hDOT1 L(1-670) transduction; "A" refers to colonies arising from MLL-AF10 transduction; "N" is a negative control for RT-PCR. (**B**) Summary of the genes overexpressed in transduced cells relative to none-transduced bone marrow cells analyzed in **A.**

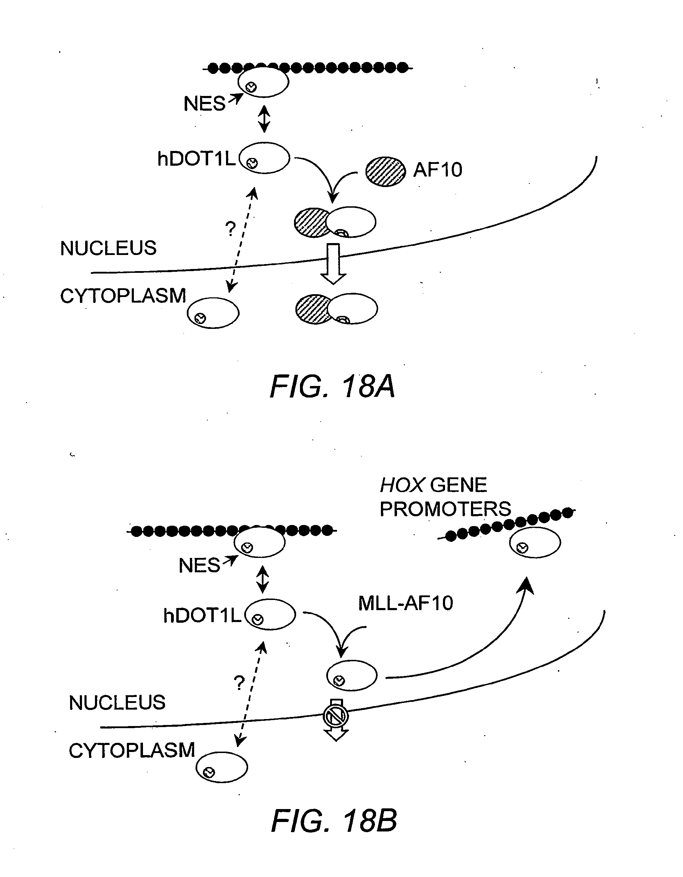

**Figures 18A and 18B** depict the relationship between hDOT1L, AF10, and MLL-AF10 in normal (**A**) and leukemia (**B**) cells.

### DETAILED DESCRIPTION OF THE INVENTION

Chromatin structure is important in gene regulation and epigenetic inherence. It is known that post-translational modifications of histones are involved in the establishment and maintenance of higher-order chromatin structure; further, it has been reported that the tails of certain core histones are modified by acetylation, methylation, phosphorylation, ribosylation and ubiquitination. The present invention is based, in part, on the first identification of a post-translational modification within the globular domain of the histone. In particular, the inventors have observed methylation of lysine 79 of histone H3 ("H3-K79") and have identified a novel class of histone methyltransferase (HMTase) designated "DOT1" that methylates H3-K79 *in vivo.* DOT1L is a member of the DOT1 HTMase family (*see, e.g.,* **SEQ ID NO:2**)**.** Similar to other HMTases, the DOT1 polypeptide contain a S-adenosylmethionine (SAM) binding site and use SAM as a methyl donor. However, unlike other reported HMTases, the DOT1 polypeptides do not contain a SET domain.

The yeast homolog of DOT1 was originally identified as a Disruptor Of Telomeric silencing (the protein and nucleic acid sequences of yeast DOT1 can be found at Accession No. NP_010728; incorporated herein by reference in its entirety). The inventors have cloned and isolated the human DOT1 homolog, designated as hDOT1L (human DOT1-like protein), and determined that hDOT1L is an HMTase. The sequences of the human nucleic acid (**SEQ ID NO:1**) and protein (**SEQ ID NO:2**) have been deposited under GenBank accession number AF509504. Only the approximately 360 N-terminal amino acids of hDOT1L share significant sequence similarity with the yeast DOT1. The inventors have further identified DOT1 homologs from *C. elegans (C. elegans,* GenBank Accession number NP_510056 and CAA90610), Drosophila (GenBank Accession No. CG10272 and AAF54122), mouse (GenBank Accession No. XP_125730), *Anopheles gambiae* (GenBank Accession No. EAA03558), and *Neurospora crassa* (GenBank Accession No. EAA33634) from sequences in public databases (the disclosures of which are incorporated by reference herein in their entireties). The SAM binding domain among these homologs is conserved (approximately 30-100% amino acid sequence identity and 50-100% amino acid similarity [*i.e.,* identical or conserved amino acids]; *see* **Figure 2A**).

The 2.5 A resolution structure of a fragment of the hDOT1L protein containing the catalytic domain (amino acids 1-416) has been solved. The atomic coordinates for amino acids 1-416 of hDOT1L have been determined and deposited in the RCSB database under ID code 1 NW3 (*see also,* Min, et al. (2003) Cell 112:711-723), the disclosures of which are incorporated herein by reference in their entireties.

The present invention will now be described with reference to the accompanying drawings, in which preferred embodiments of the invention are shown. This invention can be embodied in different forms and should not be construed as limited to the embodiments set forth herein. Rather, these embodiments are provided so that this disclosure will be thorough and complete, and will fully convey the scope of the invention to those skilled in the art. For example, features illustrated with respect to one embodiment can be incorporated into other embodiments, and features illustrated with respect to a particular embodiment can be deleted from that embodiment. In addition, numerous variations and additions to the embodiments suggested herein will be apparent to those skilled in the art in light of the instant disclosure, which do not depart from the instant invention.

Unless otherwise defined, all technical and scientific terms used herein have the same meaning as commonly understood by one of ordinary skill in the art to which this invention belongs. The terminology used in the description of the invention herein is for the purpose of describing particular embodiments only and is not intended to be limiting of the invention.

As used in the description of the invention and the appended claims, the singular forms "a," "an" and "the" are intended to include the plural forms as well, unless the context clearly indicates otherwise.

All publications, patent applications, patents, and other references mentioned herein are incorporated by reference in their entirety.

Except as otherwise indicated, standard methods can be used for the production of recombinant and synthetic polypeptides, fusion proteins, antibodies or antigen-binding fragments thereof, manipulation of nucleic acid sequences, production of transformed cells, and the like according to the present invention. Such techniques are known to those skilled in the art. *See, e.g.,* SAMBROOK et al., MOLECULAR CLONING: A LABORATORY MANUAL 2nd Ed. (Cold Spring Harbor, NY, 1989); F. M. AUSUBEL et al. CURRENT PROTOCOLS IN MOLECULAR BIOLOGY (Green Publishing Associates, Inc. and John Wiley & Sons, Inc., New York).

### I. DOT1L Polypeptides and Nucleic Acids.

The present invention describes DOT1L polypeptides. As used herein, the term "polypeptide" includes both proteins and peptides. The term "DOT1L polypeptide" is intended to encompass the DOT1L polypeptides specifically described herein (*e.g*., **SEQ ID NO:2**) as well as functional equivalents thereof that have substantival similar amino acid sequences (as described below) to the DOT1L polypeptides specifically described herein (*e.g*., **SEQ ID NO:2**) and have one or more of the functional properties (also discussed below) of the DOT1L polypeptides specifically described herein. The term "DOT1L polypeptide" also encompasses functional fragments of the full-length DOT1L polypeptides specifically disclosed herein (*e.g*., **SEQ ID NO:2**) and functional equivalents thereof that have substantially similar amino acid sequences to a fragment of a full-length DOT1L polypeptide specifically disclosed herein (*e.g*., a fragment of **SEQ ID NO:2**) and have one or more of the functional properties of the DOT1L polypeptides specifically disclosed herein.

By "functional" it is meant that the DOT1L polypeptide has the same or substantially similar H3-K79 HMTase activity, SAM binding activity, histone and/or nucleosome binding activity, AF10 binding activity, AF10-MLL or other MLL fusion protein binding activity, leukemogenic activity and/or any other biological activity of interest as compared with a full-length DO1L polypeptide (*e.g*., **SEQ ID NO:2**). To illustrate, in representative embodiments, a functionally equivalent DOT1L polypeptide or functional DOT1L fragment has at least about 50%, 75%, 85%, 90%, 95% or 98% or more of the relevant biological activity(ies) as the DOT1L polypeptide of **SEQ ID NO:2.**
Methods of assessing DOT1L binding to histones, nucleosome, nucleic acids or polypeptides can be carried out using standard techniques that will be apparent to those skilled in the art (*see* the Examples for exemplary methods). Such methods include yeast and mammalian two-hybrid assays and co-immunoprecipitation techniques.

Other biological activities associated with DOT1L such as H3-K79 HMTase and leukemogenic activity can also be evaluated using standard methods known in the art, for example, as described in the Examples below.

In particular embodiments of the invention, the DOT1L polypeptide has H3-K79 specific HMTase activity. By H3-K79 "specific" HMTase activity it is meant that all, or essentially all, of the HMTase activity is directed to H3-K79 (*e.g*., using a histone or nucleosome substrate).

In particular embodiments of the invention, the functionally equivalent DOT1L polypeptide or functional DOT1L fragment has the same or substantially similar H3-K79 HMTase activity as the DOT1L polypeptide of **SEQ ID NO:2** or the catalytically active fragment of amino acids 1-416 of **SEQ ID NO:2.** Methods of evaluating HMTase activity are known in the art, and are described in the Examples below. In representative embodiments, the functionally equivalent DOT1L polypeptide or functional DOT1L fragment has at least about 50%, 75%, 85%, 90%, 95% or 98% or more of the H3-K79 HMTase activity as the DOT1 L polypeptide of **SEQ ID NO:2** or the catalytically active fragment of amino acids 1-416 of **SEQ ID NO:2.**

An "isolated" polypeptide as used herein means a polypeptide that is separated or substantially free from at least some of the other components of the naturally occurring organism or virus, for example, the cell or viral structural components or other polypeptides or nucleic acids commonly found associated with the polypeptide. In particular embodiments, the "isolated" polypeptide is at least about 1%, 5%, 10%, 25%, 50%, 60%, 70%, 75%, 80%, 85%, 90%, 95%, 97%, 98%, 99% or more pure (w/w). In other embodiments, an "isolated" polypeptide indicates that at least about a 5-fold, 10-fold, 25-fold, 100-fold, 1000-fold, 10,000-fold, or more enrichment of the protein (w/w) is achieved as compared with the starting material.

The DOT1L polypeptides described herein can be derived from any species of interest (*e.g*., mammalian [including but not limited to human, non-human primate, mouse, rat, lagomorph, bovine, ovine, caprine, porcine, equine, feline, canine, *etc*.], insect, yeast, avian, plants, *etc*.) as well as allelic variations, isoforms, splice variants and the like. The DOT1L sequences can further be wholly or partially synthetic.

In particular embodiments, the DOT1L polypeptide comprises, consists essentially of, or consists of an isolated DOT1L polypeptide of **SEQ ID NO:2** or a functional fragment or functional equivalent thereof.

Functional equivalents of the DOT1L polypeptides encompass those that have substantial amino acid sequence similarity, for example, at least about 60%, 70%, 75%, 80%, 85%, 90%, 95%, 97% or more amino acid sequence similarity with the amino acid sequences specifically disclosed herein (*e.g*., **SEQ ID NO:2)** or a functional fragment thereof. Alternatively, nucleic acids encoding the DOT1L polypeptides have at least about 60%, 70%, 75%, 80%, 85%, 90%, 95%, 97% or more nucleotide sequence similarity with the nucleotide sequences encoding the DOT1L polypeptides specifically disclosed herein (*e.g*., **SEQ ID NO:1)** or a fragment thereof and encode a functional DOT1L polypeptide.

In alternate embodiments, nucleic acids encoding the DOT1L polypeptides have substantial nucleotide sequence similarity with the DOT1L nucleic acids specifically disclosed herein (*e.g*., **SEQ ID NO:1**) or fragments thereof and hybridize to the nucleic acid sequences disclosed herein (*e.g*., **SEQ ID NO:1**) or fragments thereof under standard conditions as known by those skilled in the art and encode a functional DOT1L polypeptide (including functional fragments).

For example, hybridization of such sequences may be carried out under conditions of reduced stringency, medium stringency or even stringent conditions (*e.g*., conditions represented by a wash stringency of 35-40% Formamide with 5x Denhardt's solution, 0.5% SDS and 1x SSPE at 37°C; conditions represented by a wash stringency of 40-45% Formamide with 5x Denhardt's solution, 0.5% SDS, and 1x SSPE at 42°C; and conditions represented by a wash stringency of 50% Formamide with 5x Denhardt's solution, 0.5% SDS and 1x SSPE at 42°C, respectively) to the nucleic acids encoding the DOT1L polypeptides (including functional fragments thereof) disclosed herein. *See, e.g.,* Sambrook et al., Molecular Cloning, A Laboratory Manual (2d Ed. 1989) (Cold Spring Harbor Laboratory).

Further, it will be appreciated by those skilled in the art that there can be variability in the nucleic acids that encode the DOT1L polypeptides due to the degeneracy of the genetic code. The degeneracy of the genetic code, which allows different nucleic acid sequences to code for the same protein, is well known in the art.

Likewise, those skilled in the art will appreciate that the methods of the present invention also encompasses fusion proteins (and nucleic acid sequences encoding the same) comprising the DOT1L polypeptides (including functional fragments). For example, it may be useful to express the DOT1L polypeptides as a fusion protein that can be recognized by a commercially available antibody (*e.g*., FLAG motifs) or as a fusion protein that can otherwise be more easily purified (*e.g*., by addition of a poly-His tail). Additionally, fusion proteins that enhance the stability of the protein may be produced, *e.g*., fusion proteins comprising maltose binding protein (MBP) or glutathione-S-transferase. As another alternative, the fusion protein can comprise a reporter molecule. In other particular embodiments, the DOT1 L fusion protein is a DOT1L-MLL fusion (*see*, *e.g*., **Example 8**). DOT1L fusion proteins can also be generated for use in yeast two-hybrid systems (*e.g*., GAL4-DOT1 L fusions), as known in the art.

It will further be understood that the DOT1L polypeptides specifically disclosed herein will typically tolerate substitutions in the amino acid sequence and substantially retain biological activity. To routinely identify polypeptides other than those specifically disclosed herein, amino acid substitutions may be based on any characteristic known in the art, including the relative similarity or differences of the amino acid side-chain substituents, for example, their hydrophobicity, hydrophilicity, charge, size, and the like. In particular embodiments, conservative substitutions (*i.e*., substitution with an amino acid residue having similar properties) are made in the amino acid sequence encoding the DOT1L polypeptide.

In making amino acid substitutions, the hydropathic index of amino acids can be considered. The importance of the hydropathic amino acid index in conferring interactive biologic function on a protein is generally understood in the art (*see*, Kyte and Doolittle, (1982) J. Mol. Biol. 157:105; incorporated herein by reference in its entirety). It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like.

Each amino acid has been assigned a hydropathic index on the basis of its hydrophobicity and charge characteristics (Kyte and Doolittle, *Id.),* and these are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

It is also understood in the art that the substitution of amino acids can be made on the basis of hydrophilicity. U.S. Patent No. 4,554,101 (incorporated herein by reference in its entirety) states that the greatest local average hydrophilicity of a protein, as governed by the hydrophilicity of its adjacent amino acids, correlates with a biological property of the protein.

As detailed in U.S. Patent No. 4,554,101, the following hydrophilicity values have been assigned to amino acid residues: arginine (+3.0); lysine (±3.0); aspartate (+3.0 **±** 1); glutamate (+3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4).

As is known in the art, a number of different programs can be used to identify whether a nucleic acid or polypeptide has sequence identity or similarity to a known sequence. One example of a useful algorithm is the BLAST algorithm, described in Altschul et al., J. Mol. Biol. 215, 403-410, (1990) and Karlin et al., Proc. Natl. Acad. Sci. USA 90, 5873-5787 (1993). A particularly useful BLAST program is the WU-BLAST-2 program which was obtained from Altschul et al., Methods in Enzymology, 266, 460-480 (1996); hftp://blast.wustl/edu/blast/ README.html. WU-BLAST-2 uses several search parameters, which are preferably set to the default values. The parameters are dynamic values and are established by the program itself depending upon the composition of the particular sequence and composition of the particular database against which the sequence of interest is being searched; however, the values may be adjusted to increase sensitivity.

An additional useful algorithm is gapped BLAST as reported by Altschul et al., (1997) Nucleic Acids Res. 25, 3389-3402.

The CLUSTAL program can also be used to determine sequence similarity. This algorithm is described by Higgins et al. (1988) Gene 73:237; Higgins et al. (1989) CABIOS 5:151-153; Corpet et al. (1988) Nucleic Acids Res. 16: 10881-90; Huang et al. (1992) CABIOS 8: 155-65; and Pearson et al. (1994) Meth. Mol. Biol. 24: 307-331.

The DOT1L polypeptides also encompass functional DOT1L polypeptide fragments (*e.g*., having HMTase activity, having SAM binding activity, histone or nucleosome binding activity, AF10 binding activity, AF10-MLL or any other MLL fusion protein binding activity, leukemogenic activity and/or any other biological activity of interest), and functional equivalents thereof, The length of the DOT1L fragment is not critical. Illustrative functional DOT1L protein fragments comprise at least about 80, 100, 200, 300, 400, 500, 600, 7Q0, 800, 1000, 1200, 1400 or more amino acids (optionally, contiguous amino acids) of a DOT1L polypeptide. The invention also describes nucleic acids encoding the functional DOT1L fragments. Exemplary nucleic acids encoding functional DOT1L fragments comprise at least about 250, 300, 400, 500, 600, 700, 800, 1000, 1500, 2000, 2500, 3000, 4000 or more nucleotide bases (optionally, contiguous bases) of a nucleic acid encoding a full-length DOT1L polypeptide.

In particular embodiments, the invention describes functional DOT1L fragments comprising the catalytic domain comprising the SAM binding domain (as well as adjacent sequences) and nucleic acids encoding the same. In representative embodiments, the functional DOT1L fragment comprises a catalytically active fragment comprising amino acids 157 to 270 of **SEQ ID NO:2** or a functional equivalent thereof. In other embodiments, the DOT1L fragment comprises a catalytically active fragment comprising amino acids 121-306 or amino acids 1-416 of **SEQ ID NO:2** or a functional equivalent of either of the foregoing.

The functional DOT1L fragment comprising the catalytic domain can optionally further comprise the DOT1L positively charged region (*e.g*., amino acids 390 to 407 of **SEQ ID NO:2** or a functional equivalent thereof).

In other embodiments, the functional fragment comprises the DOT1L leucine rich interaction domain with AF10 (*e.g*., amino acids 500-630 of **SEQ ID NO:2** or a functional equivalent thereof). As a further embodiment, the functional fragment comprises the leucine zipper region at amino acids 564-590 of **SEQ ID NO:2** and/or the coiled coil region at amino acids 561-660 of **SEQ ID NO:2** or functional equivalents of either of the foregoing.

In still other embodiments, the invention describes a functional DOT1L fragment comprising a nuclear export signal. In illustrative embodiments, the nuclear export signal comprises amino acids 482-496 of **SEQ ID NO:2** or a functional equivalent thereof, amino acids 600-635 of **SEQ ID NO:2** or a functional equivalent thereof and/or amino acids 636-650 of **SEQ ID NO:2** or a functional equivalent thereof. In other embodiments, the nuclear export signal comprises amino acids 472-767 of **SEQ ID NO:2** or a functional fragment thereof.

Those skilled in the art will appreciate that functional DOT1L fragments can comprise two or more of the functional regions discussed above.

In yet a further embodiment, the functional fragment comprises the N-terminal portion of a DOT1 polypeptide (e.g., **SEQ ID NO:2**), for example, approximately the N-terminal 10, 20, 30, 40, 50, 60, 70, 80, 90 or 100 amino acids. In other embodiments, the functional fragment is truncated at the N-terminus, e.g., less than about 100, 85, 75, 60, 50, 35, 20, 15, 10 or 5 amino acids are truncated from the N-terminus.

The invention also describes isolated nucleic acids encoding the DOT1 L polypeptides. The nucleic acid can be DNA, RNA or chimeras thereof, single stranded or double-stranded, and can be fully or partially synthetic or naturally occurring. The nucleic acids can comprise modified nucleotide or nucleotide analogs as are well-known in the art. Further, the nucleic acid can be from any species of origin, including but not limited to mammalian species such as human, non-human primate, mouse, rat, rabbit, cattle, goat, sheep, horse, pig, dog, cat, *etc*. and avian species.

In particular embodiments, the nucleic acid is an isolated nucleic acid. As used herein, an "isolated" nucleic acid means a nucleic acid separated or substantially free from at least some of the other components of the naturally occurring organism, such as for example, the cell structural components or other polypeptides or nucleic acids commonly found associated with the nucleic acid.

In representative embodiments, the invention describes an isolated DOT1L polypeptide comprising an amino acid sequence selected from the group consisting of: (a) the amino acid sequence of **SEQ ID NO:2**; (b) an amino acid sequence having at least about 60%, 70%, 75%, 80%, 85%, 90%, 95%, 97% or more amino acid sequence similarity with the amino acid sequence of **SEQ ID NO:2.** wherein the DOT1L polypeptide has H3-K79 methyltransferase activity; and (c) a functional fragment of at least about 100, 200, 300, 400, 500, 600, 700, 800, 1000, 1200, 1400 or more amino acids of the amino acid sequence of (a) or (b) above, wherein the functional fragment comprises a DOT1L histone methyltransferase catalytic domain and has H3-K79 methyttransferase activity.

In other illustrative embodiments, the invention describes an isolated nucleic acid comprising a nucleotide sequence encoding a DOT1L polypeptide, the nucleotide sequence selected from the group consisting of: (a) a nucleotide sequence comprising the nucleotide sequence of **SEQ ID NO:1**; (b) a nucleotide sequence that hybridizes to the complete complement of a nucleotide sequence comprising the nucleotide sequence of **SEQ ID NO:1** under stringent conditions (as defined above), wherein the nucleotide sequence encodes a polypeptide having H3-K79 methyltransferase activity; (c) a nucleotide sequence having at least about 60%, 70%, 75%, 80%, 85%, 90%, 95%, 97% or more nucleotide sequence similarity with the nucleotide sequence of **SEQ ID NO:1**, wherein the nucleotide sequence encodes a polypeptide having H3-K79 methyltransferase activity; (d) a nucleotide sequence that differs from the nucleotide sequence of (a) above due to the degeneracy of the genetic code; (f) a nucleotide sequence comprising a functional fragment of **SEQ ID NO:1,** wherein the functional fragment comprises at least the coding region of the histone methyltransferase catalytic domain of **SEQ ID NO:1** or a nucleotide sequence that hybridizes to the complete complement coding region of the histone methyltransferase catalytic domain of **SEQ ID NO:1** under stringent conditions, and wherein the functional fragment encodes a polypeptide having H3-K79 methyltransferase activity; (g) a nucleotide sequence comprising a functional fragment of **SEQ ID NO:1**, wherein the functional fragment comprises at least the coding region of the histone methyltransferase catalytic domain of **SEQ ID NO:1** or a nucleotide sequence having at least about 60%, 70%, 75%, 80%, 85%, 90%, 95%, 97% or more nucleotide sequence similarity thereto, and wherein the functional fragment encodes a polypeptide having H3-K79 methyltransferase activity; and (h) a nucleotide sequence comprising a functional fragment of **SEQ ID NO:1**, wherein the functional fragment comprises at least the coding region of the histone methyltransferase catalytic domain of **SEQ ID NO:1** or a nucleotide sequence that differs therefrom due to the degeneracy of the genetic code; and wherein the functional fragment encodes a polypeptide having H3-K79 methyltransferase activity.

As yet a further embodiment, the invention describes antisense oligonucleotides and siRNA that hybridize to the DOT1 L nucleic acids of the invention (*e.g*., under stringent hybridization conditions as defined above) and inhibit production of DOT1 L polypeptide. RNAi is a mechanism of posttranscriptional gene silencing in which double-stranded RNA (dsRNA) corresponding to a coding sequence of interest is introduced into a cell or an organism, resulting in degradation of the corresponding mRNA. The RNAi effect persists for multiple cell divisions before gene expression is regained. RNAi is therefore a powerful method for making targeted knockouts or "knockdowns" at the RNA level. RNAi has proven successful in human cells, including human embryonic kidney and HeLa cells (*see*, *e.g*., Elbashir et al., Nature (2001) 411:494-8). In one embodiment, silencing can be induced in mammalian cells by enforcing endogenous expression of RNA hairpins (see Paddison et al., (2002), PNAS USA 99:1443-1448). In another embodiment, transfection of small (*e.g*., 21-23 nt) dsRNA specifically inhibits nucleic acid expression (reviewed in Caplen, (2002) Trends in Biotechnology 20:49-51).

The mechanism by which RNAi achieves gene silencing has been reviewed in Sharp et al, (2001) Genes Dev 15: 485-490; and Hammond et al., (2001) Nature Rev Gen 2:110-119).

RNAi technology utilizes standard molecular biology methods. To illustrate, dsRNA corresponding to all or a part of a target coding sequence to be inactivated can be produced by standard methods, *e.g*., by simultaneous transcription of both strands of a template DNA (corresponding to the target sequence) with T7 RNA polymerase. Kits for production of dsRNA for use in RNAi are available commercially, *e.g*., from New England Biolabs, Inc. Methods of transfection of dsRNA or plasmids engineered to make dsRNA are routine in the art.

Silencing effects similar to those produced by RNAi have been reported in mammalian cells with transfection of a mRNA-cDNA hybrid construct (Lin et al., (2001) Biochem Biophys Res Commun 281:639-44), providing yet another strategy for silencing a coding sequence of interest.

In particular embodiments, the invention describes antisense oligonucleotides and siRNA that have at least about 60%, 70%, 80%, 85%, 90%, 95%, 98%, 99% or 100% sequence identity with the DOT1 L nucleic acids described above. Methods of making and using antisense oligonucleotides and siRNA are known in the art. There is no particular upper or lower limit to the antisense oligonucleotide or siRNA. Illustrative antisense oligonucleotides or siRNA will be about 8, 10, 12, 15, 18, 20, 25, 30, 40, 50, 60, 75, 90, 100, 125 or 150 or more bases in length.

The invention also describes vectors, including expression vectors and gene delivery vectors, comprising the nucleic acids of the invention. Suitable vectors include bacterial expression vectors, fungal expression vectors, mammalian vectors, yeast expression vectors and plant expression vectors. Exemplary vectors include bacterial artificial chromosomes, cosmids, yeast artificial chromosomes, phage, plasmids, lipid vectors and viral vectors (*e.g*., adenovirus, adeno-associated virus, retrovirus, baculovirus, and the like).

Expression vectors can be designed for expression of polypeptides in prokaryotic or eukaryotic cells. For example, polypeptides can be expressed in bacterial cells such as *E*. *coli,* yeast cells, insect cells (*e.g*., in the baculovirus expression system) or mammalian cells. Some suitable host cells are discussed further in Goeddel, Gene Expression Technology: Methods in Enzymology 185, Academic Press, San Diego, Calif. (1990). Examples of vectors for expression in yeast S. *cerevisiae* include pYepSecl (Baldari et al., (1987) EMBO J. 6:229-234), pMFa (Kurjan and Herskowitz, (1982) Cell 30:933-943), pJRY88 (Schultz et al., (1987) Gene 54:113-123), and pYES2 (Invitrogen Corporation, San Diego, Calif.). Baculovirus vectors available for expression of nucleic acids to produce proteins in cultured insect cells (*e.g*., Sf 9 cells) include the pAc series (Smith et al., (1983) Mol. Cell. Biol. 3:2156-2165) and the pVL series (Lucklow, V.A., and Summers, M.d. (1989) Virology 170:31-39).

Examples of mammalian expression vectors include pCDM8 (Seed, (1987) Nature 329:840) and pMT2PC (Kaufman et al. (1987), EMBO J. 6:187-195).

The vector generally comprises an expression control element (*e.g*., a promoter) operably associated with the nucleic acids of the invention. It will be appreciated that a variety of expression control elements can be used depending on the level and tissue-specific expression desired. Further, the promoter can be constitutive or inducible (*e.g*., the metalothionein promoter or a hormone inducible promoter). The expression control element can be native or foreign to the host cell and can be a natural or a synthetic sequence. The promoter is generally chosen so that it will function in the target cell(s) of interest. The nucleic acids can further be associated with other appropriate expression control sequences, *e.g*., transcription/translation control signals and polyadenylation signals. Viral regulatory elements are often employed in mammalian cells. For example, commonly used promoters in mammalian expression vectors are derived from polyoma, adenovirus 2, cytomegalovirus and Simian Virus 40.

Moreover, specific initiation signals are generally required for efficient translation of inserted protein coding sequences. These translational control sequences, which can include the ATG initiation codon and adjacent sequences, can be of a variety of origins, both natural and synthetic. Further provided are host cells (*e.g*., yeast, bacterial, mammalian, insect, plant or fungal cells) comprising the isolated nucleic acids and vectors of the invention. The cell can be transiently or stably transformed with the nucleic acid or vector of the invention. In particular embodiments, a host cell is transiently or stably (*e.g*., by stable integration into the genome of the cell or by a stably maintained episome) transformed with a nucleic acid of the invention.

Further, the cell can be cultured (*i.e*., isolated) or can be a cell *in situ* in a living organism.

Also described in the invention is a transgenic non-human animal (*e.g*., non-human mammal) comprising a recombinant nucleic acid encoding a DOT1L polypeptide or a functional fragment thereof. In alternative embodiments, the transgenic non-human animal comprises a recombinant nucleic acid encoding an antisense oligonucleotide or siRNA that inhibits production of DOT1L polypeptide. Generally, the transgenic non-human animal will be stably transformed with the nucleic acid, *e.g*., by stable integration into the genome of the animal, The transgenic non-human animal can be of any species of interest including but not limited to mouse, rat, guinea pig, rabbit, pig, horse, goat, sheep, cow, cat, dog, monkey, hamster, chicken and the like. Methods of making transgenic non-human animals are known in the art.

As yet a further embodiment, the invention describes antibodies and antibody fragments that specifically bind to the DOT1 L polypeptides

The term "antibody" or "antibodies" as used herein refers to all types of immunoglobulins, including IgG, IgM, IgA, IgD, and IgE. The antibody can be monoclonal or polyclonal and can be of any species of origin, including (for example) mouse, rat, rabbit, horse, goat, sheep or human, or can be a chimeric antibody. *See, e.g.,* Walker et al., Molec. Immunol. 26, 403-11 (1989). The antibodies can be recombinant monoclonal antibodies produced according to the methods disclosed in U.S. Patent No. 4,474,893 or U.S. Patent No. 4,816,567. The antibodies can also be chemically constructed according to the method disclosed in U.S. Patent No. 4,676,980.

Antibody fragments included within the scope of the present invention include, for example, Fab, F(ab')2, and Fc fragments, and the corresponding fragments obtained from antibodies other than IgG. Such fragments can be produced by known techniques. For example, F(ab')2 fragments can be produced by pepsin digestion of the antibody molecule, and Fab fragments can be generated by reducing the disulfide bridges of the F(ab')2 fragments. Alternatively, Fab expression libraries can be constructed to allow rapid and easy identification of monoclonal Fab fragments with the desired specificity (Huse et al., (1989) Science 254, 1275-1281).

Monoclonal antibodies used to carry out the present invention can be produced in a hybridoma cell line according to the technique of Kohler and Milstein, (1975) Nature 265,495-97. For example, a solution containing the appropriate antigen can be injected into a mouse and, after a sufficient time, the mouse sacrificed and spleen cells obtained. The spleen cells are then immortalized by fusing them with myeloma cells or with lymphoma cells, typically in the presence of polyethylene glycol, to produce hybridoma cells. The hybridoma cells are then grown in a suitable medium and the supernatant screened for monoclonal antibodies having the desired specificity. Monoclonal Fab fragments can be produced in *E*. *coli* by recombinant techniques known to those skilled in the art. *See, e.g.,* W. Huse, (1989) Science 246, 1275-81.

Antibodies specific to the target polypeptide can also be obtained by phage display techniques known in the art.

### Methods of Use.

The DOT1L polypeptides and nucleic acids described in the invention can be used in a number of research, diagnostic and/or therapeutic applications.

To illustrate, the DOT1L polypeptides and nucleic acids can be used to screen for compounds that bind to and/or modulate (*e.g*., increase or decrease) one or more biological activities of DOT1L, including but not limited to H3-K79 HMTase activity, SAM binding activity, histone and/or nucleosome binding activity, AF10 binding activity, AF10-MLL or other MLL fusion protein binding activity, leukemogenic activity and/or any other biological activity of interest. As described above, the DOT1L polypeptide can be a functional fragment of a full-length DO1L polypeptide or functional equivalent thereof and can comprise any DOT1 domain of interest, including but not limited to the catalytic domain, the SAM binding domain and/or the positively charged domain, the AF10 interaction domain and/or a nuclear export signal.

In particular embodiments, the compound enhances, elevates, increases (or similar terms) one or more biological activities of DOT1L (*e.g*., by at least about 25%, 50%, 75%, 2-fold, 3-fold, 5-fold, 10-fold, 15-fold, 20-fold or more). In other embodiments, the compound is an inhibitor that reduces, inhibits, decreases (or similar terms) of one or more biological activities of DOT1L (*e.g.,* by at least about 25%, 40%, 50%, 60%, 75%, 80%, 85%, 90%, 95%, 98% or more), In particular embodiments, an inhibitory compound results in no detectable level of the DOT1L biological activity(ies) of interest.

In one particular embodiment, the invention describes a method of identifying a compound that modulates (*e.g*., inhibits or enhances) DOT1L polypeptide binding to a histone or nucleosome substrate comprising H3, the method comprising: contacting a DOT1L polypeptide with a histone nucleosome substrate comprising H3 in the presence of a test compound; detecting the binding of the DOT1L polypeptide to the substrate under conditions sufficient for binding of the DOT1L polypeptide to the substrate, wherein an elevation or reduction in DOT1L polypeptide binding to the substrate in the presence of the test compound as compared with the level of binding in the absence of the test compound indicates that the test compound modulates binding of DOT1L polypeptide to a histone or nucleosome substrate comprising H3.

In another embodiment, the invention describes a method of identifying a compound that modulates a biological activity (as described above) of a DOT1L polypeptide, the method comprising: contacting a DOT1L polypeptide with a histone or nucleosome substrate comprising H3 in the presence of a test compound; detecting the level of the DOT1L activity of interest under conditions sufficient for the DOT1L polypeptide to exhibit the relevant biological activity, wherein an elevation or reduction in the DOT1L activity in the presence of the test compound as compared with the level in the absence of the test compound indicates that the test compound modulates DOT1L biological activity.

In a further embodiment, the invention describes a method of identifying a compound that modulates DOT1L H3-K79 HMTase activity, the method comprising: contacting a DOT1L polypeptide with a histone or nucleosome substrate comprising H3 in the presence of a test compound; detecting the level of H3-K79 methylation of the histone or nucleosome substrate under conditions sufficient to provide H3-K79 methylation, wherein an elevation or reduction in H3-K79 methylation in the presence of the test compound as compared with the level of histone H3-K79 methylation in the absence of the test compound indicates that the test compound modulates DOT1L H3-K79 HMTase activity.

The inventors have discovered that DOT1L has a number of binding partners including AF10 and MLL fusion proteins (*e.g*., AF10-MLL), which are implicated in leukemogenesis (described in more detail below). Thus, , the invention may provide a method of identifying a compound that inhibits binding of DOT1 L polypeptide to a second polypeptide, the method comprising: contacting a DOT1 L polypeptide of the invention with a second polypeptide in the presence of a test compound; detecting the level of binding between DOT1 L polypeptide and the second polypeptide under conditions sufficient for binding of DOT1 L polypeptide to the second polypeptide, wherein a reduction in binding between DOT1 L polypeptide and the second polypeptide in the presence of the test compound as compared with the level of binding in the absence of the test compound indicates that the test compound inhibits binding of DOT1L polypeptide to the second polypeptide.

In particular embodiments, the second polypeptide is AF10 (including AF10 fragments comprising the OM-LZ domain, *e.g*., amino acids 719-800 of AF10 [human AF10 sequence, Accession No. AY598745; mouse AF10 sequence, Accession No. 054826]; the disclosures of which are incorporated herein by reference in their entireties). In other representative embodiments, the second polypeptide is an MLL fusion protein (*e.g.*, an MLL-AF10 fusion protein or an MLL-ENL, MLL-ELL or MLL-CBP fusion protein).

In other particular embodiments, the DOT1L polypeptide (including functional fragments) comprises amino acids 500 to 630 of SEQ ID NO:2 or an amino acid sequence having at least 95% amino acid sequence similarity thereto.

The present invention further provides methods of identifying compounds for the treatment and/or prevention of leukemia by identifying compounds that bind to and/or modulate the biological activity (as described above) of a DOT1 polypeptide.

In one embodiment, the invention provides a method of identifying a candidate compound for the prevention and/or treatment of leukemia, the method comprising: contacting a DOT1 L polypeptide of the invention with a histone or nucleosome substrate comprising histone H3 in the presence of a test compound; detecting the level of H3-K79 methylation of the substrate under conditions sufficient to provide H3-K79 methylation, wherein an inhibition of H3-K79 methylation in the presence of the test compound as compared with the level of H3-K79 methylation in the absence of the test compound indicates that the test compound is a candidate compound for the prevention and/or treatment of leukemia.

The invention further provides a method of identifying a candidate compound for the prevention and/or treatment of leukemia, comprising: contacting a DOT1L polypeptide of the invention with AF10 or an MLL fusion protein; detecting the level of binding between the DOT1L polypeptide and AF10 or MLL fusion protein under conditions sufficient for binding therebetween, wherein a reduction in binding between DOT1L polypeptide and AF10 or the MLL fusion protein in the presence of the test compound as compared with the level of binding in the absence of the test compound indicates that the test compound is a candidate compound for the prevention and/or treatment of leukemia.

In particular embodiments, the MLL fusion protein is an MLL-AF10 fusion protein or an MLL-ENL, MLL-ELL or MLL-CBP fusion protein.

In other representative embodiments, the DOT1L polypeptide (including functional fragments) comprises the DOTIL AF1 0 interaction domain, *e.g*., amino acids 500-630 of **SEQ ID NO:2** or a functional equivalent thereof. Alternatively, DOT1 L polypeptides comprising the leucine zipper region at amino acids 564-590 of **SEQ ID NO:2** and/or the coiled coil region at amino acids 561 to 660 of SEQ **ID NO:2** or functional equivalents of either of the foregoing can be used as targets to identify compounds that disrupt interactions between DOT1 L and AF10 or an MLL fusion protein.

By the terms "treating leukemia" or "treatment of leukemia", it is intended that the severity of the disease is reduced. By the term "prevention of leukemia" or "preventing leukemia" it is intended that the methods at least partially eliminate or reduce the incidence or onset of leukemia. Alternatively stated, by treating or preventing leukemia (or grammatical variations thereof), it is meant that the methods and compounds of the invention slow, control, decrease the likelihood or probability, or delay the onset of leukemia in the subject.

Methods of assessing DOT1L binding to histones, nucleosomes, nucleic acids or polypeptides can be carried out using standard techniques that will be apparent to those skilled in the art (*see* the Examples for exemplary methods). Such methods include yeast and mammalian two-hybrid assays and co-immunoprecipitation techniques.

Other biological activities associated with DOT1 L such as H3-K79 HMTase and leukemogenic activity can also be evaluated using standard methods known in the art, for example, as described in the Examples below.

The screening methods of the invention can be carried out in a cell-based or cell-free system. As a further alternative, the assay can be performed in a whole animal (including transgenic non-human animals). Further, with respect to cell-based systems, the DOT1 L polypeptide (or any other polypeptide used in the assay) can be added directly to the cell or can be produced from a nucleic acid in the cell. The nucleic acid can be endogenous to the cell or can be foreign (*e.g*., a genetically modified cell).

Any compound of interest can be screened according to the present invention. Suitable test compounds include small organic compounds (*i.e*., non-oligomers), oligomers or combinations thereof, and inorganic molecules. Suitable organic molecules can include but are not limited to polypeptides (including enzymes, antibodies and Fab' fragments), carbohydrates, lipids, coenzymes, and nucleic acid molecules (including DNA, RNA and chimerics and analogs thereof) and nucleotides and nucleotide analogs. In particular embodiments, the compound is an antisense nucleic acid, an siRNA or a ribozyme that inhibits production of DOT1 L polypeptide.

Small organic compounds (or "non-oligomers") include a wide variety of organic molecules, such as heterocyclics, aromatics, alicyclics, aliphatics and combinations thereof, comprising steroids, antibiotics, enzyme inhibitors, ligands, hormones, drugs, alkaloids, opioids, terpenes, porphyrins, toxins, catalysts, as well as combinations thereof.

Oligomers include oligopeptides, oligonucleotides, oligosaccharides, polylipids, polyesters, polyamides, polyurethanes, polyureas, polyethers, and poly (phosphorus derivatives), *e.g*. phosphates, phosphonates, phosphoramides, phosphonamides, phosphites, phosphinamides, *etc*., poly (sulfur derivatives) *e.g*., sulfones, sulfonates, sulfites, sulfonamides, sulfenamides, *etc*., where for the phosphorous and sulfur derivatives the indicated heteroatom for the most part will be bonded to C,H,N,O or S, and combinations thereof. Such oligomers may be obtained from combinatorial libraries in accordance with known techniques.

Further, the methods of the invention can be practiced to screen a compound library, *e.g.,* a combinatorial chemical compound library (*e.g.,* benzodiazepine libraries as described in U.S. Patent No. 5,288,514; phosphonate ester libraries as described in U.S. Patent No. 5,420,328, pyrrolidine libraries as described in U.S. Patent Nos. 5,525,735 and 5,525,734, and diketopiperazine and diketomorpholine libraries as described in U.S. Patent No. 5,817,751), a polypeptide library, a cDNA library, a library of antisense nucleic acids, and the like, or an arrayed collection of compounds such as polypeptide and nucleic acid arrays.

As still a further aspect, the invention encompasses diagnostic methods for leukemia and/or prognostic methods for predicting the future course of leukemia in a subject by assessing H3-K79 histone methylation, wherein an elevation in H3-K79 methylation as compared with a normal (*e.g.,* non-leukemic) subject is diagnostic of leukemia and/or prognostic of the course of the disease. In particular embodiments, the pattern of histone H3 methylation is assessed, *e.g.,* the level of H3-K79 methylation and the level of H3-K4 methylation are determined (*e.g.,* at a particular location of the HoxA9 gene), wherein an elevation in H3-K79 methylation and/or a reduction in H3-K4 methylation as compared with a normal (*e.g.,* non-leukemic) subject is diagnostic of leukemia and/or prognostic of the course of the disease. This embodiment of the invention can be practiced with any mammalian subject including but not limited to human, non-human primate, cattle, sheep, goat, cat, dog, pig, horse, rat, mouse, rabbit or guinea pig subjects. In particular embodiments, the subject has or is believed to be at risk of developing leukemia. In other embodiments, the subject is an animal model of leukemia.

A "diagnostic method", as used herein, refers to a screening procedure that is carried out to identify those subjects that are affected with a particular disorder. A "prognostic method" refers to a method used to help predict, at least in part, the course of a disease (*e.g.,* more aggressive or less aggressive).
Alternatively stated, a prognostic method may be used to assess the severity of the disease. For example, the screening procedure disclosed herein may be carried out to both identify an affected individual, to evaluate the severity of the disease, and/or to predict the future course of the disease. Such methods may be useful in evaluating the necessity for therapeutic treatment, what type of treatment to implement, and the like. In addition, a prognostic method may be carried out on a subject previously diagnosed with a particular disorder when it is desired to gain greater insight into how the disease will progress for that particular subject (*e.g*., the likelihood that a particular patient will respond favorably to a particular drug treatment, or when it is desired to classify or separate patients into distinct and different sub-populations for the purpose of conducting a clinical trial thereon).

It will be appreciated by those skilled in the art that the diagnostic and prognostic methods of the invention may not be conclusive and may need to be supplemented with other diagnostic and/or prognostic methods to make a final diagnosis or prognosis.

One exemplary method of diagnosing whether a subject has or is at risk for developing leukemia and/or determining the prognosis for the course of the disease comprises: obtaining a biological sample comprising nucleosomes from a subject; detecting the level of H3-K79 methylation in the biological sample; wherein an elevation in H3-K79 methylation in the biological sample as compared with the level of H3-K79 methylation in a non-leukemic biological sample is diagnostic that the subject has or is at risk of developing leukemia and/or prognostic of the course of the disease in the subject. The method can optionally further include determination of the level of H3-K4 methylation, wherein a decrease in H3-K4 methylation in the biological sample as compared with a non-leukemic sample is diagnostic that the subject has or is at risk of developing leukemia and/or prognostic of the course of the disease in the subject.

According to another representative embodiment, the invention provides a method of diagnosing whether a subject has or is at risk for developing leukemia and/or determining the prognosis for the course of the disease, the method comprising: obtaining a biological sample comprising nucleosomes from a subject; detecting the level of H3-K79 methylation associated with one or more HoxA genes (*e.g.,* the HoxA9 gene) in the biological sample; wherein an elevation in HoxA gene-associated H3-K79 methylation (*e.g.,* associated with the HoxA9 gene) in the biological sample as compared with the level in a non-leukemic biological sample is diagnostic that the subject has or is at risk of developing leukemia and/or prognostic of the course of the disease in the subject. The method can optionally further include determination of the level of H3-K4 methylation associated with one or more HoxA genes (*e.g.,* the HoxA9 gene), wherein a decrease in HoxA gene associated H3-K4 methylation in the biological sample as compared with a non-leukemic sample is diagnostic that the subject has or is at risk of developing leukemia and/or prognostic of the course of the disease in the subject.

In exemplary embodiments, an "elevation" or "increase" (or similar terms) in methylation (*e.g.,* H3-K79 methylation) is at least about a 25%, 50%, 75%, 2-fold, 3-fold, 5-fold, 10-fold, 15-fold, 20-fold or more increase in methylation as compared with the level of methylation in a non-leukemic biological sample.

In other representative embodiments, a "decrease" or "reduction" (or similar terms) in methylation (*e.g.,* H3-K4 methylation) is at least about a 25%, 40%, 50%, 60%, 75%, 80%, 85%, 90%, 95%, 98% or more decrease in methylation as compared with the level of methylation in a non-leukemic biological sample. In particular embodiments, no methylation is detectable.

Any suitable biological sample can be used including cell or tissue samples, umbilical cord samples, blood, plasma or serum samples, urine or fecal samples, mucus or sputum samples, and the like. In particular embodiments, the biological sample is a B cell or bone marrow sample. In other representative embodiments, the biological sample is a histone or nucleosome preparation comprising histone H3 (*e.g.,* obtained from B cells or bone marrow cells). In representative embodiments, cells or tissue are removed from a subject, cultured and nucleosomes prepared from the cultured cells or tissue.

By "non-leukemic biological sample" it is meant a suitable control sample that is indicative of a normal subject (*i.e.,* not having or at risk for developing leukemia). For example, the sample can be isolated from a normal subject or, in some instances (*e.g*., a nucleosome preparation), can be isolated from cultured cells.

As further uses of the present invention, the DOT1 L nucleic acids and polypeptides can be used in methods of methylating histones (H3) in a cell free system, in cultured cells or *in vivo.* In particular embodiments, the substrate is a histone substrate comprising H3. In other embodiments, the substrate is a nucleosome substrate comprising H3. Other uses of the DOT1 L nucleic acids and DOT1 L polypeptides as well as antisense and siRNA molecules that inhibit DOT1 L polypeptide production include modulation of telomeric silencing, regulation of gene expression and/or cell cycle regulation.

Having described the present invention, the same will be explained in greater detail in the following examples, which are included herein for illustration purposes only, and which are not intended to be limiting to the invention.

### EXAMPLE 1

### Experimental Procedures

### HMTase-Mediated Methylation of H3-Lysine 79

*Antibodies, hDOT1L Constructs and Protein Preparation.* The methyl-K79-specific antibody was raised by injection of rabbits with a synthetic peptide coding for amino acids 73-83 of histone H3 with K79 di-methylated (Ile-Ala-Gln-Asp-Phe-mLys-Thr-Asp-Leu-Arg-Phe; **SEQ ID NO:4**). The methyl-K4, -K9 antibodies were purchased from Upstate Biotechnology (Lake Placid, NY). The full-length hDOT1L was derived from two over-lapping EST clones BF507396 and BF982417. The FLAG^{®}-tagged constructs were cloned into the *Eco*RI/*Xho*I sites of a pcDNA-derived vector by PCR. The GST-fusion of the hDOT1L N-terminal fragments were also cloned into the *Eco*RI/*Xho*I sites of pGEX-KG vector by PCR. The mutants were generated through PCR-based mutagenesis. All PCR-generated constructs were verified by sequence analysis. The GST-hDOT1L fusion proteins were purified as according to standard methods (Wang, et al. (2001) Mol. Ce// 8:1207-1217). The recombinant proteins were then cleaved using thrombin following manufacturer's instructions. Proteins were quantified by COOMASSIE^{®} staining.

*Cell Synchronization, Labeling and Flow Cytometry.* To obtain cells synchronized at the G1/S boundary, HeLa cells were treated with 2 mM thymidine (SIGMA, St. Louis, MO) for 18 hours, followed by a 9 hour release in thymidine-free medium, and then treated again with 2 mM thymidine for 17 hours to arrest cells at the beginning of S phase. The synchronized cells were released in fresh medium and harvested every two hours. HeLa cells synchronized at the mitotic stage were prepared by blocking with 2 mM thymidine for 18 hours, releasing for 3 hours, and then incubating with 100 ng/mL nocodazole for 12 hours. The cells were then washed three times with phosphate-buffered saline (PBS) to eliminate nocodazole before release into fresh medium. Cells were harvested every two hours and cell lysates and histones were prepared using well-established methods (Wang, et al. (2001) Science 293:853-857). The cell cycle position of the cells collected at different stages was determined by propidium iodide (PI) staining. For simultaneous measurement of DNA content and levels of specific proteins (Mi2 and mK79), asynchronized HeLa cells were fixed by ice-cold ethanol before treatment with 0.25% TRITON^{®} in PBS for 5 minutes. Subsequently, the cells were labeled with anti-Mi2 or anti-mK79 antibodies followed by fluorescein isothiocyanate (FITC)-conjugated goat anti-rabbit secondary antibody (Jackson ImmunoResearch Laboratories, West Grove, PA). The cells were labeled with PI before performing flow cytometry analysis using stand methods.

### EXAMPLE 2

### Results

### HMTase-Mediated Methylation of H3-Lysine 79

*Identification of H3-K79 as* a *Novel Methylation Site.* Histone methylation has emerged as an important player in regulating gene expression and chromatin function (Jenuwein and Allis (2001) Science 293:1074-1080; Zhang and Reinberg (2001) Genes Dev. 15:2343-2360; Kouzarides (2002) Curr. Opin. Genet. Dev. 12:198-209). Histone methylation occurs on arginine and lysine residues at the N-terminal tails of histones H3 and H4, and is catalyzed by two distinct family of proteins, the PRMT1 and the SET-domain-containing family of proteins (Zhang and Reinberg (2001) Genes Dev. 15:2343-2360). Since the discovery of the first histone lysine methyltransferase (Rea, et al. (2000) Nature 406:593-599), other lysine methyltransferases that methylate histone H3 at lysines 4, 9, 27, and 36 have been reported (Briggs, et al. (2001) Genes Dev. 15:3286-3295; Roguev, et al. (2001) EMBO J. 20:7137-7148; Wang, et al. (2001) Mol. Cell 8:1207-1217; Tachibana, et al. (2001) J. Biol. Chem. 276:25309-25317; Yang, et al. (2002) Oncogene 21:148-152; Nagy, et al. (2002) Proc. Natl. Acad. Sci. USA 99:90-94; Bryk, et al. (2002) Curr. Biol. 12:165-170; Nishioka, et al. (2002) Genes Dev. 16:479-489; Strahl, et al. (2002) Mol. Cell Biol. 22:1298-1306). One common feature of these histone lysine methyltransferases is that they all contain a SET domain that is required for their enzymatic activity. Thus, SET domain is believed to be a signature motif for histone lysine methyltransferase (Zhang and Reinberg (2001) Genes Dev. 15:2343-2360).

Methylation sites are known to be located at the N-terminus of histones H3 and H4 (Jenuwein and Allis (2001) Science 293:1074-1080; Zhang and Reinberg (2001) Genes Dev. 15:2343-2360). Mass spectrometry analysis has been used to identify potential novel modification sites in the histone globular domain (Feng et al., (2002) Current Biology 12:1052-1058; the disclosure of which is incorporated by reference herein it its entirety). These investigations demonstrated that histone H3 from HeLa cells is mono-methylated on K79 (Feng, *et al., Id* at Figure - 1A, panels a-c).

Based on the well-established nucleosome structure (Luger, et al. (1997) Nature 389:251-260), K79 is located in a loop connecting the first and the second alpha helixes (**Figure 1A**). This region is exposed and is adjacent to the interface between H3/H4 tetramer and the H2A/H2B dimer. Although K79 is not directly involved in the formation of the interface, it is in a position capable of influencing the access to the interface, indicating that methylation on K79 may play an important role in regulating the access of protein factors to chromatin.

*H3-K79 Methylation is Conserved from Yeast to Human.* To confirm the MS data and the conservation of K79 methylation in other organisms, we generated a polyclonal antibody against a dimethyl-K79 H3 peptide Ile-Ala-Gln-Asp-Phe-^{m}Lys-Thr-Asp-Leu-Arg-Phe (**SEQ ID NO:4**). To examine the specificity of the antibody, recombinant histone H3 that was either not methylated, or methylated on K4 or K9 by SET7 (Wang, et al. (2001) Mol. Cell 8:1207-1217) or SUV39H1 (Rea, et al. (2000) Nature 406:593-599), respectively, were subjected to COOMASSIE^{®} staining and western blot analysis. As a positive control, equivalent amounts of core histones purified from HeLa cells were also analyzed. Results shown in **Figure 1B** demonstrate that while the antibody recognized histone H3 purified from HeLa cells, it did not recognize unmethylated, or K4-, or K9-methylated H3 (**Figure 1B**, lower panel). In addition, competition with two peptides of identical amino acid sequences with or without di-methylation on K79 demonstrate that only the methylated peptide is capable of abolishing the reactivity of the antibody toward the HeLa histone H3. Thus, it was concluded that the antibody is H3-mK79-specific.

The fact that K79 and adjacent sequences of H3 are conserved in different organisms prompted us to test the possibility of H3-K79 methylation occurs in other organisms. Thus, core histones isolated from chicken, *Drosophila,* and budding yeast were analyzed by western blotting using the H3-mK79-specific antibody. As negative and positive controls, recombinant H3 and HeLa core histones were included in the assay. Results shown in **Figure 1C** demonstrate that H3-K79 methylation occurs in all the organisms analyzed. Thus, it was concluded that H3-K79 methylation is conserved from yeast to human.

*Human DOT1-Like Protein is an H3-K79-Specific Methyltransferase.* Having demonstrated that H3-K79 methylation occurs in yeast, we identified the responsible enzyme in this organism. It was reasoned that deletion of the K79 HMTase should lead to loss or reduction of K79 methylation that could be detected by western blot analysis using the mK79-specific antibody described above. The candidate genes that we screened included the six yeast SET domain-containing proteins (Set 1 to Set 6; a gift from Kevin Struhl and Huck Hui Ng, Harvard Medical School) as well as those that were predicted to encode SAM-binding domain-containing proteins (Niewmierzycka and Clarke (1999) J. Biol. Chem. 274:814-824; Dlakic (2001) Trends Biochem. Sci. 26:405-407). This screen resulted in the identification of DOT1 as the HMTase responsible for K79 methylation in yeast (Ng, et al. (2002) Genes Dev. 16:1518-27).

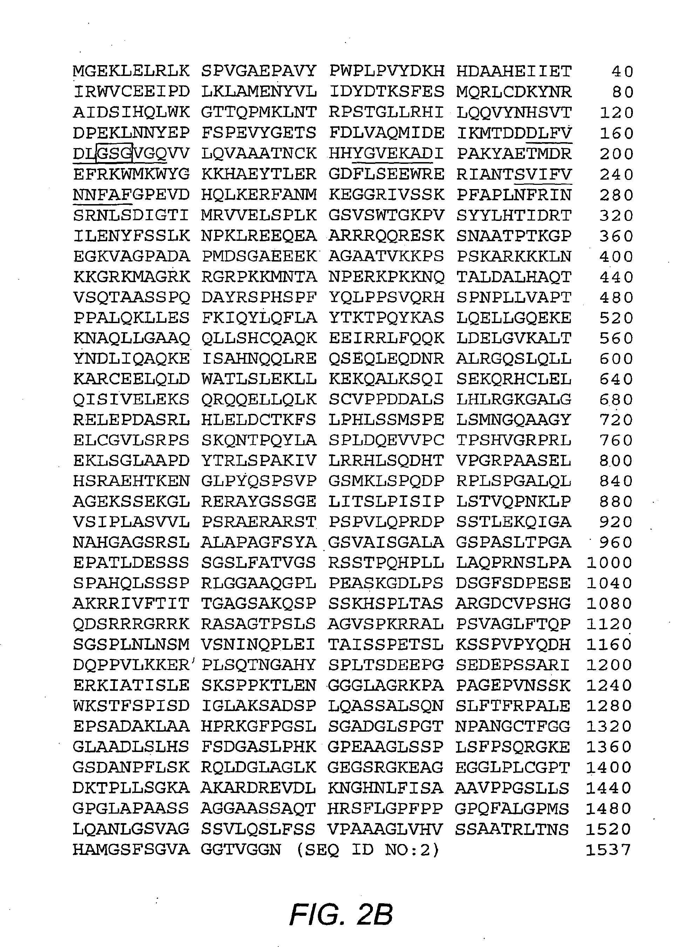

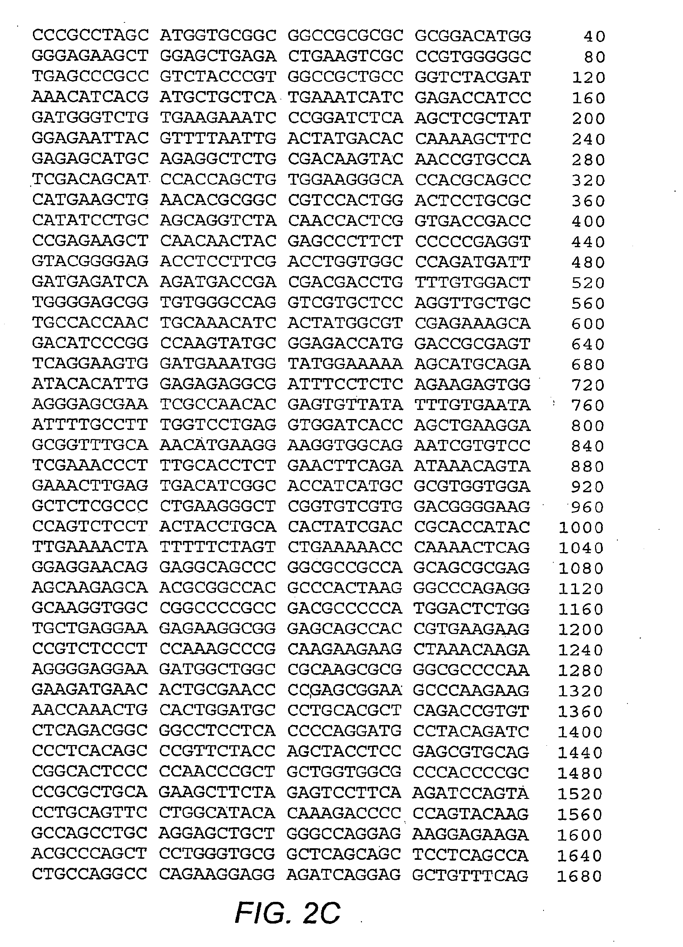

Dot1 was originally identified in a genetic screen for genes whose overexpression disrupts telomeric silencing (Singer, et al. (1998) Genetics 150:613-632). Disruption or overexpression of Dot1 not only impaired telomeric silencing, but also reduced silencing at mating type and rDNA loci (Singer, et al. (1998) Genetics 150:613-632). In addition to participating in silencing, DOT1 also plays an important role in meiotic checkpoint control (San-Segundo and Roeder (2000) Mol. Biol. Ce// 11:3601-3615). Given that K79 methylation was conserved from yeast to human (**Figure 1C**), we expected that DOT1-like (DOT1 L) proteins existed in other organisms. A BLAST search revealed,several putative proteins with significant sequence homology to DOT1. Sequence alignment of these proteins revealed several conserved blocks (**Figure 2A**) that were predicted to be involved in SAM binding (Dlakic (2001) Trends Biochem. Sci. 26:405-407). The BLAST search also revealed that a hypothetical human protein (GENBANK accession number AAC08316) encoded by a gene located on 19p13.3 is likely the human DOT1 L. However, this hypothetical protein was incomplete at both 5' and 3' ends. To clone the full-length cDNA encoding hDOT1L, several EST clones were obtained and sequenced. Two overlapping EST clones (BF507396 and BF982417) contained a single open reading frame (ORF) which was predicted to encode a 1537 amino acid protein with a calculated mass of 165 kDa (**Figure 2B**). The fact that the nucleotide sequence (**Figure 2C-E**) around the first methionine conformed to the Kozak initiator sequence in combination with the fact that the cDNA contained an upstream stop codon indicated that the 1537 amino acids encoded by the cDNA represented the full-length protein. Analysis of the hDOT1 L protein sequences revealed no known functional motif other than a putative SAM-binding domain (**Figure 2B**).

To determine whether hDOT1L possessed intrinsic HMTase activity, two recombinant proteins corresponding to the N-terminal 351 and 472 amino acids of hDOT1L, respectively, were produced in *E*. *coli.* The N-terminus of hDOT1L was selected because this region contained the putative SAM-binding motif and was most conserved among the different DOT1 L proteins (**Figure 2A**). HMTase assays revealed neither protein possessed significant enzymatic activity when free core histones were used as a substrate (**Figure 3A**, lanes 1-3). However, when nucleosomes were used as a substrate, hDOT1L(1-472) exhibited significant HMTase activity while hDOT1L(1-351) was inactive (**Figure 3A**, lanes 4 and 5). To demonstrate that the HMTase activity depended on binding to SAM, we generated a mutated version of hDOT1L(1-472) [hDOT1L(1-472)m] by changing the highly conserved Gly-Ser-Gly₁₆₃₋₁₆₅ sequence of motif I (**Figure 2A**) to Arg-Cys-Arg. This mutation completely abolished the HMTase activity (**Figure 3A**, compare lanes 5 and 6). Taken together, these results demonstrate that hDOT1 L is a nucleosomal H3-specific HMTase and that both the SAM binding motif and the sequences between amino acids 351 and 472 are critical for enzymatic activity.

Having demonstrated the HMTase activity of human DOT1 L *in vitro,* we demonstrated its HMTase activity *in vivo.* Accordingly, mammalian expression vectors encoding a FLAG^{®}-tagged hDOT1 L and a motif I mutant were transfected into 293T cells. Core histones purified from the transfected cells were analyzed by COOMASSIE^{®} and western blot analysis using antibodies specific for methylated K4, K9 or K79. Results shown in **Figure 3B** demonstrate that overexpression of hDOT1L significantly increased H3-K79 methylation while having no effect on K4 and K9 methylation (compare lanes 1 and 2). Increased K79 methylation was dependent on an intact motif I as transfection of a motif I mutant did not affect K79 methylation (**Figure 3B**, compare lanes 1 and 3). This differential effect was not caused by differential expression since both constructs expressed hDOT1L at a similar level (**Figure 3B**, lower two panels). Based on these results, we concluded that hDOT1L is an H3-K79-specific HMTase *in vivo.*

*Methylation on K79 is Regulated During the Cell Cycle.* Total histone methylation level is regulated during the cell cycle (Borun, et al. (1972) J. Biol. Chem. 247:4288-4298). Thus, it was determined whether the level of K79 methylation changed during the cell cycle. To obtain a population of synchronous cells, we arrested HeLa cells at the G1/S border using a double thymidine block. After releasing the arrested cells from the thymidine block, cells were collected every two hours for flow cytometry analysis as well as for the preparation of protein extracts and histones. Flow cytometry analysis (**Figure 4A**) indicated that more than 95% of the cells progress through S phase and enter G2 synchronously. The cells were successfully arrested at the G1/S border before release as evidenced by the accumulation of the histone mRNA stem-loop-binding-protein (SLBP) (**Figure 4B**). As has been demonstrated (Whitfield, et al. (2000) Mol. Cell Biol. 20:4188-4198), SLBP levels stayed high throughout S phase and dropped rapidly as cells exited S phase (**Figure 4B**). The cells completed mitosis about 12 hours after release, as evidenced by the degradation of cyclin A when cells entered anaphase (**Figure 4B**). To determine whether the level of K79 methylation changed during the cell cycle, histones isolated from corresponding cells were subjected to western blot analysis using the mK79-specific antibody. This analysis demonstrated that the level of K79 methylation decreased during S-phase and reached the lowest level in G2 and increased during M-phase (**Figure 4B**).

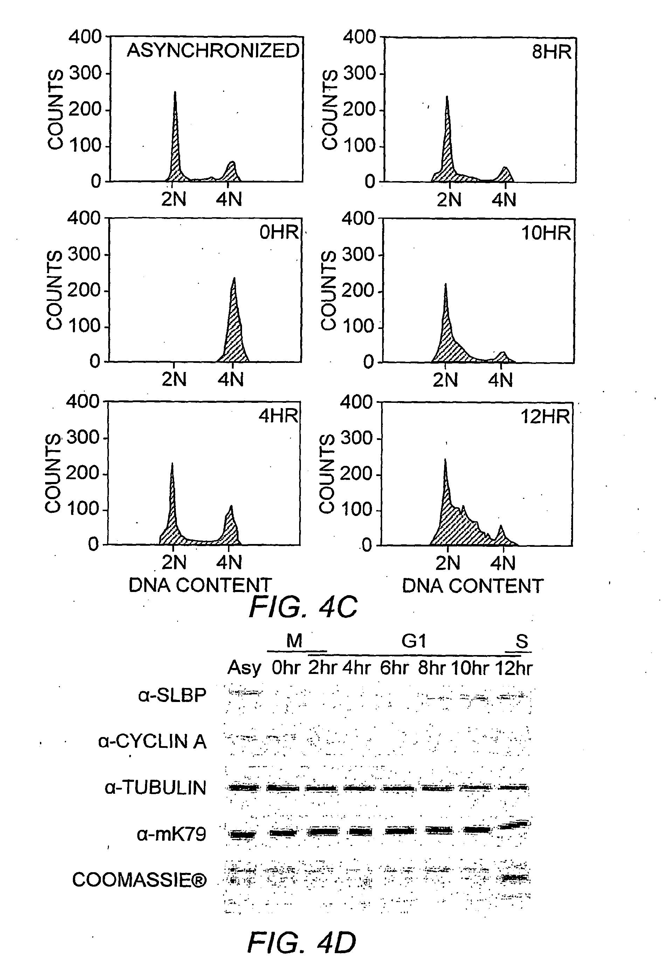

To analyze K79 methylation status in G1 phase, we arrested the cells with thymidine, released them, and then arrested them in mitosis with nocodazole. After release from the nocodazole block, the cells progressed through G1 to S phase synchronously (**Figure 4C**). The cells completed mitosis 2 hours after release from the nocodazole block as evidenced by the degradation of cyclin A (**Figure 4D**). The cells then started to enter S phase about 10 hours after release as evidenced by the accumulation of SLBP (**Figure 4D**). Western blot analysis of the histones from cells collected at different time points after the nocodazole release indicated that the K79 methylation remained at a high level throughout the G1-phase (**Figure 4D**).

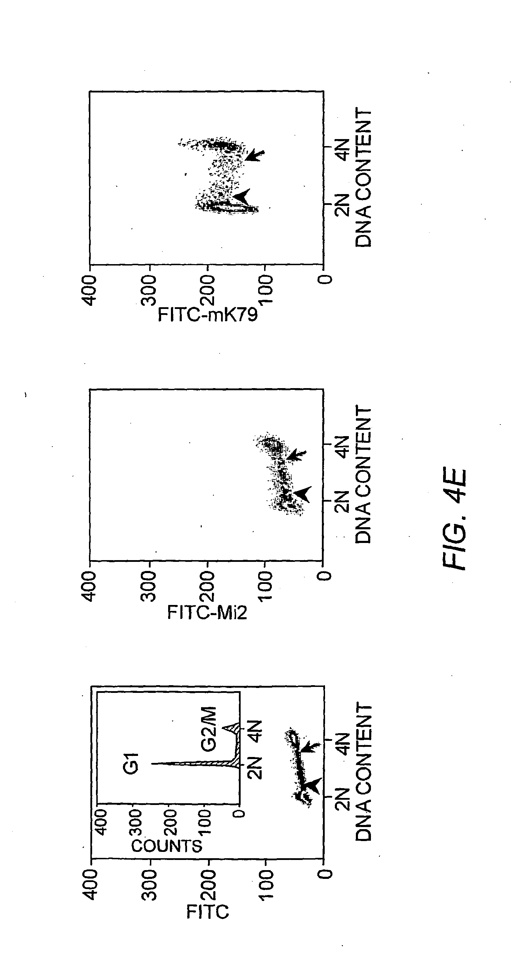

To further confirm the cell cycle-dependent changes in K79 methylation, we performed bivariate analysis of DNA content and mK79 level using a method similar to that used in analyzing cell cycle-dependent changes of cyclins using asynchronous cells (Bonifacino, et al. (1999) Current Protocols in Cell Biology (John Wiley & Sons, Inc.). Book chapter 8. Unit 8.4; Darzynkiewicz, et al. (1999) Determining Cell Cycle Stage by Flow Cytometry (John Wiley & Sons, Inc.), 8.4.1-8.4.18). For each individual cell, DNA was labeled with propidium iodide (red), while K79-methylated-H3 was labeled with an FITC-conjugated secondary antibody (green). The intensities of green color and red color of a particular cell reflected K79 methylation level and DNA content, respectively. Negative controls without a primary antibody or with antibody against Mi-2, a component of a nucleosome remodeling and deacetylase complex (Zhang, et al. (1998) Cell 95:279-289), resulted in a slight increase in FITC signals when cells proceeded from G1-phase to G2/M phase (**Figure 4E****,** left and middle panels). This result was anticipated as cell size becomes bigger during this transition. However, labeling of the cells with the mK79-specific antibody resulted in a decrease in FITC-labeling when cells went through S-phase (**Figure 4E****,** right panel, compare early and late S phases). It is unlikely that the decrease in mK79 level during S-phase was caused by 'dilution' by nascent histones during chromatin assembly as a similar decrease was not observed in other methylation sites. By using two different methods, we demonstrated that the level of K79 methylation decreased during S-phase, reached its lowest level in G2, increased during M-phase, and was maintained at a high level during G1-phase.

### EXAMPLE 3

### Experimental Procedures

### Structure of the Catalytic Domain of Human DOT1L

*Protein Methods.* To produce recombinant hDOT1L fragments containing the N-terminal 351, 370, 375, 385, 416, 452, and 472 amino acids in *E*. *coli,* corresponding cDNA fragments were amplified by PCR and cloned into a pGEX-KG vector (between *Eco*RI*-Xho*l sites). The presence of correct inserts was verified by DNA sequencing. The plasmid expressing the first 416 residues of hDOT1L, pGEX-KG/hDOT1L(1-416), was used for subsequent Δ(376-390) and Δ(390-407) deletions and N241 D, N241A, Y312F and Y312A point mutations. The deletion and point mutations were carried out by PCR and confirmed by sequencing. GST- hDOT1L fusion proteins and mutants were first purified on a glutathione-SEPHAROSE^{®} column. The GST-tag was then removed by thrombin digestion in the column. The eluted proteins were further purified on HITRAP™-SP and SUPERDEX™-75 columns (Amersham Pharmacia Biotech, Piscataway, NJ).

*Histone Methyltransferase Assay.* Histone methyltransferase assay was performed using established methods (Wang, et al. (2001) Science 293:853-857). Oligo-nucleosomes purified from HeLa cells or chicken blood cells were quantified and equal concentrations were used in each assay. Briefly, recombinant hDOT1L proteins (0.75 µM) were incubated with oligo-nucleosomes (1.5 µM) in reactions containing 20 mM Tris-HCl (pH 8.0), 4 mM EDTA, 1 mM PMSF, 0.5 mM DTT, and 0.5 µL ³H-SAM (15 Ci/mM; NEN™ Life Science Products) for 1 hour at 30°C. For peptide competition, 1.5 µM, 15 µM, or 150 µM of positively charged C-RS10 peptide (Cys-Arg-Ser-Arg-Ser-Arg-Ser-Arg-Ser-Arg-Ser-Arg-Ser-Arg-Ser-Arg-Ser-Arg-Ser-Arg-Ser; **SEQ ID NO:20**) were added to reactions. Reactions were stopped by addition of 7 µL of 5 x SDS loading buffer and proteins were separated in an 18% SDS-PAGE. Following stain and destain of COMMASSIE^{®} Blue, gels were treated with Rapid Autoradiography Enhancer (NEN™ Life Science Products, Boston MA) for 40 minutes, dried, and exposed to X-ray films. For quantitation, gel slices were excised and counted with scintillation counting. Two independent experiments were performed at the same time and average ³H (CPM) values were used in the figures.

*Gel Shift Assay.* Different amount of recombinant hDOT1L proteins were incubated with HeLa mono-nucleosomes (1.5 µM) under the same conditions as those in the histone methyltransferase assay, except that SAM is omitted. Following incubation for 1 hour at 30°C, samples were resolved in a 1.8% agarose gel and visualized under UV by ethidium bromide staining.

### EXAMPLE 4

### Results

### Structure of the Catalytic Domain of Human DOT1L

*Disordered C-terminal Region is Important for Enzyme Activity and Nucleosome Binding.* The full-length hDOT1L consists of 1537 amino acids (**Example 2**) but only the N-terminal ∼360 amino acids share significant sequence similarity with yeast Dot1 (**Figure 2A**). We determined that amino acids 1-416 of hDOT1L, hDOT1L(1-416), contain the active HMTase catalytic domain (**Figure 5**).

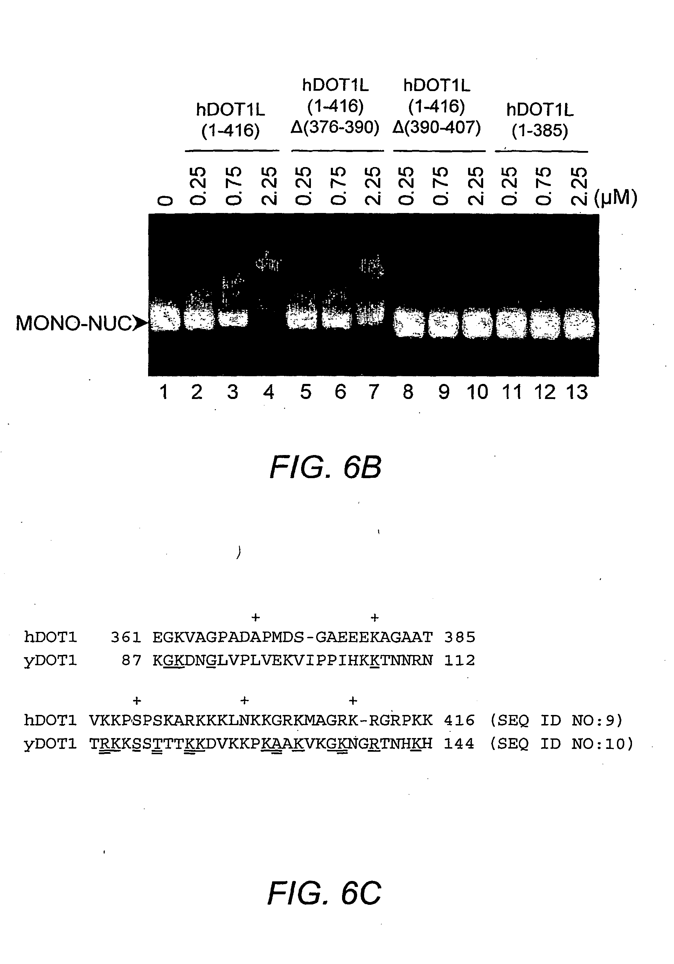

The crystal structure of hDOT1L(1-416) complexed with SAM has been solved to 2.5 A resolution using multiwavelength anomalous diffraction (MAD) of SeMet protein crystals (Min, et al. (2003) Cell 112:711-723; the disclosure of which is incorporated herein by reference). Atomic coordinates for the hDOT1L(1-416) structure have been deposited in the RCSB database under ID code INW3). Amino acids C-terminal to αK (amino acids 333-416) are disordered in the crystal structure. As disclosed above, we demonstrated that certain amino acids located between residue 351 and 416 where important for the enzymatic activity of hDOT1L, as hDOT1L(1-416) could methylate oligo-nucleosomes efficiently but hDot1 (1-351) could not under the same conditions (**Figure 5**). Since the C-terminal end of hDOT1L(1-416) was spatially distant from the SAM binding site and because access to the active site was rather restricted (Min, et al. (2003) Cell 112:711-723), the C-terminus might be important in substrate binding rather than having a direct involvement in catalysis. Accordingly, we tested the enzymatic activity (**Figure 6A**) and nucleosome binding capability (**Figure 6B**) of a series of hDOT1L variants truncated at the disordered C-terminal region.

To identify the elements within the region between amino acids 351 and 416 that were critical for the HTMase activity of hDOT1L, we added back 19, 24 and 34 amino acids to the C-terminus of hDOT1L(1-351), termed hDOT1L(1-370), hDOT1L(1-375) and hDOT1L(1-385), respectively, to determine whether the HMTase activity of hDOT1L could be restored. **Figure 6A** (lanes 2, 3 and 4) shows that all three constructs failed to restore the HMTase activity to a level comparable to that of hDOT1 L(1-416). We then fused the last 26 amino acids of hDOT1L(1-416) to the inactive hDOT1L(1-375), creating an internal deletion' mutant Δ(376-390). **Figure 6A** (lane 6) shows that this mutant was as active as the wild-type hDOT1L(1-416), indicating that amino acids 391-416 were crucial for the HMTase activity. This region of hDOT1L is enriched with positively charged residues (Figure 6C), and these residues may be important for the HMTase activity. Removing a stretch of amino acids containing 9 of the 15 positively charged residues in this region, Δ(390-407), significantly reduced the enzymatic activity (**Figure 6A**, lane 7).

We next examined mono-nucleosome binding of hDot1(1-416) and several of its truncation variants by gel mobility shift assays (GSMA). **Figure 6B** shows that enzymatically active hDOT1L(1-416) and Δ(376-390) caused slower migration of mono-nucleosomes (lanes 2-4 and 5-7), while weakly active Δ(390-407) and inactive hDOT1L(1-385) showed no detectable gel shifts within the sensitivity limit of the assay. The addition of SAM in the GSMA did not change the gel shift pattern in **Figure 6B****.** This observation qualitatively correlated the nucleosome binding ability with the HMTase activity of hDOT1 L proteins and showed the importance of the positively charged C-terminal region in nucleosome binding. Our modeling of hDOT1 L-nucleosome interaction indicated that the C-terminal region was near DNA and the disordered C-terminal positively charged region may be involved in substrate binding by interacting with the negatively charged nucleosomal DNA. Consistent with this, we could detect strong, but apparently non-sequence specific, binding of hDOT1L(1-416) with DNA extracted from mono-nucleosomes. To further probe the nature of hDOT1 L-nucleosome interaction, we used a 21-residue highly charged peptide, C-RS₁₀, containing 10 tandem repeats of arginine and serine as a competitor in our nucleosome binding and HMTase assays. Adding the C-RS₁₀ peptide in GSMA prevented mono-nucleosomes from entering the gel well. The HMTase activities of hDOT1 L(1-416) and Δ(376-390), shown in lanes 1-4 and 5-8 of **Figure 6D****,** respectively, were effectively competed with increasing concentrations of the peptide. These results clearly demonstrate the functional importance of the C-terminal charged region of hDOT1L(1-416).

### EXAMPLE 5

### Experimental Procedures

### Characterization of hDOT1L Localization

*Constructs.* Full-length FLAG^{®}-hDOT1L are described in **Example 1**. For FLAG^{®}-tagged hDOT1 L deletion mutants, PCR-amplified inserts were ligated to p FLAG^{®}-beta-cDNA3 vector digested with *Eco*RI and *Xho*I. When constructing GFP-fused plasmids, all hDOT1 L deletion mutants were constructed by cloning the inserts into the EcoRI and *Kpn*I sites of pEGFP-C3 vector (CLONTECH™, Palo Alto, CA). For fragments larger than 20 amino acids, PCR was used to amplify the inserts. For fragments less than 20 amino acids, DNA were synthesized and annealed before ligation to the vector. For the construction of the hDOT1 L-767-3xNLS construct, hDOT1 cDNA encoding amino acid 1-767 was fused with three tandem repeats of a nuclear localization signal (NLS) derived from SV40 T-antigen (Asp-Pro-Lys-Lys-Lys-Arg-Lys-Val; **SEQ ID NO:21**), and subcloned into pcDNA3b-FLAG^{®} vector. All fragments amplified by PCR and all constructs involving mutagenesis were verified by sequencing.

*Cell Culture, Transfection, and Immunoprecipitation.* 293T or U2OS cell were maintained in Dulbecco's modified Eagle's medium (DMEM) supplemented with 10% fetal calf serum and grown at 37°C, 5%CO₂. Cells were transfected with QIAGEN^{®} EFFECTENE™ Transfection Reagents. Twenty-four hours after transfection, cells were harvested and washed with ice-cold phosphate-buffered saline (PBS) before being lysed with lysis buffer (50 mM Tris, 150 mM NaCl, 0.1% NP-40, 50 mM NaF, 1 mM dithiothreitol, 1 mM phenylmethylsulfonyl fluoride, 1 µg of aprotinin/mL, 0.5 µg of leupeptin/mL, and 0.7 µg of pepstatin/mL). After incubation for 30 minutes at 4°C, the cell debris was removed by centrifugation at 14,000 rpm for 5 minutes in an EPPENDORF^{®} centrifuge (Brinkmann Instruments, Inc., Westbury, NY). About 1 mg of protein extracts was incubated with 20 µL of FLAG^{®}-M2 agarose beads (Sigma, St. Louis, MO) at 4°C for 4 hours. After three washes with lysis buffer containing 300 mM salt, the immunoprecipitated proteins were analyzed by western blot.

*In vivo Ubiquitination Assays.* pcDNA3b-FLAG^{®}-hDOT1L and pcDNA3b-FLAG^{®}-Tat were transfected to 293T cells with or without pHA-Ubiquitin (Ub) by using FUGENE™ 6 transfection reagent (Roche Diagnostics, Indianapolis, IN). Twenty-four hours after transfection, MG132 was added at the final concentration of 25 mM, and the cells were cultured for 4 more hours. Cells were then lysed with lysis buffer (50 mM Tris-HCl, pH 7.5, 0.5 mM EDTA, 1% SDS, and 1 mM DTT), and boiled for 10 minutes before centrifugation at 14,000 rpm for 10 minutes. The supernatant was diluted by10-fold with NP40 lysis buffer (50 mM Tris-HCl, pH 7.5, 150 mM NaCl, 0.1 % NP40, 50 mM NaF, 10% glycerol, 1 mM DTT, and 1 mM PMSF), and incubated with 1.5 mg of FLAG^{®}(M2) antibody for 4 hours at 4°C. Protein G beads were then added and incubated further for one hour. The beads were washed three times with lysis buffer containing 0.5% of NP40 before eluting the protein with SDS sample buffer for SDS-PAGE analysis.

*Immunofluorescence Staining.* U2OS cells (4x10⁴) were seeded in each well of a 12-well plate one day before transfection. 0.3 µg DNA was used for each transfection by QIAGEN^{®} EFFECTENE™ Transfection Reagents. Twenty-four hours after transfection, cells were washed twice with PBS and then fixed in 3% paraformaldehyde (in PBS) for 10 minutes at room temperature. After washing once with PBS, the cells were incubated with 0.2% TRITON^{®} X-1 00 (in PBS) for 5 minutes at 4°C. Then the cells were blocked with 0.5% bovine serum albumin (in PBS) for 30 minutes and stained with mouse anti-FLAG^{®}M2 antibody (IBI, Napa, CA; 1:1,000) or rabbit anti-HA polyclonal antibody (Abcam, Cambridge; MA) for 1 hour at room temperature. After three washes with PBS, the cells were first incubated with rhodamine (or FITC)-conjugated Donkey anti-mouse (or rabbit) immunoglobulin G (IgG) (Jackson ImmunoResearch Laboratories, West Grove, PA) diluted 1:50 with 0.5% bovine serum albumin in PBS for 30 minutes and then stained with 1 µg of 4',6'-diamidino-2-phenylindole (DAPI)/mL (Sigma, St. Louis, MO) for 10 seconds. The cells were then washed three times with PBS, mounted with DAKO fluorescent mounting medium, and visualized by a Zeiss immunofluorescence microscope.

### EXAMPLE 6

### Results

### Characterization of hDOT1L Localization

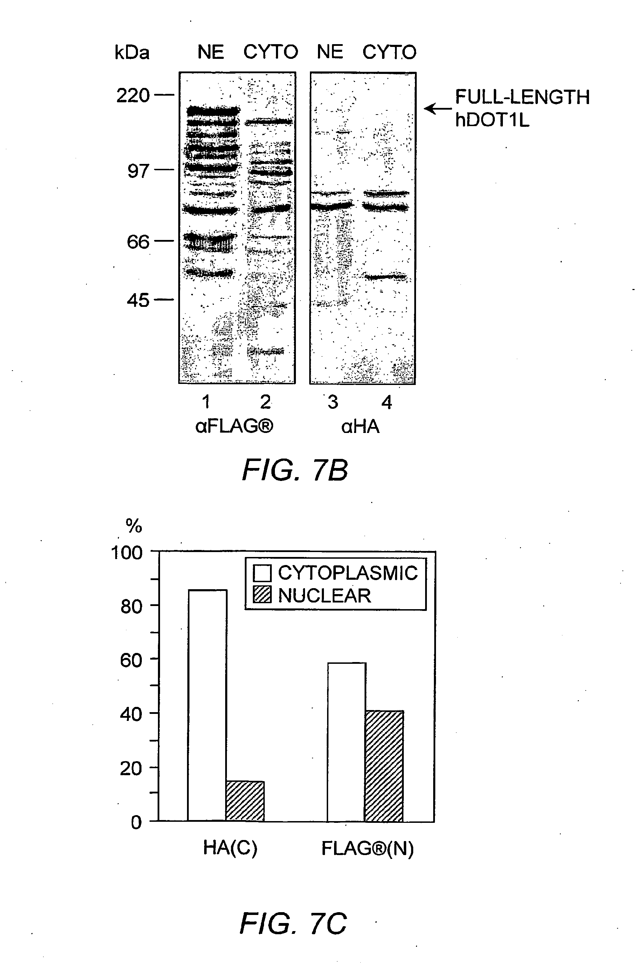

*Rapid in vivo Degradation of hDOT1L.* The *in vivo* function of hDOT1 Lwas analyzed using a rabbit polyclonal antibody generated against an N-terminal fragment (amino acids 1-351) of hDOT1L. The affinity-purified antibody recognized 3 ng of recombinant GST-hDOT1L(1-351) (**Figure 7A**, lane 6) but does not recognize any protein bands around the expected size of about 180 kDa either in nuclear extracts or nuclear pellet proteins derived from HeLa nuclei (**Figure 7A****,** lanes 4 and 5). Although two specific protein bands below and above the 97 kDa marker were detected in the protein fraction derived from nuclear pellet (**Figure 7A**, compare lanes 2 and 5), purification and identification of these proteins indicated that they were not hDOT1 L (data not shown). The fact that we could not detect endogenous hDOT1 L in spite of the extensive effort, indicated that either hDOT1 L existed at very low abundance or it was subjected to rapid degradation in cells. To examine the stability of hDOT1 L, two constructs expressing an N-terminal FLAG^{®}-tagged hDOT1L or a C-terminal HA-tagged hDOT1L were transiently expressed in 293T cells. The cytosol and nuclear extracts derived from the transfected cells were subjected to western blot analysis using FLAG^{®} and HA antibody, respectively. Results shown in **Figure 7B** indicate that hDOT1 L was subjected to rapid degradation as numerous protein bands were recognized by FLAG^{®} antibody, and less than half of the FLAG^{®}-hDOT1L existed in the full-length form although mixtures of proteinase inhibitors were included during the preparation of the protein extracts. Interestingly, almost no full-length FLAG^{®}-hDOT1 L protein was detected in the cytosol while shorter forms of FLAG^{®}-hDOT1 L proteins detected by FLAG^{®} antibody were comparable to that in the nuclear extracts (**Figure 7B**, compare lanes 1 and 2). This indicated that hDOT1 L was more rapidly degraded in the cytosol than in the nucleus. Furthermore, the HA antibody that recognized the C-terminal tag of the recombinant protein detected much fewer protein bands indicating that the C-terminus of the protein was extremely labile.

Subsequently, the stability of the hDOT1 L and its subcellular localization was analyzed using hDOT1 L simultaneously tagged with an N-terminal FLAG^{®} and a C-terminal HA. After transfection, cells were immunostained with antibodies against FLAG^{®} and HA, respectively (Figure 7C). Analysis of the transfected cells indicated that the great majority (>80%) of hDOT1 L containing the C-terminal HA tag was localized to cytosol, while the majority (about 60%) of hDOT1 L containing the N-terminal FLAG^{®} tag was localized to the nucleus. This result is consistent with the observation that full-length hDOT1L is mainly observed in nuclear fractions, while partial hDOT1L protein is enriched in cytosol fraction. This indicates that degradation of hDOT1L may require export of hDOT1L out of the nucleus.

*Ubiquitination of hDOT1L.* Protein ubiquitination is the major pathway for rapid protein degradation (Pickart (2001) Annu. Rev. Biochem 70:503-533). Toward this end, we asked whether hDOT1 L is subjected to ubiquitination. We transfected 293T cells with a construct encoding FLAG^{®}-hDOT1 L with or without co-transfection of a construct encoding HA-tagged ubiquitin. After immunoprecipitation with anti-FLAG^{®} antibody, the immunoprecipitates were subjected to western blot analysis using anti-HA antibodies. To ensure that FLAG^{®}-hDOT1 L was successfully immunoprecipitated, the immunoprecipitates were also blotted with anti-FLAG^{®} antibody (**Figure 8A****,** bottom panels). Results shown in **Figure 8A** indicate that FLAG^{®}-hDOT1L was ubiquitinated in the presence of HA-Ub (top panel, compare lanes 3 and 4). However, the presence of proteasome inhibitor MG132 successfully stabilized ubiquitinated Tat (**Figure 8A****,** top panel, compare lanes 6 and 7), but did not appear to stabilize the ubiquitinated form of FLAG^{®}-hDOT1L (**Figure 8A****,** compare lanes 4 and 5), indicating that hDOT1 L ubiquitination was not directly linked to its stability.

To analyze the potential function of hDOT1 L ubiquitination, hDOT1L ubiquitination sites were mapped using the same approach described above. Different FLAG^{®}-tagged hDOT1L deletion constructs were co-transfected with HA-Ub and the ubiquitination of the different hDOT1L mutants was detected by western blot analysis of HA after immunoprecipitation with the anti-FLAG^{®} antibody. These analyses, summarized in **Figure 8B****,** allowed us to map the ubiquitination sites to a region between amino acids 469-756 of hDOT1L.

As demonstrated below, hDOT1L(1-767) is localized to cytosol. To determine whether ubiquitination of hDOT1 L requires cytoplasm localization, we artificially prevented its cytoplasmic distribution by adding three NLSs to this construct and compared its ubiquitination level with a construct lacking these signals. Immunostaining of transfected cells (data not shown) confirmed that addition of the three NLSs efficiently prevented nuclear export of hDOT1L(1-767). However, western blot analysis of hDOT1L(1-767) with or without the three NLSs indicated that they had similar ubiquitination levels (**Figure 8C****,** compare lanes 5 and 6). Therefore, it was concluded that hDOT1 L ubiquitination can occur both in the cytosol and the nucleus with similar efficiency.

*Control of Cellular Localization of hDOT1 L.* Given that hDOT1L is a nucleosomal histone-specific methyltransferase, we expected that hDOT1 L would function in the nucleus. However, results presented in **Figure 7B** indicated that transfected hDOT1L could localize to both the nucleus and cytoplasm. To further verify this observation, we transfected a plasmid encoding FLAG^{®}-hDOT1 L and visualized the protein localization by immunofluorescent staining using anti-FLAG^{®} antibody (data not shown). These studies confirmed that transiently expressed FLAG^{®}-hDOT1 L protein localized to both the nucleus and the cytoplasm.

Active nucleocytoplasmic transport plays an important role in proper regulation and trafficking of nuclear proteins involved in transcription, DNA replication and chromatin remodeling (Schwoebel and Moore (2000) Essays Biochem. 36:105-113). Many of these nuclear proteins contain both NLS and nuclear export signal (NES) sequences. In contrast to the sequences of NLS that are more conserved and could be predicted by certain software such as PredictNLS, the sequences of NES vary and no accurate prediction is available. However, most NES sequences do share some common features such as a high percentage of Leucine and other hydrophobic residues. Based on sequences of NES first identified from HIV Rev protein and protein kinase A inhibitor (PKI), a generally accepted loose consensus Leu-Xaa(2,3)-[Leu-Ile-Val-Phe-Met]-Xaa(2,3)-Leu-Xaa-[Leu-Ile] (**SEQ ID NO:22**) has been proposed. A NES database has .been constructed and contains all previously identified NESs; only 36% of the NES sequences fit the widely accepted NES consensus (la Cour, et al. (2003) Nucleic Acids Res. 31:393-396).

When the primary sequence of hDOT1 L was examined, two potential NLSs were predicted to exist. One was located at the N-terminus and the other at the C-terminus of hDOT1 L (**Figure 9**). In addition, a putative coiled-coil domain was predicted between amino acid 516-649, which includes a Leucine zipper between amino acids 564-599 (**Figure 9**). Thus, we made three deletion mutant constructs of hDOT1 L and examined their cellular localization by immunostaining. The N-terminal 472 amino acids mainly exhibited nucleolar localization in U2OS cells (data not shown), which is consistent with the presence of a predicted N-terminal NLS. However, the N-terminal 767 amino acids mainly showed cytoplasmic localization; indicating that there was a strong NES between amino acid 472 and 767, which could override the N-terminal NLS signal. The 756-1537 fragment was mainly localized in the nucleus, consistent with the presence of an NLS in this fragment.

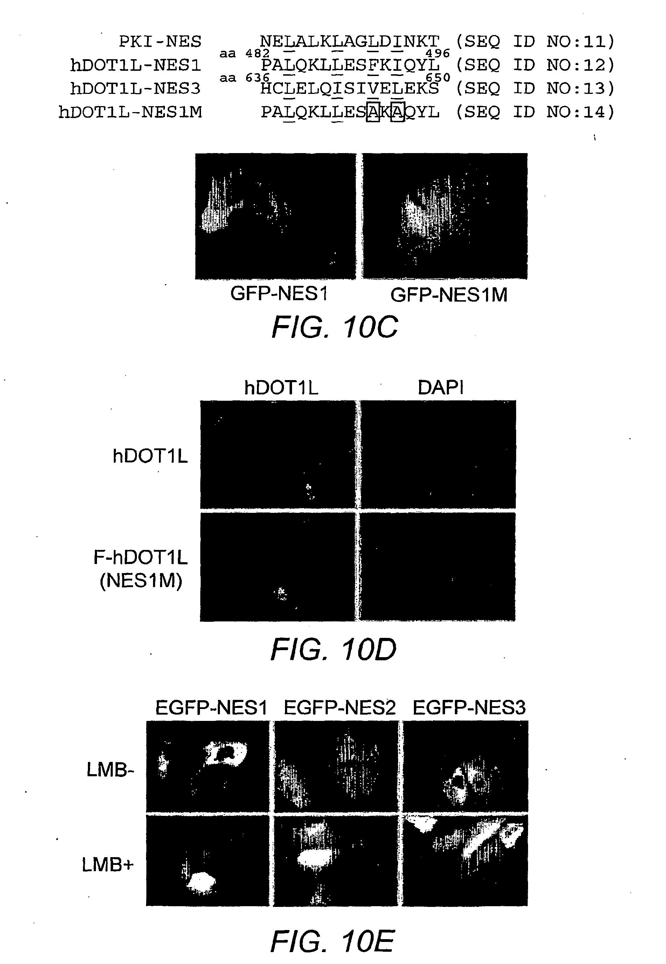

*NESs in hDOT1L.* Data presented above strongly indicated that there was at least one NES present between amino acids 473-767 of hDOT1L. Inspection of the amino acid sequences in this region failed to identify sequences that matched the NES consensus. To identify the NES in this region, a series of deletion mutants of hDOT1 L (469-767) constructs were fused to EGFP (**Figure 10A**). After transfection of these deletion mutants into U2OS cells, their cellular localization was visualized in living cells directly. Results presented in **Figure 10B** indicate that there were three putative NES sequences in this region. The first NES (NES1) resided between amino acids 482 and 496 having the sequence Pro-Ala-Leu-Gln-Lys-Leu-Leu-Glu-Ser-Phe-Lys-Ile-Gln-Tyr-Leu (**SEQ ID NO:12**) (**Figure 10B****,** panel 12). The second NES (NES2) resided between amino acids 600 and 635 (**Figure 10B****,** panel 13) and the third NES (NES3) resided between amino acids 636 and 650 having the sequence of His-Cys-Leu-Glu-Leu-Gly-lle-Ser-lle-Val-Glu-Leu-Glu-Lys-Ser (**SEQ ID NO:13**) (**Figure 10B****,** panel 14). Although all three NESs were able to localize fused EGFP to cytoplasm (**Figure 10B****,** panels 12-14), NES1 and NES3 appeared to be much stronger than NES2. More than 95% of cells transfected with EGFP-NES1 and EGFP-NES3 showed clear nuclear exclusion, whereas about 60% of cells transfected with EGFP-NES2 showed nuclear exclusion, with trace amounts of EGFP still remaining in nuclei. When the sequences of NES1 and NES3 were compared with the NES of the human cAMP-dependent protein kinase inhibitor (PKI), several critical hydrophobic residues were found to be conserved (**Figure 10C**). While most of the conserved amino acids were Leucine or Isoleucine, NES1 and NES3 have Phenylalanine or Valine instead of Leucine at one of the conserved residues. Previous studies indicate that C-terminal hydrophobic residues in NES sequences are more important for nuclear export function than N-terminal Leucines (Zhang and Xiong (2001) Science 292(5523):1910-5). To examine whether this was true for NES1, both the Phenylalanine and the Isoleucine of NES1 were mutated to Alanine. It was found that C-terminal hydrophobic residues were important for NES function as mutations on the two residues completely abrogated the nuclear exclusion ability of NES1 (**Figure 10C**). While the NES1 itself was capable of localizing EGFP to cytoplasm, it was important to demonstrate that NES1 was functional in nuclear exclusion in the context of full-length hDOT1L. Thus, we examined the localization of a full-length hDOT1L NES1 mutant and found that mutation of NES1 prevented cytoplasm localization of hDOT1 L (**Figure 10D**) indicating that NES1 is important for hDOT1L nuclear export.

To further characterize the nuclear export ability of the three NESs identified, it was determined whether the NESs were involved in active nuclear export mediated by the CRM1 (chromosomal region maintenance) pathway. CRM1 was initially identified as *a S. pombe* protein whose mutation affects higher order chromosome structure (Adachi and Yanagida (1989) J. Cell Biol. 108:1195-1207). Subsequent studies indicate that CRM1 is an export receptor for Leucine-rich nuclear export signals (Fornerod, et al. (1997) Cell 90:1051-1060; Ossareh-Nazari, et al. (1997) Science 278:141-144; Stade, et al. (1997) Cell 90:1041-1050). Leptomycin B (LMB), a *Streptomyces* metabolite, has been shown to be capable of preventing CRM1-mediated active nuclear export through covalent attachment to a Cysteine residue in the central region of CRM1 (Kudo, et al. (1998) Exp. Cell Res. 242:540-547; Nishi, et al. (1994) J. Biol. Chem. 269:6320-6324). To investigate the effect of LMB treatment on the nuclear export ability of the NESs, U2OS cells were transfected with constructs encoding each of the three NESs fused with EGFP in the presence or absence of 20 nM of LMB. Eight hours after LMB treatment, green fluorescence was observed under a microscope in living cells. Results shown in **Figure 10E** demonstrate that LMB treatment prevented nuclear export by all the three NESs. These results indicate that hDOT1L nuclear export is mediated through the CRM1 pathway.

### EXAMPLE 7

### Experimental Procedures

### hDOT1L and MLL-AF10-Mediated Leukemogenesis

*Constructs.* For yeast two-hybrid screening, the coding sequence of hDOT1 L was cloned in-frame with the Gal4 DNA-binding domain in the pGBKT7 plasmid. For the mammalian two-hybrid assay (CLONTECH™, Palo Alto, CA), various regions of hDOT1 L were cloned into the pM vector (CLONTECH™) to generate fusions with the Gal4 DNA-binding domain. Various regions of AF10 were cloned into the pVP16 vector (CLONTECH™) to generate fusions with the VP16 transcriptional activation domain. pG5LUC is a reporter vector which contains the luciferase coding region downstream of the minimal promoter of the adenovirus E1b gene and five GAL4 binding sites. For retroviral vector construction, 5' LTR region of murine stem cell virus vector (MSCVneo, CLONTECH™) were replaced by cytomegalovirus immediate early promoter sequences (MSCN). MSCN-MLL(N) and MSCN-MLL-AF10 were constructed from MSCV-5'MLL and MSCV-MLL-AF10 with a FLAG^{®}-tag upstream of MLL gene. MSCN-hDOT1L constructs were constructed by inserting a hDOT1L cDNA (encoding amino acids 1-416 and 1-670, respectively) downstream of MLL gene. MSCB vector was constructed by the replacement of neomycin-resistant gene of MSCN with the blasticidin-resistant gene, and insertion of the full-length of hDOT1 L (encoding amino acids 1-1,537) downstream of a FLAG^{®}-tag.

*Yeast Two-Hybrid Screen.* To identify hDOT1L interacting proteins, yeast two-hybrid screening was performed with the MATCHMAKER™ Gal4 two-hybrid system 3 (CLONTECH™). The cDNA encoding the full-length hDOT1 L was fused in-frame to the GAL4 DNA-binding domain in the bait vector-pGBKT7. This construct was transformed into *Saccharomyces cerevisiae* host strain AH109. A Pre-transformed Mouse Testis MATCHMAKER™ cDNA Library (CLONTECH™) was screened by mating with the bait strain in accordance with the manufacturer's instructions. Approximately 4.8x10⁶ individual clones were screened and about 53 clones grew on the selected medium lacking His, Ade, Trp and Leu. The clones were further selected by growth on SD/-Ade/-His/-Leu/- Trp/X-a-Gal master plates. The prey plasmids were rescued and electroporated into *E*. *coli strain* Top10. The DNA recovered from the bacteria was sequenced to identify the candidate proteins that interact with hDOT1 L.

### Cell Culture, Transfections, Immunoprecipitation and

*Immunofluorescence.* 293T and U2OS cells were maintained in DMEM supplemented with 10% fetal calf serum and grown at 37°C with 5% CO₂. Cell transfection and immunoprecipitations were conducted as described in **Example 5** above.

*Immunofluorescence Staining.* Performed as described in **Example 5** above.

*Retrovirus Preparation and Transduction.* MSCN vector containing MLL-AF10 or MLL-hDOT1L were co-transfected with pGag-pol and pVSVG to human embryonic kidney cells (293T) by a standard calcium-phosphate method. After 48 to 72 hours of transfection, the supernatants were collected and were used for bone marrow cell transduction as follows: 4 to 12-week old C57BL/6 mice were injected intravenously with 5-fluorouracil (150 mg/kg), and bone marrow (BM) cells were harvested from both femurs at 5 days post-injection. Retroviral supernatants were used to transduce BM cells by spinoculation. After two derail infections, infected cells were plated into methylcellulose cultures.

*Methylcellulose ColonyAssays.* Retrovirally-infected BM cells (1x10⁴) were plated in 0.9% methylcellulose (Stem Cell Technologies, Vancouver, BC) supplemented with 10 ng/mL murine IL-3, IL-6, GM-CSF, and 50 ng/mL SCF in the presence of 1mg/mL G418 (GIBCO, Grand Island, NY). After 7 days of culture, the number of colonies was counted and 1x10⁴ cells of the single-cell suspensions prepared from G418-resistant colonies were replated into methylcellulose supplemented with the same growth factors without G418. Further plating was repeated every 10 days. For the experiment in **Figure 15****,** 1x 10⁴ cells were plated with 5 µg/mL of Blasticidin.

*RT-PCR Analysis of Hox Genes.* Total RNA was isolated from primary bone marrow cells or MLL-AF10/MLL-hDOT1 L-transduced cells using RNEASY^{®} (QIAGEN^{®}. One microgram of total RNA was treated with RNase-free DNase I, and applied for reverse transcription using IMPROM-II™ (PROMEGA^{®}, Madison, WI) according to manufacturer's protocol. The resulting cDNA was used as template for PCR amplification of Hox genes using PLATINUM^{®} Taq Polymerase (INVITROGEN™, Carlsbad, CA). Primers sequences were designed based on the known hox gene sequences.

*Mouse Transplantation and Histological Analysis.* Six-week-old non-obese/severe combined immunodeficiency (NOD/SCID) mice were injected intravenously through ophthalmic vein with 10⁶ MLL-hDOT1L(1-670)- or MLL-AF10-transduced BM cells derived from the 3^{rd} round of methylcellulose colonies. For tumor analysis, tumor tissues were fixed in 4% paraformaldehyde. After being paraffin embedding and sectioning, slides were stained with hematoxylin and eosin using standard techniques.

### EXAMPLE 8

### Results

### hDOT1 L and MLL-AF10-Mediated Leukemogenesis

*AF10 Interacts with hDOT1L.* Having cloned and characterized the structure, H3-K79-specific methyltransferase activity, and subcellular localization of hDOT1 L, we searched for functional partners by yeast two-hybrid screening. This screen resulted in repeated isolation of cDNAs encoding the C-terminal half of AF10 (Figure **11A**), a frequent fusion partner of MLL and CALM in leukemia (Chaplin, et al. (1995) Blood 86:2073-2076; Dreyling, et al. (1996) Proc. Natl. Acad. Sci. USA 93:4804-4809). To confirm the interaction, we co-expressed AF10 and hDOT1 L, respectively tagged with FLAG^{®} and HA. Following immunoprecipitation with anti-FLAG^{®} antibody and western blot analysis with anti-HA antibody, we demonstrated that the two proteins can be coimmunoprecipitated (**Figure 11B****,** compare lanes 3 and 6). In addition, the interaction was also confirmed by mammalian two-hybrid assays (**Figure 12** and **Figure 13**)**.** Therefore, AF10 associates with hDOT1L *in vivo.*

*The OM-LZ Region of AF10 is Necessary and Sufficient for the AF10-hDOT1L Interaction.* AF10 was initially discovered by virtue of its involvement in t(10;11)(p12;q23) chromosomal translocations found in acute myeloid leukemia patients (Chaplin, et al. (1995) Blood 86:2073-2076). This genetic rearrangement results in fusion of the C-terminal half of AF10 to the N-terminal third of MLL. AF10 contains several motifs common to transcription factors, and the minimal portion of AF10 fused to MLL contains a leucine zipper (LZ) motif that is highly conserved between homologs of AF10 from C. *elegans* to human (**Figure 12A**). To characterize the nature of the AF10-hDOT1 L interaction, we mapped the region(s) of AF10 involved in the interaction. Mammalian two-hybrid assays were performed in 293T cells. AF10 and hDOT1L, fused to the VP16 activation domain and the Gal4-DBD, respectively, were co-expressed in the presence of a luciferase reporter containing five Gal4 binding sites. Results shown in **Figure 12B** demonstrate that a region of about 80 amino acids (amino acids 719-800) is sufficient to mediate the interaction. Interestingly, this same region has been recently demonstrated to be required for leukemic transformation of the MLL-AF10 fusion protein (DiMartino, et al. (2002) Blood 99:3780-3785). To evaluate whether this 80-amino acid region was necessary for the interaction, we created an AF10 construct where this region was deleted. FLAG^{®}-tagged AF10 with or without this region was co-expressed with hDOT1L-HA and their ability to interact with each other was analyzed by co-immunoprecipitation. Although both constructs were expressed to a similar level (Figure 12C, left panel), deletion of this 80 amino acid region abrogated the ability of AF10 to interact with hDOT1 L (**Figure-12C,** right panel, compare lanes 6 and 9). Therefore, we concluded that the 80-amino acid region was necessary for the AF10-hDOT1L interaction.

In addition to a leucine zipper, this 80-amino acid region also includes an octapeptide motif (OM), (Glu-Gln-Leu-Leu-Glu-Arg-Gln-Trp; **SEQ ID NO:19**), which is highly conserved among AF10 homologs (**Figure 12A**). Previous studies indicate that fusion of OM or LZ alone to MLL is not sufficient for leukemic transformation. However, fusion of MLL to OM+LZ is sufficient for leukemic transformation (DiMartino, et al. (2002) Blood 99:3780-3785). To evaluate the relative contribution of OM and LZ to hDOT1 L interaction, we created constructs harboring mutations in the two regions individually or in combination and analyzed their effect on the AF10-hDOT1L interaction using the mammalian two-hybrid assay described herein. Results shown in **Figure 12D** indicate that while mutation in each of the two regions greatly reduced their interaction, mutation in both regions simultaneously completely destroyed the interaction. Thus, it was concluded that both OM and LZ contributed to the hDOT1L-AF10 interaction. Collectively, these data indicate that the OM-LZ region required for leukemic transformation is both necessary and sufficient for hDOT1 L-AF10 interaction.

*A Leucine-Rich Region Mediates the AF10-hDOT1 L Interaction.* Having defined the region in AF10 required for the AF10-hDOT1L interaction, we mapped the region(s) in hDOT1 L involved in the interaction. Constructs encoding different regions of the hDOT1 L were generated and co-expressed with AF10 in 293T cells. The ability of these hDOT1L deletion mutant proteins to interact with AF10 was analyzed by co-immunoprecipitation. Results shown in **Figure 13A** indicate that a region of 294 amino acids (amino acids 473-766) to be important for the interaction (compare lanes 8 and 10).

To confirm these results and narrow down the interaction region, the mammalian two-hybrid assay described above4 was used. Specifically, a plasmid encoding the VP16 activation domain fused to the OM-LZ region of AF10 was co-expressed with various constructs encoding Gal4DBD fused to different portions of hDOT1 L(472-767). Interaction was measured by activation of a luciferase reporter containing five Gal4 binding sites. Comparison of the activation ability of the first five constructs allowed us to map the interaction domain to a region of 130 amino acids (amino acids 500-630) predicted to form a coiled-coil domain with potential to be a leucine zipper (**Figure 13B****,** 1-5). Further deletion and mutational studies identified two leucine residues (L532 and L592) critical to the interaction (**Figure 13B****,** 6-9). The importance of these two leucine residues in mediating the hDOT1L and AF10 interaction was verified in the context of partial and full-length hDOT1L (**Figure 13B****,** 10-13). Accordingly, it was concluded that a leucine rich region between amino acids 500-630 of hDOT1 L is involved in hDOT1L and AF10 interaction and at least two of the leucine residues (L532 and L592) are critical for the interaction.

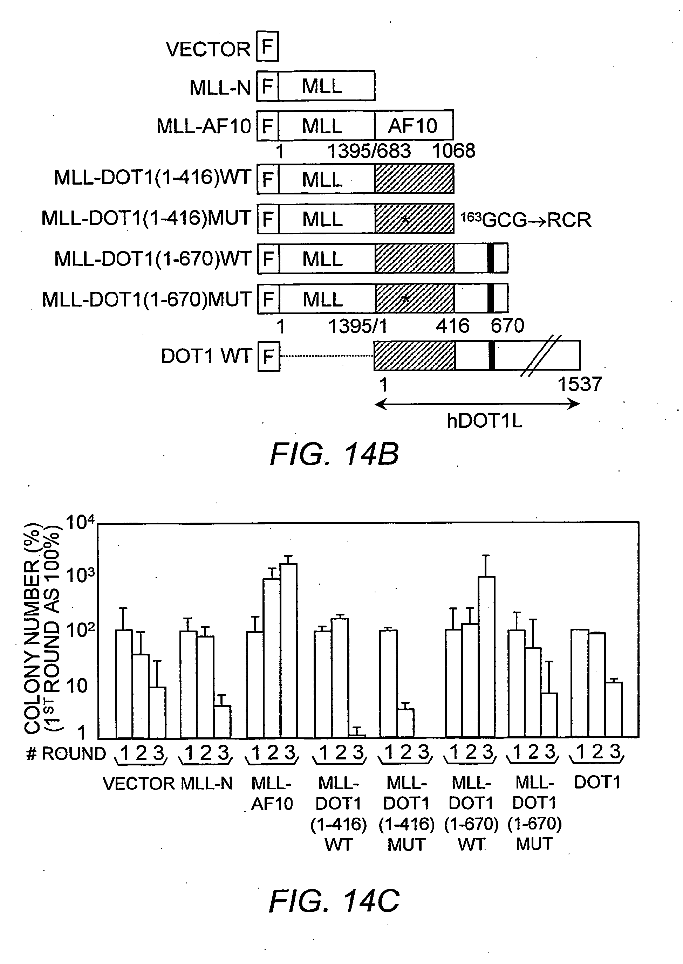

*Immortalization of Primary Murine Myeloid Progenitor Cells by MLL-hDOT1L.* The fact that the OM-LZ domain of AF10 is both necessary and sufficient in mediating AF10 and hDOT1 L interaction in combination with the fact that the same region is required for leukemic transformation (DiMartino, *et al*. (2002) *Blood* **99**:3780-3785) raised the possibility that MLL-AF10-mediated leukemic transformation may involve recruitment of hDOT1 L and its associated HMTase activity to affect expression of MLL target genes. Consistent with this, immunofluorescence staining revealed co-localization of transfected hDOT1 L and MLL-AF10 as large nuclear foci (data not shown). If the fused AF10 in MLL-AF10 only serves to recruit hDOT1 L, leukemic transformation may be achieved by direct fusion of hDOT1 L to MLL. To test this, a methylcellulose serial replating assay, outlined in **Figure 14A****,** was used to assess immortalization of murine myeloid progenitor cells by MLL-hDOT1 L. A modified murine stem cell virus (MSCV)-derived retroviral vector, designated as MSCN, was used to transduce freshly harvested bone marrow cells from mice pretreated with 5-flurouracil. MSCN constructs encoding MLL sequences 5' of the translocation breakpoint with AF1 0 (MLL-N), MLL-AF10 that mimic the most frequent fusion involving the two proteins, MLL fused to various length of hDOT1L, and hDOT1 L were made (**Figure 14B**). To facilitate expression analysis of these constructs, an N-terminal FLAG^{®}-tag was added to each of the constructs. Due to the size limitation for efficient packaging of retroviral vectors, MLL-hDOT1L fusion constructs encoding more than the first 670 amino acids of hDOT1L had dramatically decreased retroviral transduction efficiency (data not shown), which prevented us from analyzing MLL-hDOT1L that contained the full-length hDOT1 L using this assay. Nevertheless, retroviruses with similar titer as that of MLL-AF10 were successfully generated with MLL-hDOT1L(1-416) and MLL-hDOT1L(1-670) (data not shown). As disclosed herein, hDOT1L(1-416) has robust H3-K79 methyltransferase activity *in vitro.* Importantly, transient transfection of MLL-hDOT1 L(1-416) and MLL-hDOT1 L(1-670) into 293T cells resulted in increased H3-K79 methylation *in vivo* (data not shown) indicating that both fusion proteins were enzymatically active. Similar to the full-length hDOT1 L mutant, the two parallel constructs that harbor "GSG163-165RCR" mutations were enzymatically inactive in the same assay (data not shown). Western blot analysis of extracts from transiently-transfected retroviral packaging cells confirmed that MLL-N, MLL-AF10, MLL-hDOT1L(1-416), MLL-hDOOT1L(1-670), and hDOT1 L constructs were efficiently expressed (data not shown). Plating of cells transduced with the various MSCN constructs under selective conditions showed a variable number (50-100) of colonies that were consistent with their respective virus titer. However, significant differences were observed in a second round of plating of 10⁴ cells pooled from colonies harvested from the first round of cultures. Compared to the first round, vector alone, MLL-N, hDOT1 L, and the two MLL-hDOT1L mutants transduced cultures produced a decreased number of secondary colonies. In contrast, MLL-hDOT1 L(1-416), MLL-hDOT1L(1-670) and MLL-AF10 transduced cells gave rise to hundreds of colonies, an amount significantly higher than that from the first round of plating. When a third round of plating was assayed, only MLL-hDOT1L(1-670) and MLL-AF10 transduced cells gave rise to an increased number of colonies compared with that from the second round of plating. It was therefore concluded that similar to MLL-AF10, MLL-hDOT1L(1-670) has leukemic transformation capability. This ability depends on both the hDOT1 L H3-K79 methyltransferase activity and the region between amino acid 417-670.

*HMTase Activity of hDOT1L is Required to Maintain the Growth Capacity of MLL-AF10- Transformed Cells.* To further demonstrate that the enzymatic activity of hDOT1 L is critical in MLL-AF10-mediated leukemogenesis, we examined the effect of the wild-type hDOT1 L and the HMTase-defective mutant hDOT1 L on the sustaining growth capability of MLL-AF10-transduced bone marrow cells. Nucleic acid sequences encoding full-length wild-type and HMTase-defective mutant hDOT1L proteins were cloned into the retroviral vector pMSCB which expresses the Blasticidin resistant gene. Viruses generated using this vector were used to infect MLL-AF10-transduced bone marrow cells derived from the third round of plating (**Figure 15A**). Cells expressing hDOT1 L were selected in the presence of Blasticidin. After 10 days of methylcellulose culture, we observed a dramatic difference in the colony formation assay (**Figure 15B**). While transduction of wild-type hDOT1 L into MLL-AF10 expressing-cells still supported colony formation, transduction of HMTase-defective hDOT1L into MLL-AF10 expressing-cells completely eliminated the ability of MLL-AF10 to support colony formation. These results support the finding that MLL-AF10's ability to sustain the growth of transformed bone marrow cells is dependent upon the HMTase activity of hDOT1 L. It also indicates that the HMTase-defective hDOT1L protein can function as a dominant-negative factor in maintaining the growth capacity of MLL-AF10 transformed cells.

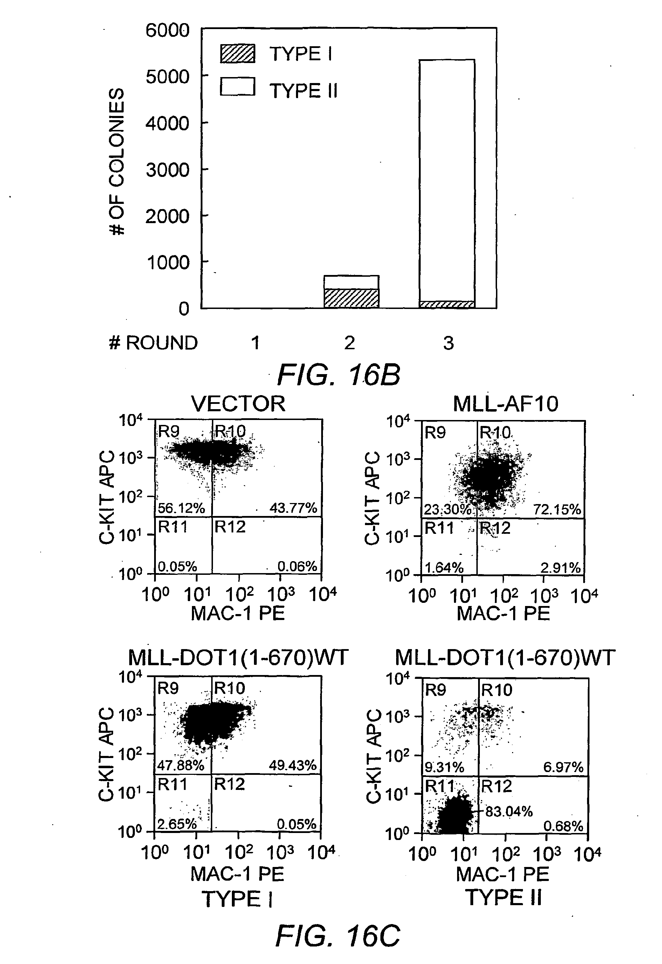

*Immortalization of Murine Myeloid Progenitors by MLL-hDOT1L.* While MLL-hDOT1 L(1-670) is capable of transforming bone marrow cells, the number of colonies formed is different from that of MLL-AF10 (**Figure 14C**). In addition, the morphology of the colonies formed also appeared to be different (**Figure 16A**). Similar to a previous report (DiMartino, et al. (2002) Blood 99:3780-3785), colonies arising from MLL-AF10-transduced bone marrow cells exhibited round, compact morphology (**Figure 16A**). In contrast, MLL-hDOT1L(1-670)-transformed bone marrow cells form colonies with two types of morphologies that were different from that of MLL-AF10 transformed cells (**Figure 16A**). Interestingly, the cells of type II colonies from MLL-DOT1 (1-670)-transformed cells have a significant growth advantage relative to that of type I colonies in the third round of plating (**Figure 16B**). To determine the cell lineage of these colonies, immunophenotyping of these cells in early liquid cultures was performed. Consistent with myeloid cell lineage, many cells derived from MLL-AF10-transformed colonies or the type I MLL-hDOT1 L-transformed colonies expressed the early myeloid markers Mac-1 and c-Kit (**Figure 16C**). In contrast, the majority of cells from the type II colonies of MLL-hDOT1L-transformed cells were negative for Mac-1 or c-Kit, indicating they were most likely not of myeloid lineage (**Figure 16C**). These results indicated that although MLL-hDOT1L(1-670) was capable of immortalizing bone marrow cells arrested at a relatively early stage of myeloid differentiation, it could not completely phenocopy MLL-AF10. Whether the difference was caused by the use of partial, instead of the full-length, hDOT1 L in the MLL fusion and colony assay remains to be determined.

*Up-Regulation of a Specific Subset of Hox Genes in MLL-hDOT1L-Mediated Myeloid Transformation.* Similar to its *Drosophila* homologue Trx, mouse MLL,plays an important role in maintaining the Hox gene expression pattern during embryogenesis (Yu, et al. (1995) Nature 378:505-508). In addition to participating in embryogenesis, Hox gene expression also plays important roles in normal hematopoiesis and leukemogenesis processes (Buske and Humphries (2000) Int. J. Hematol. 71:301-308; Sauvageau, et al. (1994) Proc. Natl. Acad. Sci. USA 91:12223-12227). To understand how hDOT1 L and its associated HMTase activity may contribute to leukemogenesis, we determined the expression profile of Hox genes in murine primary bone marrow cells before and after immortalization by MLL-AF10 or MLL-hDOT1 L(1-670). Since these cell lines represent the earliest stages of myeloid leukemogenesis, they likely have sustained very few, if any, secondary mutations. Given that MLL-hDOT1 L-transformed bone marrow cells give rise to two types of morphologically different colonies, we analyzed their Hox gene expression patterns separately. Accordingly, RNAs were isolated from primary, MLL-AF10- or MLL-hDOT1 L-transformed bone marrow cells. Expression of individual Hox genes in these cells was analyzed by reverse transcriptase PCR (RT-PCR). GAPDH was used as a control for equal RNA input for the different samples. Similar to a previous report (Ayton and Cleary (2003) Genes Dev. 17:2298-2307), cells immortalized by MLL-AF10 have the most numbers of genes in the HoxA complex up-regulated relative to that in primary bone marrow cells (**Figure 17A**). Consistent with the colony assay result demonstrating that the colonies generated from MLL-hDOT1 L(1-670)-transduced cells were different from those generated from MLL-AF10 transduced cells, cells derived from both types of MLL-hDOT1L(1-670)-transduced colonies had fewer numbers of Hox genes up-regulated relative to the MLL-AF10-transformed cells. Indeed, a complete analysis of all the 39 Hox genes identified HoxA9 as the only gene that was up-regulated in MLL-AF10 and both types of MLL-hDOT1 L(1-670)-transformed cells (**Figure 17B**). These data indicate that HoxA9 is likely to be a critical gene in leukemogenesis-mediated by MLL fusion proteins.

*MLL-hDOT1L-Transduced Cells Induce Tumors in Mice.* The fact that MLL-hDOT1L can transform bone marrow cells in the colony assay indicates that hDOT1L may be an oncogene. To evaluate the tumorgenic potential of MLL-hDOT1L, cells derived from third round of plating were transplanted into SCID mice. Of the four mice transplanted with MLL-hDOT1 L-transformed cells, two developed terminal tumor within four weeks of transplantation, the other two mice still appeared healthy at five weeks after transplantation (data not shown). The control mice and the mice transplanted with MLL-AF10-transformed cells still appeared healthy at four weeks of transplantation, consistent with previous deports that SCID mice transplanted with MLL-AF10-transformed bone marrow cells have at least eight weeks of latency. Histological analysis of the tumor derived from transplant of MLL-hDOT1 L-transformed bone marrow cells revealed the tumor as undifferentiated tumor (data not shown).

*hDOT1L Transports AF10 to the Cytosol.* Results provided herein indicate that hDOT1 L participates in MLL-AF10-mediated leukemogenesis. To understand the functional consequence of the AF10 and hDOT1 L interaction in a non-leukemic situation, we examined the effect of their association on their cellular localization in view of the fact that the three NESs of hDOT1 L overlap with the region involved in the AF10 interaction. AF10 and hDOT1L, tagged with green fluorescent protein (GFP) and FLAG^{®}, respectively, were transfected into U2OS cells and their cellular localization was viewed by microscopy. Immunostaining with anti-FLAG^{®} antibody revealed that HOT1L localized to both the nucleus and the cytoplasm while GFP-AF10 localized to the nucleus exclusively when the two proteins were expressed individually (data not shown). Unexpectedly, when co-expressed, these proteins not only co-localized, but were also excluded from nucleus (data not shown), indicating that one of the consequences of AF10 and hDOT1 L association is export of both proteins to cytoplasm. Given that the function of hDOT1 L is to methylate nucleosomal histone H3, association with AF1 0 and subsequent transport to cytosol likely impedes the ability of hDOT1 L to methylate nucleosomal H3. In addition, the nuclear function of AF10 is most likely affected by its export from nucleus. It is possible that the association of AF10 with hDOT1 L may expose the NLS of hDOT1 L and facilitate its nuclear export.

These results, in combination with the finding that MLL-AF10 can direct the nuclear localization of hDOT1 L allowed us to propose a function for hDOT1 L in normal and leukemia cells. Not to be bound by theory, AF10 is possibly exported from nucleus when it associates with hDOT1 L under normal conditions (**Figure 18A**). However, in leukemia cells, where the C-terminal part of AF10 is fused to MLL, although the MLL-AF10 fusion protein is still capable of interacting with hDOT1 L through the OM-LZ present in the C-terminal half of AF10, the MLL-AF10/hDOT1L complex cannot export from the nucleus either because the N-terminal of AF10 is missing or because the nuclear localization signal in MLL keeps the protein complex from exporting (**Figure 18B**). Regardless of the underlying mechanism, one significant epigenetic change in leukemia cells relative to that of normal cells is predictable. It has been demonstrated that MLL is an H3-K4 methyltransferase (Milne, et al. (2002) Mol. Cell 10:1107-1117; Nakamura, et al. (2002) Mol. Cell 10:1119-1128)*.* The promoter of MLL target genes, for example HoxA9, are likely to be enriched for H3-K4 methylation in normal cells.
However, in leukemia cells, as a consequence of MLL and AF10 fusion, the promoter of MLL target gene will not be enriched for H3-K4 methylation due to the loss of the MLL C-terminal SET domain. Instead, the MLL-AF10 fusion will bring in the hDOT1 L resulting in H3-K79 methylation of the MLL target gene promoter (**Figure 18A** and **Figure 18B**).

## Claims

1. A method of identifying a candidate compound for the prevention and/or treatment of leukemia, comprising:
contacting a DOT1 L polypeptide or a fusion protein comprising the DOT1 L polypeptide with a nucleosome substrate comprising histone H3 in the presence of a test compound, the DOT1 L polypeptide comprising an amino acid sequence selected from the group consisting of:
(a) the amino acid sequence of SEQ ID NO:2;
(b) an amino acid sequence having at least 95% amino acid sequence similarity with the amino acid sequence of SEQ ID NO:2, wherein said DOT1L polypeptide has histone H3 lysine 79 (H3-K79) methyltransferase activity; and
(c) a functional fragment of at least 400 amino acids of the amino acid sequence of (a) or (b) above, wherein said functional fragment comprises a DOT1 L histone methyltransferase catalytic domain and has H3-K79 methyltransferase activity;
detecting the level of histone H3 lysine 79 (H3-K79) methylation of the nucleosome substrate under conditions sufficient to provide H3-K79 methylation,
wherein a reduction in H3-K79 methylation in the presence of the test compound as compared with the level of H3-K79 methylation in the absence of the test compound indicates that the test compound is a candidate compound for the prevention and/or treatment of leukemia.

2. The method of claim 1, wherein the method is carried out as a cell-free assay or as a cell-based assay.

3. The method of claim 2, wherein in the instance of using a cell-based assay the DOT1 L polypeptide is expressed from a nucleic acid.

4. A method of identifying a candidate compound for the prevention and/or treatment of leukemia, comprising:
contacting a DOT1 L polypeptide with AF10 or an MLL fusion protein in the presence of a test compound, the DOT1L polypeptide comprising an amino acid sequence selected from the group consisting of:
(a) the amino acid sequence of SEQ ID NO:2;
(b) an amino acid sequence having at least 95% amino acid sequence similarity with the amino acid sequence of SEQ ID NO:2, wherein said DOT1L polypeptide has histone H3 lysine 79 (H3-K79) methyltransferase activity; and
(c) a functional fragment of at least 400 amino acids of the amino acid sequence of (a) or (b) above, wherein said functional fragment comprises a DOT1 L histone methyltransferase catalytic domain and has H3-K79 methyltransferase activity;
detecting the level of binding between the DOT1L polypeptide and AF10 or MLL fusion protein under conditions sufficient for binding of DOT1 L polypeptide to AF10 or MLL fusion protein,
wherein a reduction in binding between DOT1L polypeptide and AF1 0 or the MLL fusion protein in the presence of the test compound as compared with the level of binding in the absence of the test compound indicates that the test compound is a candidate compound for the prevention and/or treatment of leukemia.

5. The method of claim 4, wherein the MLL fusion protein is an MLL-AF10 fusion protein.

6. The method of either claim 4 or 5, wherein the method is carried out as a cell-free assay or as a cell-based assay.

7. The method of claim 6, wherein in the instance of using a cell-based assay the DOT1 L polypeptide and/or AF10 or MLL fusion protein is expressed from a nucleic acid.

8. An *in vitro* method of diagnosing whether a subject has or is at risk for developing leukemia and/or determining the prognosis for the course of the disease, comprising:
detecting the level of histone H3 lysine 79 (H3-K79) methylation associated with the HoxA9 gene in a biological test sample comprising nucloesomes;
wherein an elevation in HoxA9-associated H3-K79 methylation in the biological sample as compared with the level of HoxA9-associated H3-K79 methylation in a non-leukemic biological sample is diagnostic that the subject has or is at risk of developing leukemia and/or is prognostic of the course of the disease in the subject.

9. The method of claim 8, wherein the biological sample is selected from the group comprising; a cell; tissue sample; B cell sample; bone marrow sample; and a nucleosome preparation.

10. The method of claim 9, wherein the method further comprises detecting the level of histone H3 lysine 4 methylation associated with the HoxA9 gene.

## Patentansprüche

1. Ein Verfahren zur Identifizierung einer Verbindung als Kandidat für die Prävention und/oder Behandlung von Leukämie, umfassend:
das Inkontaktbringen eines DOT1L-Polypeptides oder -Fusionsproteins umfassend das DOT1L-Polypeptid mit einem Nukleosomensubstrat, umfassend Histon H3 in Anwesenheit einer Testsubstanz, wobei das DOT1L-Polypeptid eine Aminosäuresequenz umfasst, die ausgewählt ist aus der Gruppe bestehend aus
(a) der Aminosäuresequenz der SEQ-ID NO. 2;
(b) einer Aminosäuresequenz, die mindestens 95% Aminosäuresequenz-Ähnlichkeit zu einer Aminosäuresequenz der SEQ-ID NO. 2 aufweist, worin das DOT1L-Polypeptid eine Histon H3 Lysine 79 (H3-K79-) Methyltransferaseaktivität aufweist; und
(c) einem funktionalen Fragment von mindestens 400 Aminosäuren aus (a) oder (b), worin das funktionale Fragment eine DOTIL-Histon-Methyltransferasekatalytische Domäne umfasst und eine H3-K79-Methyltransferaseaktivität aufweist; und
das Detektieren des Levels der Histon-H3-Lysin 79-(H3-K79-Methylierung des Nukleosomensubstrates unter Bedingungen, die ausreichend sind, um H3-K79-Methylierung zu ermöglichen,
worin eine Reduktion in der H3-K79-Methylierung in Anwesenheit der Testverbindung verglichen mit dem Level der H3-K79-Methylierung in Abwesenheit der Testverbindung anzeigt, dass die Testverbindung eine Verbindung als Kandidat zur Prävention und/oder Behandlung von Leukämie ist.

2. Verfahren gemäß Anspruch 1, worin das Verfahren als ein zellfreier Assay oder zellbasierter Assay ausgeführt wird.

3. Verfahren gemäß Anspruch 2, worin im Falle der Verwendung eines zellbasierten Assays das DOT1L-Polypeptid aus einer Nukleinsäure exprimiert wird.

4. Ein Verfahren zur Identifizierung einer Verbindung als Kandidat für die Prävention und/oder Behandlung von Leukämie, umfassend:
das Inkontaktbringen eines DOTIL-Polypeptides mit AF 10 oder einem MLL-Fusionsprotein in Anwesenheit einer Testverbindung, wobei das DOT1L-Polypeptid eine Aminosäuresequenz umfasst, die ausgewählt ist aus der Gruppe bestehend aus
(a) der Aminosäuresequenz der SEQ-ID NO. 2;
(b) einer Aminosäuresequenz, die mindestens 95% Aminosäuresequenz-Ähnlichkeit zu eine Aminosäuresequenz der SEQ-ID NO. 2 aufweist, worin das DOT1L-Polypeptid eine Histon H3-Lysine 79-(H3-K79-) Methyltransferaseaktivität aufweist; und
(c) einem funktionalen Fragment von mindestens 400 Aminosäuren aus der obigen Aminosäuresequenz (a) oder (b), worin das funktionale Fragment eine DOT1L-Histon-Methyltransferasekatalytische Domäne umfasst und eine H3-K79-Methyltransferaseaktivität aufweist; und
das Detektieren des Bindungslevels zwischen dem DOT1L-Polypeptid und AF10 oder dem MLL-Fusionsprotein unter Bedingungen, die ausreichend sind, um das DOT1L-Polypeptid an AF10 oder an das MLL-Fusionsprotein zu binden,
worin eine Reduktion der Bindung zwischen dem DOT1L-Polypeptid und AF10 oder dem MLL-Fusionsprotein in Anwesenheit der Testverbindung verglichen mit dem Bindungslevel in Abwesenheit der Testverbindung anzeigt, dass die Testverbindung eine Verbindung als Kandidat zur Prävention und/oder Behandlung von Leukämie ist.

5. Verfahren gemäß Anspruch 4, worin das MLL-Fusionsprotein ein MLL-AF10-Fusionsprotein ist.

6. Verfahren gemäß einem der Ansprüche 4 oder 5, worin das Verfahren als zellfreier Assay oder zellbasierter Assay ausgeführt wird.

7. Verfahren gemäß Anspruch 6, worin im Falle der Verwendung eines zellbasierten Assays, das DOT1L-Polypeptid und/oder AF10 oder das MLL-Fusionsprotein aus einer Nukleinsäure exprimiert wird.

8. Ein *in* vitro-Verfahren zur Diagnose, ob ein Patient eine Leukämie entwickelt hat oder das Risiko hat, eine Leukämie zu entwickeln, und/oder zur Bestimmung der Prognose des Krankheitsverlaufs, umfassend:
das Detektieren des Levels an HoxA9-Gen-assoziierter H3 Lysin 79-(H3-K79-) Methylierung in einer Nukleosomen umfassenden biologischen Testprobe;
worin eine Erhöhung der HoxA9-assoziierten H3-K79-Methylierung in der biologischen Probe, verglichen mit dem Level der HoxA9-assoziierten H3-K79 Methylierung in einer nicht leukämischen biologischen Probe, diagnostiziert, dass der Patient Leukämie entwickelt hat oder ein Risiko hat, Leukämie zu entwickeln und/oder den Verlauf der Krankheit des Patienten prognostiziert.

9. Verfahren gemäß Anspruch 8, worin die biologische Probe ausgewählt ist aus der Gruppe umfassend: eine Zelle; Gewebeprobe; B-Zellprobe; Knochenmarksprobe und eine Nukleosomenzubereitung.

10. Verfahren gemäß Anspruch 9, worin das Verfahren weiter umfasst das Detektieren des Levels der mit dem HoxA9-Gen assoziierten Histon H3-Lysin 4-Methylierung.

## Revendications

1. Procédé d'identification d'un composé candidat pour la prévention et/ou le traitement de la leucémie, comprenant :
- la mise en contact d'un polypeptide DOT1L ou d'une protéine de fusion comprenant le polypeptide DOT1L avec un substrat de nucléosome comprenant l'histone H3 en présence d'un composé d'essai, le polypeptide DOT1L comprenant une séquence d'acides aminés choisie dans le groupe consistant en :
(a) la séquence d'acides aminés de SEQ ID NO:2 ;
(b) une séquence d'acides aminés ayant au moins 95 % de similarité de séquence d'acides aminés avec la séquence d'acides aminés de SEQ ID NO:2, ledit polypeptide DOT1L ayant une activité histone H3 lysine 79 (H3-K79) méthyltransférase ; et
(c) un fragment fonctionnel d'au moins 400 acides aminés de la séquence d'acides aminés de (a) ou (b) ci-dessus, ledit fragment fonctionnel comprenant un domaine catalytique DOT1L histone méthyltransférase et ayant une activité H3-K79 méthyltransférase ;
- la détection du taux de méthylation par histone H3 lysine 79 (H3-K79) du substrat de nucléosome dans des conditions suffisantes pour fournir une méthylation par H3-K79,
une réduction de la méthylation par H3-K79 en présence du composé d'essai par comparaison avec le taux de méthylation par H3-K79 en l'absence du composé d'essai indiquant que le composé d'essai est un composé candidat pour la prévention et/ou le traitement de la leucémie.

2. Procédé selon la revendication 1, dans lequel le procédé est réalisé comme essai exempt de cellules ou comme essai à base de cellules.

3. Procédé selon la revendication 2, dans lequel, dans le cas de l'utilisation d'un essai à base de cellules, le polypeptide DOT1L est exprimé à partir d'un acide nucléique.

4. Procédé d'identification d'un composé candidat pour la prévention et/ou le traitement de la leucémie, comprenant :
- la mise en contact d'un polypeptide DOT1L avec AF10 ou une protéine de fusion MLL en présence d'un composé d'essai, le polypeptide DOT1L comprenant une séquence d'acides aminés choisie dans le groupe constitué par :
(a) la séquence d'acides aminés de SEQ ID NO:2 ;
(b) une séquence d'acides aminés ayant au moins 95 % de similarité de séquence d'acides aminés avec la séquence d'acides aminés de SEQ ID NO:2, ledit polypeptide DOT1L ayant une activité histone H3 lysine 79 (H3-K79) méthyltransférase ; et
(c) un fragment fonctionnel d'au moins 400 acides aminés de la séquence d'acides aminés de (a) ou (b) ci-dessus, ledit fragment fonctionnel comprenant un domaine catalytique DOT1L histone méthyltransférase et ayant une activité H3-K79 méthyltransférase ;
- la détection du taux de liaison entre le polypeptide DOT1L et AF10 ou la protéine de fusion MLL dans des conditions suffisantes pour la liaison du polypeptide DOT1L à AF10 ou à la protéine de fusion MLL,
une réduction de la liaison entre le polypeptide DOT1L et AF10 ou la protéine de fusion MLL en présence du composé d'essai par comparaison avec le taux de liaison en l'absence du composé d'essai indiquant que le composé d'essai est un composé candidat pour la prévention et/ou le traitement de la leucémie.

5. Procédé selon la revendication 4, dans lequel la protéine de fusion MLL est une protéine de fusion MLL-AF10.

6. Procédé selon l'une ou l'autre des revendications 4 ou 5, dans lequel le procédé est réalisé comme essai exempt de cellules ou comme essai à base de cellules.

7. Procédé selon la revendication 6, dans lequel, dans le cas de l'utilisation d'un essai à base de cellules, le polypeptide DOT1L et/ou AF10 ou la protéine de fusion MLL est exprimé à partir d'un acide nucléique.

8. Procédé *in vitro* de diagnostic du point de savoir si un sujet a ou est à risque de développer une leucémie et/ou de détermination du pronostic pour l'évolution de la maladie, comprenant :
- la détection du taux de méthylation par histone H3 lysine 79 (H3-K79) associée au gène HoxA9 dans un échantillon d'essai biologique comprenant des nucléosomes ;
une élévation de la méthylation par H3-K79 associée à Hox-A9 dans l'échantillon biologique par comparaison avec le taux de méthylation par H3-K79 associée à HoxA9 dans un échantillon biologique non leucémique étant le diagnostic que le sujet a ou est à risque de développer une leucémie et/ou est un pronostic de l'évolution de la maladie dans le sujet.

9. Procédé selon la revendication 8, dans lequel l'échantillon biologique est choisi dans le groupe comprenant : une cellule ; un échantillon de tissu ; un échantillon de lymphocytes B ; un échantillon de moelle osseuse ; et une préparation de nucléosome.

10. Procédé selon la revendication 9, dans lequel le procédé comprend en outre la détection du taux de méthylation par histone H3 lysine 4 associée au gène HoxA9.
